# FASCICULE DE BREVET EUROPEEN

(11) **EP 2 094 865 B1**
(45) Date de publication et mention de la délivrance du brevet: **04.08.2010**
(21) Numéro de dépôt: 07858655.9
(22) Date de dépôt: 24.10.2007
(51) Int. Cl.: C12Q 1/68

(54) **PROCEDE D'IDENTIFICATION MOLECULAIRE ET GENOTYPAGE DES BACTERIES DU COMPLEXE MYCOBACTERIUM TUBERCULOSIS**
VERFAHREN ZUR MOLEKULAREN IDENTIFIZIERUNG UND GENOTYPISIERUNG VON BAKTERIEN DES MYKOBAKTERIUMTUBERKULOSE-KOMPLEXES
METHOD FOR MOLECULAR IDENTIFICATION AND GENOTYPING OF BACTERIA OF THE MYCOBACTERIUM TUBERCULOSIS COMPLEX

(30) Priorité: 26.10.2006 FR 0654549
(43) Date de publication de la demande: 02.09.2009
(73) Titulaire: Université de la Méditerranée (Aix-Marseille II), 13284 Marseille Cédex 07 (FR); Centre National de la Recherche Scientifique (CNRS), 75794 Paris Cedex 16 (FR)
(72) Inventeur: DRANCOURT, Michel, 13012 Marseille (FR); DJELOUADJI, Zoheira, 13005 Marseille (FR); RAOULT, Didier, 13008 Marseille (FR)
(74) Mandataire: Domange, Maxime
(86) Numéro de dépôt international: PCT/FR2007/052236
(87) Numéro de publication internationale: WO 2008/050064

(56) Documents cités:
- WO-A-2004/083448
- US-A- 6 074 820
- US-A1- 2003 219 757
- FOURNIER PIERRE-EDOUARD ET AL: "Use of highly variable intergenic spacer sequences for multispacer typing of Rickettsia conorii strains." JOURNAL OF CLINICAL MICROBIOLOGY DEC 2004, vol. 42, no. 12, décembre 2004 (2004-12), pages 5757-5766, XP002434928 ISSN: 0095-1137 cité dans la demande
- COLE S T ET AL: "Deciphering the biology of mycobacterium tuberculosis from the complete genome sequence" NATURE 11 JUN 1998 UNITED KINGDOM, vol. 393, no. 6685, 11 juin 1998 (1998-06-11), pages 537-544, XP002434927 ISSN: 0028-0836 cité dans la demande
- DATABASE EMBL [Online] 11 juin 2004 (2004-06-11), "mth2-190A3FM1 BAC end, cultivar Jemalong A17 of Medicago truncatula." XP002476004 extrait de EBI accession no. EMBL:CR479678 Database accession no. CR479678
- DATABASE EMBL [Online] 24 février 2001 (2001-02-24), DUNN ET AL.: "Mouse whole genome scaffolding with paired end reads from 10kb plasmid insert." XP002434930 extrait de EMBL-EBI accession no. AZ875467
- DATABASE Geneseq [Online] 15 janvier 2002 (2002-01-15), "Mycobacterium tuberculosis strain H37Rv genome SEQ ID NO 1." XP002476005 extrait de EBI accession no. GSN:AAI99682 Database accession no. AAI99682
- DATABASE Geneseq [Online] 15 janvier 2002 (2002-01-15), "Mycobacterium tuberculosis strain H37Rv genome SEQ ID NO 2." XP002476006 extrait de EBI accession no. GSN:AAI99683 Database accession no. AAI99683
- DATABASE EMBL [Online] 10 juin 2003 (2003-06-10), "Mycobacterium bovis subsp. bovis AF2122/97 complete genome; segment 2/14" XP002476007 extrait de EBI accession no. EMBL:BX248335 Database accession no. BX248335
- DATABASE EMBL [Online] 9 juin 1997 (1997-06-09), "Mycobacterium tuberculosis senX3, regX3 genes and 3 putative ORF's" XP002476008 extrait de EBI accession no. EMBL:Y13628 Database accession no. Y13628
- DATABASE Geneseq [Online] 8 décembre 1998 (1998-12-08), "Mycobacterium bovis intercistronic repeat sequence." XP002476009 extrait de EBI accession no. GSN:AAV49483 Database accession no. AAV49483
- FLEISCHMANN R D ET AL: "Whole-genome comparison of Mycobacterium tuberculosis clinical and laboratory strains" JOURNAL OF BACTERIOLOGY, vol. 184, no. 19, octobre 2002 (2002-10), pages 5479-5490, XP002434926 ISSN: 0021-9193

## Description

La présente invention concerne une méthode d'identification moléculaire des différentes espèces de bactéries du complexe *Mycobacterium tuberculosis* et le génotypage desdites espèces par des techniques de détection et/ou d'amplification et séquençage d'acides nucléiques desdites bactéries à l'aide de sondes ou d'amorces oligonucléotidiques.

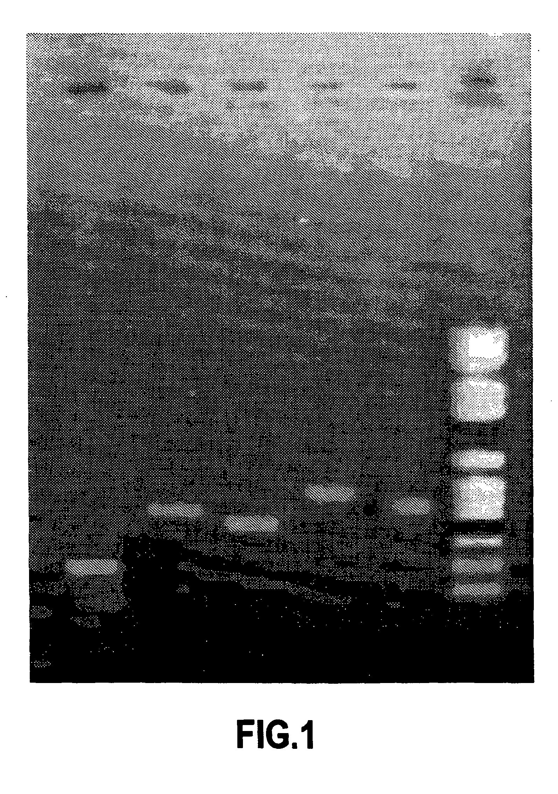

Les bactéries du complexe *Mycobacterium tuberculosis* sont des bactéries bacillaires, mal colorées par la coloration de Gram et bien colorées par la coloration de Ziehl-Neelsen dont on reconnaît actuellement 5 espèces (figure 1) : *Mycobacterium tuberculosis* est une espèce strictement humaine, il s'agit d'un agent ubiquitaire de la tuberculose ; *Mycobacterium africanum,* qui comporte deux sous-types I et II, est une espèce strictement humaine, il s'agit d'un agent de la tuberculose en Afrique sub-saharienne ; le statut taxonomique de cette espèce est discuté, et le sous-type II est pratiquement indiscernable de l'espèce *Mycobacterium tuberculosis* en dehors de l'aspect macroscopique des colonies et de la décoloration du milieu au bromocrésol [Arnold C. et al. Single-nucleotide polymorphism-based differentiation and drug résistance detection in Mycobacterium tuberculosis from isolates or directly from sputum. Clin. Microbiol. Infect. 2005 ; 11:122-130 ; Niemann S, Harmsen D, Rusch-Gerdes S, Richter E. Differentiation of clinical Mycobacterium tuberculosis complex isolates by gyrB DNA sequence polymorphism analysis. J Clin Microbiol. 2000 ;38:3231-4] ; le sous-type II de l'espèce *Mycobacterium africanum* est maintenant considéré comme un variant phenotypique de l'espèce *Mycobacterium tuberculosis* [Nieman S. et al. The species Mycobacterium africanum in the light of new molecular markers. J. Clin. Microbiol. 2004 ; 42:3958-3962] ;

*Mycobacterium canettii* est une espèce de réservoir inconnu, responsable en Afrique de tuberculose ganglionnaire et de tuberculose systémique chez les patients co-infectés par le virus de l'immunodéficience humaine ; *Mycobacterium bovis* (qui comporte trois sous-espèces, *Mycobacterium bovis,* ssp. *bovis, Mycobacterium bovis* ssp. *caprae* et *Mycobacterium bovis* souche vaccinale BCG (ci-après *Mycobacterium* ssp BCG)) est une espèce ubiquitaire animale, responsable de tuberculose chez les animaux à sang chaud, y compris l'homme ; *Mycobacterium microti* est une espèce ubiquitaire animale responsable de tuberculose chez les animaux à sang chaud, et exceptionnellement de tuberculose humaine [Pfyffer GE, Brown-Elliott BA, Wallace RJ, Jr. Mycobacterium : general characteristics, isolation, and staining procedures. In : Murray PR, baron EJ, Jorgensen JH, Pfaller MA, Yolken RH : Manual of Clinical Microbiology 8th Edition, American Society for Microbiology, Washington DC, 2003, pp. 532-559]. Ces espèces sont fréquemment cultivées à partir de prélèvements cliniques vétérinaires et de prélèvements cliniques humains. Chez l'homme, ces différentes espèces du complexe *Mycobacterium tuberculosis* sont responsables de tuberculose communautaire et exceptionnellement de tuberculose nosocomiale. Toutefois, l'espèce *Mycobacterium tuberculosis* est la souche la plus fréquemment isolée dans les cas de tuberculose humaine.

La tuberculose est une maladie infectieuse contagieuse par transmission par voie aérienne, spontanément mortelle, curable par traitement antibiotique comportant 3 à 4 antibiotiques différents, prolongé pendant plusieurs mois. Un problème émergent est celui des souches de l'espèce *Mycobacterium tuberculosis* dites multi-résistantes, c'est-à-dire résistantes à la rifampicine et à au moins un autre antibiotique antituberculeux. Ces souches sont plus difficiles à traiter et sont associées à un mauvais pronostic de la tuberculose. L'espèce *Mycobacterium tuberculosis* est une bactérie persistante dans l'organisme du fait d'une immunité non-stérilisante, c'est-à-dire que les bactéries transmises par voie aérienne demeurent vivantes et conservent une capacité à se multiplier, dans des gîtes ganglionnaires durant la vie entière. Ces bactéries quiescentes sont capables de se multiplier et de provoquer une tuberculose maladie lors d'une diminution de l'immunité de la personne.

Ces différentes espèces bactériennes posent le problème de leur détection dans les prélèvements pathologiques chez l'homme, et de leur identification au niveau de l'espèce et de la sous-espèce et de leur génotypage au niveau des souches lorsqu'elles ont été isolées à partir desdits prélèvements. Les méthodes conventionnelles de détection reposent en effet sur la mise en évidence de bactéries bacillaires Ziehl positif, à l'examen direct du produit pathologique. Il est cependant connu que cette détection microscopique des bactéries du genre *Mycobacterium* dans les prélèvements cliniques a un seuil de sensibilité de 10⁴ CFU/ml. Il est donc tout à fait possible qu'un prélèvement pathologique chez l'homme ou chez l'animal contienne une des espèces considérées qui ne soit pas détectée à l'examen microscopique direct de ce prélèvement pathologique.

Lorsque qu'une bactérie d'une des espèces considérées est isolée au laboratoire, les méthodes conventionnelles d'identification phénotypique sont les plus couramment utilisées pour l'identification des bactéries des espèces du genre *Mycobacterium.* Elles comprennent l'étude du métabolisme du glycérol et du pyruvate sur milieu de Lowenstein-Jensen, l'étude du caractère microaérophile, l'accumulation de la niacine, l'activité nitrate-réductase, l'aspect des colonies et l'étude de la résistance au thiophen-2-carboxylic-acid hydrazide et au pyrazinamide. Sur ce plan, le degré d'identification en pratique courante est variable. Il est reconnu qu'il existe un certain degré de subjectivité dans l'appréciation des résultats des tests phénotypiques, en particulier pour apprécier l'aspect des colonies qui peut changer en fonction de la virulence de la souche et de sa résistance aux antibiotiques [Kamerbeek J et al. Simultaneous detection and strain differentiation of Mycobacterium tuberculosis for diagnosis and epidemiology. J Clin Microbiol. 1997; 35:907-914]. Parmi les méthodes phénotypiques, l'analyse des acides mycoliques de paroi par chromatographie liquide ne permet de distinguer que *Mycobacterium bovis* souche BCG des autres espèces du complexe *Mycobacterium tuberculosis* [Floyd M. Separation of Mycobacterium bovis BCG from Mycobacterium tuberculosis and Mycobacterium bovis by using high-performance liquid chromatography of mycolic acids. J Clin Microbiol. 1992 ; 30 :1327-1330].

Plusieurs systèmes moléculaires ont été développés pour l'identification des différentes espèces du complexe *Mycobacterium tuberculosis* mais aucune méthode ne permet actuellement de réaliser l'identification d'espèce en une seule étape par l'analyse de séquences génomiques.

En effet, la méthode princeps basée sur la séquence du gène 16S ARNr codant l'ARN ribosomal 16S, est commercialisée sous forme d'un système d'hybridation de sonde froide AccuProbe (Gen-Probe, San Diego, CA) très utilisé pour la détection et l'identification des bactéries du complexe *Mycobacterium tuberculosis.* Cependant, le fait que la séquence du gène 16S ARNr comme celle de l'ITS 16S-23S [Frothingham R. et al. Extensive DNA sequence conservation throughout the Mycobacterium tuberculosis complex. J. Clin. Microbiol. 1994 ; 32:1639-1643] soient rigoureusement identiques pour les différentes espèces du complexe, ne permet pas à ces systèmes de distinguer chacune des cinq espèces du complexe [Kirschner P. Genotypic identification of mycobacteria by nucleic acid séquence determination : report of a two-year experience in a clinical laboratory. J Clin Microbiol. 1993 ; 31 :2882-2889].

Un deuxième système d'hybridation basé sur la séquence du gène 23S ARNr, commercialisé sous le nom de GenoType MTBC (Hain Lifescience GmbH, Nehren, Allemagne) permet de distinguer *M. tuberculosis, M. bovis* ssp. *bovis* et *M. africanum* sous-type I [Richter E. et al. Usefulness of the GenoType MTBC assay for differentiating species of the Mycobacterium tuberculosis complex in cultures obtained from clinical "specimens. J Clin Microbiol. 2004; 42:4303-4306], mais ne permet pas de distinguer les autres espèces de ce complexe.

De même, la séquence du gène *rpo*B est identique pour les différentes espèces du complexe *Mycobacterium tuberculosis* et ne permet donc pas l'identification d'espèce [Arnold C. et al. Single-nucleotide polymorphism-based differentiation and drug résistance detection in Mycobacterium tuberculosis from isolates or directly from sputum. Clin. Microbiol. Infect. 2005 ; 11:122]. Les seules variations de séquence *rpo*B connues sont liées à la résistance à la Rifampicine chez *Mycobacterium tuberculosis.*

L'analyse de la séquence du gène *oxy*R permet de différencier uniquement l'espèce *Mycobacterium bovis* au sein du complexe *Mycobacterium tuberculosis* [Sreevatsan S. et al. Identification of a polymorphic nucleotide in oxyR specific for Mycobacterium bovis. J Clin Microbiol 1996; 34:2007-2010].

De la même façon, la séquence partielle du gène *hupB* permet d'identifier *Mycobacterium tuberculosis* et *Mycobacterium bovis* [Prabhakar S. et al. Use of the hupB gene encoding a histone-like protein of Mycobacterium tuberculosis as a target for detection and differentiation of M. tuberculosis and M. bovis. J Clin Microbiol 2004; 42:2724-2732].

L'analyse des profils de régions de différences ("DR") qui sont des régions répétées, obtenus par amplification permet de différencier les différentes espèces du complexe par différence de taille des amplifiats obtenus [Parsons LM et al. Rapid and simple approach for identification of Mycobacterium tuberculosis complex isolates by PCR-based genomic deletion analysis. J Clin Microbiol. 2002; 40:2339-45]. Egalement, l'analyse des profils des restriction du gène *gyr*B permet de distinguer *Mycobacterium tuberculosis* et *Mycobacterium bovis* [Chimara E. et al. Mycobacterium tuberculosis complex differentiation using gyrB-restriction fragment length polymorphism analysis. Mem Inst Oswaldo Cruz 2004; 99:745-748]. Cependant les résultats des analyses des profils ne peuvent pas être comparés d'une expérience aux suivantes car les conditions de migration des gels modifient sensiblement les profils obtenus. Il faut donc introduire systématiquement des témoins dans chaque expérience. Les résultats sont difficilement exportables et comparables d'un laboratoire à un autre.

Le séquençage du gène *gyr*B permet de distinger *M*. *tuberculosis, M. bovis, M. african* type I et *M*. *microti* [Niemann S, Harmsen D, Rusch-Gerdes S, Richter E. Differentiation of clinical Mycobacterium tuberculosis complex isolates by gyrB DNA sequence polymorphism analysis. J Clin Microbiol. 2000 ;38:3231-4]. De façon dérivée du séquençage, l'analyse des profils de restriction du gène *gyr*B amplifié ou l'analyse des single-nucleotide polymorphims (SNP) par pyroséquençage [Arnold C. et al. Single-nucleotide polymorphism-based differentiation and drug resistance détection in Mycobacterium tuberculosis from isolates or directly from sputum. Clin. Microbiol. Infect. 2005 ; 11:122] permettent également de distinguer les mêmes espèces. Cependant, l'utilisation du gène *gyr*B ne permet pas de distinguer *M. tuberculosis, M. africanum* type II et *M. canettii.*

Un premier but de l'invention est de fournir une méthode d'identification moléculaire des différentes espèces du complexe *Mycobacterium tuberculosis* reposant sur l'analyse de séquences nucléotidiques du génome desdites bactéries.

Lorsqu'un isolat clinique a été identifié comme appartenant à une espèce, notamment l'espèce *Mycobacterium tuberculosis,* il est utile de disposer, en outre, d'un système de génotypage permettant de regrouper cet isolat avec d'autres isolats réalisés chez des patients différents au sein d'une même souche, c'est-à-dire de reconnaître les isolats clonaux, issus d'une même souche de ladite espèce, notamment *Mycobacterium tuberculosis.* Les différentes techniques de génotypage contribuent ainsi à distinguer, chez un patient donné, la ré-infection (liée à une nouvelle souche) de la rechute (liée à une souche unique, latente dans un site ganglionnaire) de tuberculose. De même, le génotypage contribue à déterminer les patients exposés à une source unique devant des cas groupés (cas familiaux par exemple) et à l'étude des épidémies ; l'utilisation de techniques de génotypage permet ainsi de démasquer des épidémies passées inaperçues par les méthodes purement cliniques et épidémiologiques. Enfin, le génotypage permet de démasquer les contaminations des prélèvements au laboratoire, qui sont une source de résultats faux-positifs. En effet, les méthodes actuelles reposent sur l'analyse de profils obtenus :
- soit par restriction de l'ADN chromosomique : méthode par électrophorèse sur gel par champ pulsé ("pulsed-field gel electrophoresis") après restriction par des enzymes à basse fréquence de coupure [Zhang, Y., Mazurek, GH., Cave, MD., Eisnach, KD., Pang, Y., Murphy, DT., and RJ. Wallace. 1992. DNA polymorphism in strain of Mycobacterium tuberculosis analysed by Pulsed Gel Electrophoresis a tool for epidemiolgy. J. Clin. Microbiol. 30: 1551-1556; Singh, SP., Salamon, H., Lahti, CJ., Farid-Moyer, M., And PM. Small. 1999. Use of "Pulsed Field Gel Electrophoresis for molecular epidemiologic and population genetic studies of Mycobacterium tuberculosis. J. Clin. Microbiol. 6: 1927-1931] et méthode d'hybridation de sonde de l'élément d'insertion IS*6110* [Eisenach, KD., Crawford, JT., and JH. Bates. 1988. Repetitive DNA sequences as probes of Mycobacterium tuberculosis. J. Clin. Microbiol. 11: 2240-2245; Van Embden, JD., Caves, MD., Crawford, JT., Dale, JW., Eisenach, KD., Gicquel, B., Hermans, P., Martin, C. Mc., Adam, R., and TM. Shinnick. 1993. Strain identification of Mycobacterium tuberculosis by DNA fingerprinting recommendation for standardized methodology. J Clin Microbiol. 31:406-409];
- soit par amplification d'éléments répétés dans des régions du génome appelées "direct repeat regions" (spoligotyping) [Kamerbeek, J., Schouls, L., Kolk, A., Van Agterveld, M., Van soolingen, D., Kuijper, S., Bunschoten, A., Molhuizen, H., Shaw, R., Goyal, M.,and JD. Van Embden. 1997. Simultaneous detection and strain differentiation of mycobacterium tuberculosis for diagnosis and epidemiology. J. Clin. Microbiol. 35:907-914; Dale, JW., Brittain, D., Cataldi, AA., Cousins, D., Crawford, JT., Driscoll, J., Heersma, H., Lillebaek, T., Quitugua, T., Rastogi, N., Skuce, RA., Sola, C., Van soolingen, D., Vincent, V.,and al. Spacer oligonucleotide typing of bacteria of the Mycobacterium tuberculosis complex: recommendations for standardised nomenclature. 2001. Int J Tuberc Lung Dis. 5:216-219], ou par amplification de "variable number tandem repeats" (VNTR) dans des régions disséminées dans l'ensemble du génome, ou encore par amplification de "mycobacterial interspersed repeat units" (MIRU) [Frothingham, R., Meeker-O'Connel. WA. 1998. Genetic diversity in the Mycobacterium tuberculosis complex based on variable numbers of tandem DNA repeats. Microbiology. 144:1189-96 ; Mazars, E., Lesiean, S., Banuls, AL., Gilbert, M., Vincent, V., Gicquel, B., Tibayrenc, M., Locht, C.,and P. Supply. 2001. High-resolution minisatellite-based typing as a portable approach to global analysis of Mycobacterium tuberculosis molecular epidemiology. Proc Natl Acad Sci USA. 98:1901-1906 ; Supply, P., Pan, X., Stockbaner, KE., Connel, ND., Kreiswirth, BN., Whittman, TS., and JM. Musser. 2001. Restricted structural gene polymorphism in the Mycobacterium tuberculosis complex indicates evolutionarily recent global dissimination. J. Clin. Microbiol. 39:3563-3571; Gibson, A., Brown, T., Baker, L.,and F Drobniewski. 2005. Can 15-locus mycobacterial interspersed repetitive unit-variable-number tandem repeat analysis provide insight into the évolution of Mycobacterium tuberculosis. Appl. Environ. Microbiol. 71:8207-13].

Un concept de génotypage par analyse de zones intergéniques a déjà été appliqué à d'autres espèces bactériennes, telles que *Yersinia pestis* [Drancourt M, Roux V, Dang LV, Tran-Hung L, Castex D, Chenal-Francisque V, Ogata H, Fournier PE, Crubezy E, Raoult D. Genotyping, Orientalis-like Yersinia pestis, and plague pandemics. Emerg Infect Dis. 2004 Sep;10(9):1585-92], *Bartonella quintana* [Foucault C, La Scola B, Lindroos H, Andersson SG, Raoult D. Multispacer typing technique for sequence-based typing of Bartonella quintana. J Clin Microbiol. 2005 Jan;43(1):41-8], *Bartonella benselae* [Li W, Chomel BB, Maruyama S, Guptil L, Sander A, Raoult D, Fournier PE. Multispacer typing to study the genotypic distribution of Bartonella benselae populations. J Clin Microbiol. 2006 Jul;44(7):2499-506.], *Rickettsia prowazekii* [Zhu Y, Fournier PE, Ogata H, Raoult D. Multispacer typing of Rickettsia prowazekii enabling epidemiological studies of epidemic typhus. J Clin Microbiol. 2005 Sep;43(9):4708-12], *Rickettisia conorii* [Fournier PE, Zhu Y, Ogata H, Raoult D. Use of highly variable intergenic spacer sequences for multispacer typing of Rickettsia conorii strains. J Clin Microbiol. 2004 Dec;42(12):5757-66], *Coxiella burnetii* [Glazunova O, Roux V, Freylikman O, Sekeyova Z, Fournous G, Tyczka J, Tokarevich N, Kovacava E, Marrie TJ, Raoult D. Coxiella burnetii genotyping. Emerg Infect Dis. 2005 Aug;11 (8):1211-7].

Mais, les zones intergéniques concernées n'étaient pas applicables aux espèces du complexe *Mycobacterium tuberculosis,* et en outre, ces méthodes de génotypage permettaient de réaliser seulement le génotypage des isolats d'une même espèce bactérienne, sans différencier les espèces de bactéries d'un même genre.

Il n'existe actuellement aucune technique de génotypage des espèces du complexe *Mycobacterium tuberculosis* et notamment de l'espèce *Mycobacterium tuberculosis* par analyse de séquence.

Un deuxième but de la présente invention est donc de fournir une méthode d'identification moléculaire qui permette, en outre, le génotypage des isolats et souches d'une espèce du complexe *Mycobacterium tuberculosis* et notamment de l'espèce *Mycobacterium tuberculosis* reposant sur l'analyse de séquences nucléotides et, plus particulièrement fournir une méthode de génotypage plus discriminante que les méthodes actuellement disponibles.

Les inventeurs ont déterminé selon la présente invention, un système appelé "Multi Spacer Typing" (abrégé ci-après en MST) constitué d'une série de fragments d'acides nucléiques de zones intergéniques non codantes du génome des bactéries du complexe *Mycobacterium tuberculosis,* lesdits fragments constituant des marqueurs génétiques permettant l'identification des différentes espèces du complexe *Mycobacterium tuberculosis* et le génotypage des isolats d'une même espèce du complexe *Mycobacterium tuberculosis,* et notamment de l'espèce *Mycobacterium tuberculosis,* par l'analyse des séquences desdits fragments des zones appelées MST. Ces régions intergéniques non codantes qui ne codent pas pour des protéines ni pour des ARNs, sont aussi appelées ci-après "spacers". Pour chaque fragment desdites zones intergéniques, plusieurs événements génétiques de même signification évolutive peuvent distinguer les différents variants génotypiques : mutation ponctuelle, délétion ou insertion. La combinaison des différents variants génotypiques de chaque fragment de zone intergénique constitue un code qui permet d'identifier les espèces au sein du complexe *Mycobacterium tuberculosis.* D'autre part, l'analyse comparative des séquences de plusieurs fragments de "spacers", simultanée et combinée de plusieurs, notamment de 2 à 8 séquences de "spacers", qui sont des régions génomiques situées entre les gènes bactériens, permet de réaliser le génotypage des isolats au sein d'une même espèce et, notamment de l'espèce *Mycobacterium tuberculosis.*

Avant d'exposer plus en détail l'invention, différents termes, utilisés dans la description et les revendications, sont définis ci-après :
- Par "acide nucléique extrait de bactéries" on entend soit l'acide nucléique total, soit l'ADN génomique, soit les ARN messagers, soit encore l'ADN obtenu à partir de la transcription inverse des ARN messagers.
- Un "fragment nucléotidique" ou un "oligonucléotide" sont deux termes synonymes désignant un enchaînement de motifs nucléotidiques caractérisé par une séquence informationnelle des acides nucléiques naturels (ou éventuellement modifiés) et susceptibles de s'hybrider, comme les acides nucléiques naturels, avec un fragment nucléotidique complémentaires ou sensiblement complémentaire, dans des conditions prédéterminées de stringence stricte. L'enchaînement peut contenir des motifs nucléotidiques de structure différente de celle des acides nucléiques naturels. Un fragment nucléotidique (ou oligonucléotide) peut contenir par exemple jusqu'à 100 motifs nucléotidiques. Il contient généralement au moins 8, et en particulier au moins 12 motifs nucléotidiques, en particulier de 18 à 35, et peut être obtenu à partir d'une molécule d'acide nucléique naturelle et/ou par recombinaison génétique et/ou par synthèse chimique.
- Un motif nucléotidique est dérivé d'un monomère qui peut être un nucléotide naturel d'acide nucléique dont les éléments constitutifs sont un sucre, un groupement phosphate et une base azotée choisie parmi l'adénine (A), la guanine (G), l'uracile (U), la cytosine (C), la thymine (T) ; ou bien le monomère est un nucléotide modifié dans l'un au moins des trois éléments constitutifs précédents ; à titre d'exemple, la modification peut intervenir soit au niveau des bases, avec des bases modifiées telles que l'inosine, qui peut s'hydrider avec toute base A, T, U, C ou G, la méthyl-5-désoxycytidine, la désoxyuridine, la diméthylamino-5-désoxyuridine ou toute autre base modifiée capable d'hybridation, soit au niveau du sucre, par exemple le remplacement d'au moins un désoxyribose par un polyamide [Nielsen PE et al. Sequence-selective recognition of DNA by strand displacement with a thymine-substituted polyamide. Science. 1991 ; 254:1497-1500], soit encore au niveau du groupement phosphate, par exemple par remplacement par des esters choisis notamment parmi les diphosphates, les alkylphosphonates et les phosphorothioates.
- Par "hybridation", on entend le processus au cours duquel, dans des conditions appropriées, deux fragments nucléotidiques ayant des séquences suffisamment complémentaires sont susceptibles de s'associer par des liaisons hydrogène stables et spécifiques, pour former un double brin. Les conditions d'hybridation sont déterminées par la "stringence", c'est à dire la rigueur des conditions opératoires. L'hybridation est d'autant plus spécifique qu'elle est effectuée à plus forte stringence. La stringence est fonction notamment de la composition en bases d'un duplex sonde/cible, ainsi que par le degré de mésappariement entre deux acides nucléiques. La stringence peut également être fonction des paramètres de la réaction d'hybridation, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépend notamment des sondes utilisées. Toutes ces données sont bien connues de l'homme de l'art et les conditions appropriées peuvent éventuellement être déterminées dans chaque cas par des expériences de routine. En général, selon la longueur des sondes utilisées, la température pour la réaction d'hybridation est comprise entre environ 20 et 65°C, en particulier entre 35 et 65°C dans une solution saline à une concentration d'environ 0,8 à 1 M.
- Une "sonde" est un fragment nucléotidique possédant une spécificité d'hybridation dans des conditions déterminées pour former un complexe d'hybridation avec un acide nucléique ayant, dans le cas présent, une séquence nucléotidique comprise soit dans un ARN messager, soit dans un ADN obtenu par transcription inverse dudit ARN messager, produit de transcription; une sonde peut être utilisée à des fins de diagnostic (notamment sondes de capture ou de détection) ou à des fins de thérapie,
- Une "sonde de capture" est une sonde immobilisée ou immobilisable sur un support solide par tout moyen approprié, par exemple par covalence, par adsorption, ou par synthèse directe sur un solide. Des exemples de supports comprennent les plaques de microtitration et les puces à ADN.
- Une "sonde de détection" est une sonde marquée au moyen d'un agent marqueur choisi par exemple parmi les isotopes radioactifs, les enzymes, en particulier les enzymes susceptibles d'agir sur un substrat chromogène, fluorigène ou luminescent (notamment une peroxydase ou une phosphatase alcaline), les composés chimiques chromophores, les composés chromogènes, fluorigènes ou luminescents, les analogues des bases nucléotidiques et les ligands tels que la biotine.
- Une "sonde d'espèce" est une sonde permettant l'identification spécifique de l'espèce d'une bactérie.
- Une "sonde de genre" est une sonde permettant l'identification spécifique du genre d'une bactérie.
- Une "amorce" est une sonde comprenant par exemple 10 à 100, de préférence 12 à 35, de préférence encore 18 à 30 motifs nucléotidiques et possédant une spécificité d'hybridation dans des conditions déterminées pour les réactions d'amplification enzymatique.
- Par "amplification" ou "réaction d'amplification", on entend une réaction de polymérisation enzymatique, par exemple dans une technique

d'amplification telle que la PCR, initiée par des oligonucléotides amorces et utilisant une ADN polymérase.
- Par "séquençage" ou "réaction de séquençage", on entend l'obtention de la séquence d'un fragment d'acide nucléique ou d'un gène complet par la technique de Sanger, à savoir un procédé de polymérisation abortive à partir d'amorces oligonucléotidiques et utilisant lesdits didésoxynucléotides [Sanger F, Coulson AR. A rapid method for determining sequences in DNA by primed synthesis with DNA polymerase. J.Mol.Biol. 1975 ; 94 : 441] ou séquençage par incoporation sucessive de bases avec libération de pyrophosphate dit "pyroséquençage" [Diggle MA, Clarke SC. Related Articles, Links Pyrosequencing: sequence typing at the speed of light. Mol Biotechnol. 2004 ; 28:129-37] ou hybridations multiples avec des sondes multiples fixées sur support solide telles qu'utilisées dans les puces ADN par exemple.
- par "génotypage", on entend une méthode permettant d'identifier une souche bactérienne donnée par la caractérisation de positions nucléotidiques spécifiques,
- par "taux de similarité", on entend un pourcentage d'identité de séquence, c'est-à-dire un pourcentage de nucléotides de la séquence qui sont identiques et à la même position entre deux séquences, les deux séquences étant alignées à partir de leurs extrémités 5',
- par "séquence spécifique d'une bactérie d'une espèce donnée ou d'une bactérie d'un genre donné", on entend que ladite séquence peut constituer une sonde ou amorce d'espèce ou respectivement sonde ou amorce de genre, c'est-à-dire capable de s'hybrider avec ou respectivement d'amplifier uniquement le génome d'une dite bactérie de ladite espèce ou dudit genre dans certaines conditions d'hybridation ou respectivement d'amplification.

La présente description fournit des séquences d'acides nucléiques permettant l'identification de chaque espèce du complexe *Mycobacterium tuberculosis,* et le génotypage de chaque souche au sein de chaque espèce du complexe *Mycobacterium tuberculosis* et, notamment, de l'espèce *Mycobacterium tuberculosis.*

Pour ce faire, est fourni un fragment isolé d'acide nucléique de zone intergénique non codante d'une bactérie du complexe *Mycobacterium tuberculosis* choisi parmi les fragments d'acides nucléiques des 16 zones intergéniques MST1 à MST16 amplifiables par des couples d'amorces constituées par des oligonucléotides de séquences consistant en au moins 10 nucléotides consécutifs, de préférence au moins 18 nucléotides consécutifs, tirés des séquences SEQ. ID n°1 à 32, ou de préférence encore consistant dans lesdites séquences SEQ. ID. n°1 à 32 suivantes du listage de séquences et leurs séquences complémentaires, avec les couples d'amorces 5', 3', tirés des séquences suivantes pour les fragments suivants :
- pour lé fragment de la zone intergénique MST1 :
   - amorce 5'= SEQ. ID. n°1
   - amorce 3'= SEQ. ID. n°2
- pour le fragment de la zone intergénique MST2 :
   - amorce 5'= SEQ. ID. n°3
   - amorce 3'= SEQ. ID. n°4
- pour le fragment de la zone intergénique MST3 :
   - amorce 5'= SEQ. ID. n°5
   - amorce 3'= SEQ. ID. n°6
- pour le fragment de la zone intergénique MST4 :
   - amorce 5'= SEQ. ID. n°7
   - amorce 3'= SEQ. ID. n°8
- pour le fragment de la zone intergénique MST5 :
   - amorce 5'= SEQ. ID. n°9
   - amorce 3'= SEQ. ID. n°10
- pour le fragment de la zone intergénique MST6 :
   - amorce 5'= SEQ. ID. n°1
   - amorce 3'= SEQ. ID. n°12
- pour le fragment de la zone intergénique MST7 :
   - amorce 5'= SEQ. ID. n°13
   - amorce 3'= SEQ. ID. n° 14
- pour le fragment de la zone intergénique MST8 :
   - amorce 5'= SEQ. ID. n°15
   - amorce 3'= SEQ. ID. n°16
- pour le fragment de la zone intergénique MST9 :
   - amorce 5'= SEQ. ID. n°17
   - amorce 3'= SEQ. ID. n°18
- pour le fragment de la zone intergénique MST10 :
   - amorce 5'= SEQ. ID. n°19
   - amorce 3'= SEQ. ID. n°20
- pour le fragment de la zone intergénique MST11 :
   - amorce 5'= SEQ. ID. n°21
   - amorce 3'= SEQ. ID. n°22
- pour le fragment de la zone intergénique MST12 :
   - amorce 5'= SEQ. ID. n°23
   - amorce 3'= SEQ. ID. n°24
- pour le fragment de la zone intergénique MST13 :
   - amorce 5'= SEQ. ID. n°25
   - amorce 3'= SEQ. ID. n°26
- pour le fragment de la zone intergénique MST14 :
   - amorce 5'= SEQ. ID. n°27
   - amorce 3'= SEQ. ID. n°28
- pour le fragment de la zone intergénique MST15 :
   - amorce 5'= SEQ. ID. n°29
   - amorce 3'= SEQ. ID. n°30
- pour le fragment de la zone intergénique MST16 :
   - amorce 5'= SEQ. ID. n°31
   - amorce 3'= SEQ. ID. n°32

On comprend que le signe "=" signifie ici que l'amorce 5', 3', mentionnée est tirée de la séquence SEQ. ID. n°i mentionnée ci-dessus.

Les amorces ci-dessus permettent d'amplifier les fragments respectivement des zones intergéniques MST1 à MST16 comme indiqué et ce, pour toutes les bactéries du complexe *Mycobacterium tuberculosis.*

Dans la méthode d'identification selon la présente invention explicitée ci-après, ces fragments permettent l'identification d'espèces au sein du complexe *Mycobacterium tuberculosis*, à savoir : *Mycobacterium tuberculosis* ; *Mycobacterium africanum,* qui comporte deux sous-types I et II ; *Mycobacterium canettii; Mycobacterium bovis* (qui comporte trois sous-espèces, *Mycobacterium bovis,* ssp. bovis, *Mycobacterium bovis,* ssp. *caprae* et *Mycobacterium bovis* souches vaccinales BCG et *Mycobacterium microti.* Mais, en pratique, les infections humaines sont principalement dues à *Mycobacterium tuberculosis* et plus rarement *Mycobacterium africanum, Mycobacterium bovis* ssp *bovis* et les souches vaccinales BCG de *Mycobacterium bovis.*

Plus particulièrement, est fourni un fragment isolé d'acide nucléique de zone intergénique non codante d'une bactérie du complexe *Mycobacterium tuberculosis* choisi parmi les fragments d'acides nucléiques des 16 zones intergéniques MST1 à MST16 selon l'invention, caractérisé en ce qu'il présente une séquence choisie parmi les séquences SEQ. ID. n°33 à 79 du listage de séquences et leurs séquences complémentaires, avec les séquences suivantes pour les espèces suivantes :
- pour l'espèce *Mycobacterium tuberculosis* :
   les séquences du fragment de la zone intergénique MST1, consistant dans les 4 séquences SEQ. ID. n°52 à 55,
   • les séquences du fragment de la zone intergénique MST2, consistant dans les 3 séquences SEQ. ID. n°56 à 58,
   • les séquences du fragment de la zone intergénique MST3, consistant dans les 2 séquences SEQ. ID. n°59 à 60,
   • les séquences du fragment de la zone intergénique MST4, consistant dans les 5 séquences SEQ. ID. n°61 à 65,
   • les séquences du fragment de la zone intergénique MST8, consistant dans les 3 séquences SEQ. ID. n°66 à 68,
   • les séquences du fragment de la zone intergénique MST11, consistant dans les 4 séquences SEQ. ID. n°33 à 36,
   • les séquences du fragment de la zone intergénique MST12, consistant dans les 7 séquences SEQ. ID. n°41 à 47,
   • les séquences du fragment de la zone intergénique MST13, consistant dans les 11 séquences SEQ. ID. n°69 à 79,
- pour *Mycobacterium africanum*
   - la séquence du fragment de la zone intergénique MST11 consistant dans SEQ. ID. n°37, et
   - la séquence du fragment de la zone intergénique MST12 consistant dans SEQ. ID. n°48,
- pour *Mycobacterium bovis ssp bovis* :
   - la séquence du fragment de la zone intergénique MST11 consistant dans SEQ. ID. n°38, et
   - la séquence du fragment de la zone intergénique MST12 consistant dans SEQ. ID. n°49,
- pour *Mycobacterium bovis, ssp BCG* :
   - la séquence du fragment de la zone intergénique MST11 consistant dans SEQ. ID. n°39, et
   - la séquence du fragment de la zone intergénique MST12 consistant dans SEQ. ID. n°50.
- pour *Mycobacterium canettii* :
   - la séquence du fragment de la zone intergénique MST11 consistant dans SEQ. ID. n°40, et
   - la séquence du fragment de la zone intergénique MST12 consistant dans SEQ. ID. n°51.

Les séquences SEQ. ID. n°33 à 79 ci-dessus sont amplifiables par les couples d'amorces indiqués précédemment pour les fragments de zones intergéniques MST1 à MST 16 concernées.

La présente description a également pour objet un oligonucléotide isolé apte à servir d'amorce d'amplification d'un fragment d'acide nucléique de dite zone intergénique choisi parmi les fragments d'acide nucléique des 16 zones intergéniques MST1 à MST16 selon l'invention, caractérisé en ce qu'il présente une séquence comprenant 12 à 35, de préférence de 18 à 30 nucléotides consécutifs situés à l'une des extrémités 5' ou 3' des séquences desdits fragments de zones intergéniques MST1 à MST116.

Plus particulièrement, la présente description fournit un oligonucléotide isolé amorce selon l'invention, caractérisé en ce qu'il présente une séquence comprenant 12 à 35, de préférence 18 à 30 nucléotides consécutifs inclus dans l'une des séquences SEQ. ID. n°1 à 32, de préférence consistant dans l'une des séquences SEQ. ID. n°1 à 32 du listage de séquences et leurs séquences complémentaires.

La présente description fournit donc, plus particulièrement, des oligonucléotides présentant des séquences tirées des séquences SEQ. ID. n°33 à 79 et leurs séquences complémentaires situées aux extrémités 5' et 3' desdites séquences SEQ. ID. n°33 à 79, ledit oligonucléotide étant apte à servir d'amorce d'amplification desdites séquences SEQ. ID. n°33 à 79.

Plus particulièrement encore, la présente description fournit des oligonucléotides isolés présentant des séquences nucléotidiques consistant dans 12 à 35 nucléotides consécutifs, de préférence 18 à 30, inclus dans l'une des séquences SEQ. ID. n°1 à 16 et 21 à 26 et leurs séquences complémentaires, avec les séquences suivantes pour les fragments amplifiés suivants, lesdits fragments étant amplifiés par les amorces 5', 3' mentionnées précédemment :
- pour le fragment de la zone intergénique MST1 : SEQ. ID n°52 à 55
- pour le fragment de la zone intergénique MST2 : SEQ. ID n°56 à 58
- pour le fragment de la zone intergénique MST3 : SEQ. ID n°59 à 60
- pour le fragment de la zone intergénique MST4 : SEQ. ID n°61 à 65
- pour le fragment de la zone intergénique MST8 : SEQ. ID n°66 à 68
- pour le fragment de la zone intergénique MST11 : SEQ. ID n°33 à 40
- pour le fragment de la zone intergénique MST12 : SEQ. ID n°41 à 51
- pour le fragment de la zone intergénique MST13 : SEQ. ID n°69 à 79

La présente description a également pour objet un oligonucléotide isolé présentant une séquence tirée des séquences des fragments selon l'invention, de préférence une séquence tirée des séquences SEQ. ID. n°33 à 79 et leurs séquences complémentaires, de préférence comprenant 12 à 35, de préférence encore 18 à 30 nucléotides consécutifs inclus dans lesdites séquences, caractérisé en ce que ladite séquence nucléotidique dudit oligonucléotide inclut une séquence spécifique d'une bactérie, d'une espèce, sous espèce, sous type ou génotype donné(e) du complexe *Mycobacterium tuberculosis,* utilisable à titre de sonde d'une dite bactérie.

Les sondes selon l'invention peuvent être utilisées, à des fins de diagnostic, comme mentionné précédemment, par la détermination de la formation ou de l'absence de formation d'un complexe d'hybridation entre la sonde et un acide nucléique cible dans un échantillon, selon toutes les techniques d'hybridation connues et notamment les techniques de dépôt ponctuel sur filtre, dites " DOT-BLOT " [Maniatis et al. (1982) Molecular Cloning, Cold Spring Harbor], les techniques de transfert d'ADN dites " SOUTHERN BLOT " [Southern EM. Detection of specific séquences among DNA fragments separated by gel electrophoresis. J Mol Biol. 1975 5 ; 98:503-17], les techniques de transfert d'ARN dites " NOTHERN BLOT ", ou les techniques dites " sandwich ", en particulier avec une sonde de capture et/ou une sonde de détection, lesdites sondes étant capables de s'hybrider avec deux régions différentes de l'acide nucléique cible, et l'une au moins desdites sondes (généralement la sonde de détection étant capable de s'hybrider avec une région de la cible qui est spécifique de l'espèce, étant entendu que la sonde de capture et la sonde de détection doivent avoir des séquences nucléotidiques au moins partiellement différentes.

L'acide nucléique à détecter (cible) peut être de l'ADN ou de l'ARN (le premier obtenu après amplification par PCR). Dans le cas de la détection d'une cible de type acide nucléique double brin, il convient de procéder à la dénaturation de ce dernier avant la mise en oeuvre du procédé de détection. L'acide nucléique cible peut être obtenu par extraction selon les méthodes connues des acides nucléiques d'un échantillon à examiner. La dénaturation d'un acide nucléique double brin peut être effectuée par les méthodes connues de dénaturation chimique, physique ou enzymatique, et en particulier par chauffage à une température appropriée, supérieure à 80°C.

Pour mettre en oeuvre les techniques d'hybridation précitées, et en particulier les techniques "sandwich", une sonde de l'invention, appelée sonde de capture est immobilisée sur un support solide, et une autre sonde de l'invention, appelée sonde de détection, est marquée avec un agent marqueur. Les exemples de support et d'agent marqueur sont tels que définis précédemment.

De manière avantageuse, une sonde d'espèce est immobilisée sur un support solide, et une autre sonde d'espèce est marquée par un agent marqueur.

Un oligonucléotide de l'invention utilisé comme amorce nucléotidique comprenant une oligonucléotide monocaténaire choisie parmi les oligonucléotides ayant une séquence d'au moins 12 motifs nucléotidiques inclus dans l'une des séquences SEQ. ID. n°1 à 32, est utilisable dans la synthèse d'un acide nucléique en présence d'une polymérase par un procédé connu en soi, notamment dans des méthodes d'amplification utilisant une telle synthèse en présence d'une polymérase (PCR, retro-transcription-PCR, real-time PCR, etc.).

Selon un cas particulier ladite amorce comprenant un oligonucléotide de l'invention comprend en outre la séquence sens ou anti-sens d'un promoteur reconnu par une ARN polymérase (promoteurs T7, T3, SP6 par exemple [Studier FW, Moffatt BA. Use of bacteriophage T7 RNA polymerase to direct selective high-level expression of cloned gènes. J Mol Biol. 1986 May 5;189(1):113-30] : de telles amorces sont utilisables dans des procédés d'amplification d'acide nucléique faisant intervenir une étape de transcription, tels que, par exemple, les techniques NASBA ou 3SR [Van Gemen B. et al. Abstract MA 1091, 7th International Conférence on AIDS (1991) Florence, Italy].

On peut tirer des différentes séquences issues des fragments d'acide nucléique correspondant aux dites zones intergéniques MST1-MST16, des pluralités d'oligonucléotides sondes correspondant aux différents sites discriminants dans les différents fragments desdites zones intergéniques MST1 à MST16, différents oligonucléotides sondes étant présentés sous forme de "biopuces", c'est-à-dire fixés sur des supports solides, notamment en verre, selon le procédé décrit dans le brevet US 5 744 305 (Affymetrix, Fodor et al) en utilisant la stratégie de reséquençage décrite dans la demande WO 95/11995 (Affymax, Chee et al) ou selon la méthode décrite par A Troesch et al. [A Troesch et al. dans J. Clin. Microbiol., vol. 37(1), p 49-55, 1999]. Les oligonucléotides synthétisés sur la "biopuce" réalisent le re-séquençage de chacun desdits "spacers", c'est-à-dire la description de la séquence du spacer basée non pas sur le séquençage direct de ce spacer, mais sur l'analyse des hybridations permettant de retrouver sa séquence antérieurement décrite (re-séquençage). Ce procédé présente un avantage considérable en terme de coût de production et sans compromis sur la qualité de l'identification des différentes espèces et des différents génotypes de l'espèce *Mycobacterium tuberculosis* de part le choix de ces séquences d'identification. De préférence, ces oligonucléotides fixés sur le support solide de la "biopuce" comportent de 10 à 30 bases par exemple 20 bases, avec une position d'interrogation située dans la région centrale comme par exemple en 12ème position par rapport à l'extrémité 3' de la séquence pour des oligonucléotides de 20 bases. Un autre exemple consiste à utiliser des oligonucléotides de 17 bases, avec 2 positions d'interrogations : une en 10ème et une en 8ème position. D'autres oligonucléotides ont des longueurs comprises entre 10 et 25 nucléotides. Les positions d'interrogation varient alors en fonction de la longueur de l'oligonucléotide.

L'analyse est effectuée sur le système complet GeneChip® (référence 900228, Affymetrix, Santa Clara, CA) qui comprend le lecteur GeneArray®, le four d'hybridation GeneChip®, la station fluidique GeneChip® et le logicel d'analyse GeneChip®.

La présente invention fournit également un procédé d'identification d'une bactérie du complexe *Mycobacterium tuberculosis* permettant l'identification de l'espèce, le cas échéant la sous espèce ou le sous type de ladite bactérie, et le cas échéant permettant le génotypage des dites espèces, sous espèces et sous types de bactéries dans un échantillon biologique contenant des acides nucléiques d'une dite bactérie dans lequel on effectue les étapes suivantes dans lesquelles:
1) on réalise l'amplification enzymatique de type PCR dans l'acide nucléique dudit échantillon, d'au moins 1 fragment d'acide nucléique d'une zone intergénique choisie parmi l'une des 2 dites zones intergéniques MST11 et MST 12, à l'aide d'un couple d'amorces aptes à amplifier ledit fragment, ledit couple d'amorces étant constitué par des oligonucléotides de séquences tirées des séquences SEQ. ID. n°21 à 24 suivantes du listage de séquences et leurs séquences complémentaires :
   - pour le fragment de la zone intergénique MST11 :
      - amorce 5'= SEQ. ID. n°21
      - amorce 3'= SEQ. ID. n°22
   - pour le fragment de la zone MST12 :
      - amorce 5' : SEQ. ID. n°23
      - amorce 3' : SEQ. ID. n°24
2) on réalise le séquençage du fragment amplifié à l'étape 1), et
3) on identifie l'espèce, la sous espèce ou le sous type de ladite bactérie, en comparant la séquence obtenue à l'étape 2) avec les séquences d'une banque de données des séquences d'au moins ledit fragment d'acide nucléique d'une dite zone intergénique choisie parmi les 2 dites zones intergéniques MST11 et MST 12 respectivement des différentes espèces, sous espèces et sous type de bactéries du complexe *Mycobacterium tuberculosis.*

Ladite banque de séquences est donc constituée à partir d'une collection d'isolats de souche appartenant à une espèce, sous espèce, sous type ou génotype connu ou précédemment déterminé.

Plus particulièrement, à l'étape 3), lesdites séquences constituant ladite banque ont été déterminées par amplification et séquençage de dit(s) fragment(s) de zone(s) intergénique(s) des différentes bactéries d'une collection de différents isolats de bactéries de différentes espèces, sous espèces et sous types de bactéries du complexe *Mycobacterium tuberculosis* dont les espèces, sous espèces et sous types et, le cas échéant, génotypes, sont connus.

Avantageusement, dans un procédé selon l'invention, on identifie une espèce, sous espèce ou sous type de bactérie parmi les bactéries *Mycobacterium tuberculosis, Mycobacterium bovis ssp bovis, Mycobacterium bovis, ssp BCG, Mycobacterium bovis ssp caprae, Mycobacterium africanum sous type I, Mycobacterium canettii, Mycobacterium microti.*

De préférence, on identifie une espèce sous espèce ou sous type de bactérie parmi les bactéries *Mycobacterium tuberculosis, Mycobacterium bovis ssp bovis, Mycobaclerium bovis ssp BCG, Mycobacterium africanum sous type I, Mycobacterium canettii.*

Plus particulièrement, à l'étape 3), lesdites séquences de ladite banque comprennent les séquences SEQ. ID. n°33 à 51 suivantes du listage de séquences et leurs séquences complémentaires pour les espèces suivantes :
- pour *Mycobacterium tuberculosis* :
   - les séquences du fragment de la zone intergénique MST11 consistant dans l'une des séquences SEQ. ID. n°33 à 36,
   - les séquences du fragment de la zone intergénique MST112 consistant dans l'une des séquences SEQ. ID. n°41 à 47,
- pour *Mycobacterium africanum* :
   - la séquence du fragment de la zone intergénique MST11 consistant dans SEQ. ID. n°37,
   - la séquence du fragment de la zone intergénique MST12 consistant dans SEQ. ID. n°48,
- pour *Mycobacterium bovis ssp bovis* :
   - la séquence du fragment de la zone intergénique MST11 consistant dans SEQ. ID. n°38,
   - la séquence du fragment de la zone intergénique MST12 consistant dans SEQ. ID. n°49,
- pour *Mycobacterium bovis ssp BCG* :
   - la séquence du fragment de la zone intergénique MST11 consistant dans SEQ. ID. n°39,
   - la séquence du fragment de la zone intergénique MST12 consistant dans SEQ. ID. n°50,
- pour *Mycobacterium canettii* :
   - la séquence du fragment de la zone intergénique MST11 consistant dans SEQ. ID. n°40,
   - la séquence du fragment de la zone intergénique MST12 consistant dans SEQ. ID. n°51,

Dans un autre mode de réalisation d'un procédé selon l'invention, on identifie le génotype d'une espèce, sous espèce ou sous type donné d'une bactérie parmi les bactéries du complexe *Mycobacterium tuberculosis,* en réalisant les étapes dans lesquelles :
a) à l'étape 1) on réalise l'amplification enzymatique de type PCR dans l'acide nucléique dudit échantillon, d'au moins 8 fragments d'acides nucléiques d'au moins 8 zones intergéniques choisies parmi 16 dites zones intergéniques MST1 à MST16 dont les 2 zones intergéniques. MST11 et MST12, à l'aide respectivement des au moins 8 dits couples d'amorces aptes à amplifier les dits fragments des au moins 8 dites zones intergéniques, lesdites amorces étant constituées par des oligonucléotides de séquences comprenant 12 à 35 nucléotides consécutifs, de préférence 18 à 30, inclus ou de préférence encore consistant dans les séquences suivantes du listage de séquences et leurs séquences complémentaires, avec les couples d'amorces 5', 3', tirés des séquences suivantes pour les fragments suivants :
   - pour le fragment de la zone intergénique MST1 :
      - amorce 5'= SEQ. ID. n°1
      - amorce 3'= SEQ. ID. n°2
      - pour le fragment de la zone intergénique MST2 :
      - amorce 5'= SEQ. ID. n°3
      - amorce 3'= SEQ. ID. n°4
   - pour le fragment de la zone intergénique MST3 :
      - amorce 5'= SEQ. ID. n°5
      - amorce 3'= SEQ. ID. n°6
   - pour le fragment de la zone intergénique MST4 :
      - amorce 5'= SEQ. ID. n°7
      - amorce 3'= SEQ. ID. n°8
   - pour le fragment de la zone intergénique MST5 :
      - amorce 5'= SEQ. ID. n°9
      - amorce 3'= SEQ. ID. n° 10
   - pour le fragment de la zone intergénique MST6 :
      - amorce 5'= SEQ. ID. n°11
      - amorce 3'= SEQ. ID. n° 12
   - pour le fragment de la zone intergénique MST7 :
      - amorce 5'= SEQ. ID. n° 13
      - amorce 3'= SEQ. ID. n° 14
   - pour le fragment de la zone intergénique MST8 :
      - amorce 5'= SEQ. ID. n°15
      - amorce 3'= SEQ. ID. n°16
   - pour le fragment de la zone intergénique MST9 :
      - amorce 5'= SEQ. ID. n° 17
      - amorce 3'= SEQ. ID. n° 18
   - pour le fragment de la zone intergénique MST10 :
      - amorce 5'= SEQ. ID. n° 19
      - amorce 3'= SEQ. ID. n°20
   - pour le fragment de la zone intergénique MST11 :
      - amorce 5'= SEQ. ID. n°21
      - amorce 3'= SEQ. ID. n°22
   - pour le fragment de la zone intergénique MST12 :
      - amorce 5'= SEQ. ID. n°23
      - amorce 3'= SEQ. ID. n°24
   - pour le fragment de la zone intergénique MST13 :
      - amorce 5'= SEQ. ID. n°25
      - amorce 3'= SEQ. ID. n°26
   - pour le fragment de la zone intergénique MST14 :
      - amorce 5'= SEQ. ID. n°27
      - amorce 3'= SEQ. ID. n°28
   - pour le fragment de la zone intergénique MST15 :
      - amorce 5'= SEQ. ID. n°29
      - amorce 3'= SEQ. ID. n°30
   - pour le fragment de la zone intergénique MST16 :
      - amorce 5'= SEQ. ID. n°3
      - amorce 3'= SEQ. ID. n°32
   , et
b) à l'étape 2) on réalise le séquençage des fragments amplifiés à l'étape a), et
c) à l'étape 3) on identifie en outre le génotype de l'espèce, sous espèce ou sous type de dite bactérie en comparant les séquences des fragments amplifiés obtenues à l'étape b) avec les séquences d'une banque de données des séquences des au moins 8 dits fragments d'acides nucléiques des au moins 8 dites zones intergéniques choisie parmi 16 dites zones intergéniques des différents génotypes de dites bactéries du complexe *Mycobacterium tuberculosis,* les différents génotypes correspondant aux différentes combinaisons possibles des au moins 8 séquences des au moins 8 dits fragments d'acide nucléique des au moins 8 dites zones intergéniques, les séquences constituant ladite banque ayant été déterminées par amplification et séquençage desdits au moins 8 fragments des au moins 8 dites zones intergéniques des différentes bactéries d'une collection de différents isolats de bactéries de ladite espèce, sous espèce ou sous type connues du complexe Mycobacterium tuberculosis, les différents génotypes correspondant à des combinaisons différentes des au moins 8 séquences desdites au moins 8 zones intergéniques des bactéries de ladite collection.

On entend ici par "combinaisons différentes de séquences" que dans chaque combinaison l'une au moins des séquences d'une dite zone intergénique est différente, chaque combinaison comprenant autant de séquences que de fragments de dites zones intergéniques choisies parmi MST1 à MST16 prises en considération.

Plus particulièrement, dans ce procédé selon l'invention, lesdits fragments des au moins 8 zones intergéniques sont les 8 fragments desdites zones intergéniques MST1, MST2, MST3, MST4, MST8, MST11, MST12 et MST13, amplifiables par leurs dits couples d'amorces respectives de séquences tirées des séquences SEQ. ID. n°1 à 16 et 21 à 26, avec:
- pour le fragment de la zone intergénique MST1 :
   - amorce 5'= SEQ. ID. n°1
   - amorce 3'= SEQ. ID. n°2
- pour le fragment de la zone intergénique MST2 :
   - amorce 5'= SEQ. ID. n°3
   - amorce 3'= SEQ. ID. n°4
- pour le fragment de la zone intergénique MST3 :
   - amorce 5'= SEQ. ID. n°5
   - amorce 3'= SEQ. ID. n°6
- pour le fragment de la zone intergénique MST4 :
   - amorce 5'= SEQ. ID. n°7
   - amorce 3'= SEQ. ID. n°8
- pour le fragment de la zone intergénique MST8 :
   - amorce 5'= SEQ. ID. n°15
   - amorce 3'= SEQ. ID. n°16
- pour le fragment de la zone intergénique MST11 :
   - amorce 5'= SEQ. ID. n°21
   - amorce 3'= SEQ. ID. n°22
- pour le fragment de la zone intergénique MST12 :
   - amorce 5'= SEQ. ID. n°23
   - amorce 3'= SEQ. ID. n°24
- pour le fragment de la zone intergénique MST13 :
   - amorce 5'= SEQ. ID. n°25
   - amorce 3'= SEQ. ID. n°26

On identifie donc le génotype d'une bactérie de l'espèce *Mycobacterium tuberculosis* par amplification et séquençage des fragments des au moins 8 zones intergéniques MST1, MST2, MST3, MST4, MST8, MST11, MST12 et MST13 de la bactérie à identifier, puis comparaison avec les séquences d'une dite banque de séquences des fragments desdites 8 zones intergéniques. On comprend qu'à partir de ladite banque de séquences, on peut discriminer différents génotypes correspondant aux différentes combinaisons possibles de 8 dites séquences de fragments MST1, MST2, MST3, MST4, MST8, MST11, MST12 et MST13.

Plus particulièrement encore, lesdites séquences de ladite banque comprennent les séquences SEQ. ID. n°33 à 79 du listage de séquences et leurs séquences complémentaires pour les fragments de zones intergéniques MST1 à MST16 suivants :
- pour le fragment de la zone intergénique MST1, les 4 séquences SEQ. ID. n°52 à 55
- pour le fragment de la zone intergénique MST2, les 3 séquences SEQ. ID. n° 56 à 58
- pour le fragment de la zone intergénique MST3, les 2 séquences SEQ. ID. n°59 à 60
- pour le fragment de la zone intergénique MST4, les 5 séquences SEQ. ID. n°61 à 65
- pour le fragment de la zone intergénique MST8, les 3 séquences SEQ. ID. n°66 à 68
- pour le fragment de la zone intergénique MST11, les 4 séquences SEQ. ID. n°33 à 36
- pour le fragment de la zone intergénique MST12, les 7 séquences SEQ. ID. n°41 à 47
- pour le fragment de la zone intergénique MST13, les 11 séquences SEQ. ID. n°69 à 79.

Dans un mode préféré de réalisation des procédés selon l'invention, on vérifie au préalable que l'échantillon sur lequel on réalise ladite identification, contient effectivement des acides nucléiques d'une dite bactérie du complexe *Mycobacterium tuberculosis.*

Pour ce faire, on peut réaliser un examen direct sur la méthode dite Ziehl ou par culture.

Dans un mode préféré de réalisation, on réalise une identification moléculaire de la manière suivante :
a- on réalise l'amplification du fragment d'acide nucléique d'une zone intergénique 16S-23S appelée ITS, avec des amorces suivantes :
   - amorce 5' : 5'-GGGTGGGGTGTGGTGTTTGA-3' (SEQ. ID. n°80) - amorce 3' : 5'- CAAGGCATCCACCATGCGC-3' (SEQ. ID. n°81)
   et on vérifie la présence d'une bactérie du genre *Mycobacterium* par hybridation avec une sonde de genre de séquence 5'-TGGATAGTGGTTGCGAGCATC-3' (SEQ. ID. n°82), et
b- si le résultat de l'étape a- est positif, on vérifie que ladite bactérie du genre *Mycobacterium* est bien une bactérie du complexe *Mycobacterium tuberculosis* par hybridation avec une sonde spécifique du complexe de séquence nucléotidique suivantes 5'-GCTAGCCGGCAGCGTATCCAT-3' (SEQ. ID. n°83),

De préférence après une amplification des acides nucléiques de l'échantillon à tester en présence des mêmes amorces qu'à l'étape a-.

Cette méthode d'identification d'une bactérie du complexe *Mycobacterium tuberculosis* est décrite dans les références [Bruijnesteijn Van Coppenraet ES, Lindeboom JA, Prins JM, Peeters MF, Claas EC, Kuijper EJ. Real-time PCR assay using fine-needle aspirates and tissue biopsy specimens for rapid diagnosis of mycobacterial lymphadenitis in children J Clin Microbiol. 2004 Jun; 42(6):2644-50)].

Les différentes séquences d'acides nucléiques décrites dans la présente description sont identifiées dans le listage de séquences annexé à la présente description, dans les séquences SEQ. ID. n°1 à 83.

La présente invention fournit des fragments d'acide nucléique des 8 zones MST1, MST2, MST3, MST4, MST8, MST11, MST12 et MST13 de séquences SEQ. ID. n°33 à 79, telles que définies dans les revendications 11 et 12, ainsi que des oligonucléotides aptes à servir d'amorces d'amplification desdits fragments d'acide nucléiques, consistant dans les séquences SEQ. ID. n°1 à 8, 15-16 et 21 à 26, telles que définies dans la revendication 10.

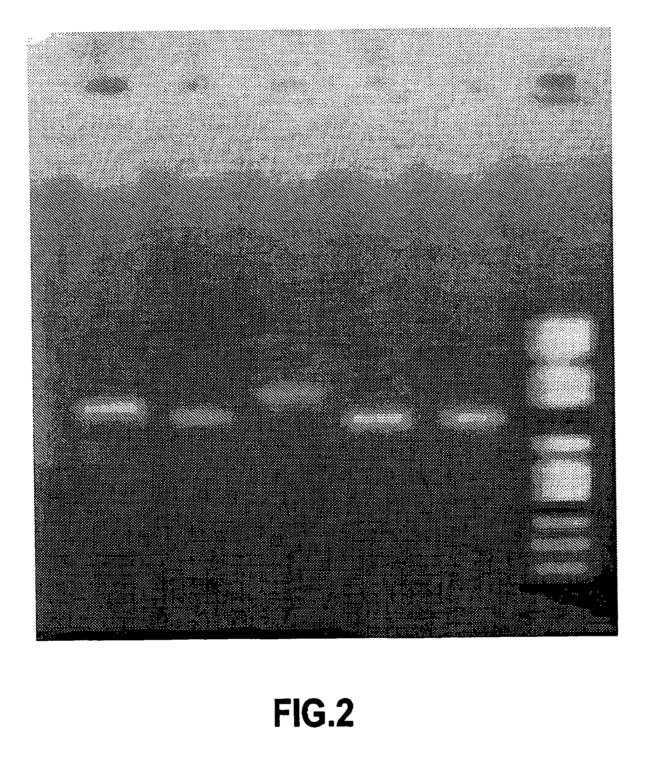

L'invention sera mieux comprise à l'aide de la description détaillée sous forme de deux exemples de réalisation faite en référence aux figures 1 et 2, dans lesquelles :
la figure 1 représente un profil d'électrophorèse en gel d'Agarose des amplifiats obtenus par PCR utilisant les amorces des zones intergéniques MST11 avec les espèces du complexe *Mycobacterium tuberculosis, respectivement de gauche à droite M. tuberculosis, M. bovis* ssp *bovis, M. africanum, M. Canetti et M. bovis ssp BCG,* et
la figure 2 représente un profil d'électrophorèse en gel d'Agarose des amplifiats obtenus par PCR utilisant les amorces des zones intergéniques MST12 avec les espèces du complexe *Mycobacterium tuberculosis, respectivement de gauche à droite M. tuberculosis, M. bovis* ssp *bovis, M. africanum, M. Canetti et M. bovis ssp BCG).*

### Exemple 1. Génotypage d'isolats de Mycobacterium tuberculosis.

Les inventeurs ont réalisé l'alignement de deux génomes entiers de *Mycobacterium tuberculosis:*
- la souche H37Rv (GenBank accession number AL 123456) [Cole, ST., Broch, R., Parkhill, J., Garnier, T., Churcher, C., Harris, D., Gordon, SV., Eiglmeier, K., Gas, S., Barry, CE., Tekaia, F., Badcock, K., Basham, D., Brown, D., Chillingworth, T., Connor, R., Davies, R., and BG. Barrel. 1998. Deciphering the biology of Mycobacterium tuberculosis from the complete genome séquence. Nature. 6685: 537-544] qui servira de séquence de référence dans cet exemple, et
- la souche CDC1551 (Gen Bank accession number AE 000516) [Fleischmann, RD., Alland, D., Eisen, JA., Carpenter, L., White, O., Peterson, J., DeBoy, R., Dodson, R., Gwinn, M., Haft, D., Hickey, E., Kolonay, JF., Nelson, WC., Umayam, LA., Ermolaeva, M., Salzberg, SL., Delcher, A., Utterback, T., Weidman, J., Khouri, H., Gill, J., Mikula, A., Bishai, W., Jacobs, Jr., Venter, JC., and CM. Fraser. 2002. Whole-genome comparison of Mycobacterium tuberculosis clinical and laboratory strains. J Bacteriol. 184:5479-90].

Des fragments de zones intergéniques appelés MST1. à MST 16 ou "spacers" ont été déterminés, répondant aux critères suivants:
1) taille du "spacer" chez les 2 souches de référence ≤ 600 pb; cette longueur de séquence étant choisie pour être compatible avec les capacités des séquenceurs capillaires ayant une capacité de séquençage de 800 paires de bases, auxquelles il convient de retrancher 100 paires de bases à chaque extrémité correspondant à la longueur des amorces et à la zone d'ombre de mauvaise qualité de séquence en aval de l'amorce nucléotidique;
2) similarité des séquences des "spacers" entre les deux génomes comprise entre 70% et 99% ; la valeur basse de 70% étant choisie afin de garantir dans la première phase de recherche un minimum de stringence garantissant l'homologie du spacer entre les deux génomes pris pour référence; la valeur haute de 99% étant choisie afin de garantir un minimum de variabilité de séquence du spacer entre les différentes souches testées;
3) les "spacers" ne correspondant pas aux régions génomiques précédemment utilisées pour le génotypage par VNTR, ou MIRU, ou spoligotyping.

Il a été ainsi possible de déterminer 16 fragments de zones intergéniques appelés MST1-MST16 présentant les 3 critères mentionnés. La localisation de ces 16 "spacers" MST1-MST16 sur le chromosome de l'espèce *Mycobacterium tuberculosis* souche H37Rv (GenBank accession number AL 123456) est présentée dans le tableau 1 ci-après.

Dans le tableau 1 ci-après, la position des 16 "spacers" MST1-MST16 est présentée par rapport aux gènes en amont et aval.

Les inventeurs ont ensuite extrait de chaque séquence de spacer une séquence d'au maximum 350 paires de bases afin de désigner pour chaque spacer une paire d'amorces d'amplification par PCR à l'aide du logiciel Pimer3 software program (INFOBIOGEN, Evry, France). La séquence de ces amorces est présentée dans le tableau 2 suivant.

Ces amorces ont été utilisées pour l'amplification des 16 "spacers" identifiés selon les modalités suivantes : 20 isolats de *Mycobacterium tuberculosis* choisis au hasard dans une collection de 105 isolats cliniques dont la nature et l'origine sont explicité au tableau 3, ont été inactivés selon un protocole publié par deux des inventeurs [Djelouadji, Z. and M. Drancourt. 2006. Inactivation of cultured Mycobacterium tuberculosis organisms prior to DNA extraction. J. Clin. Microbiol. 44:1594-1595]. Après extraction de l'ADN de chaque isolat selon les recommendations du fournisseur (Qiagen, Courtaboeuf, France), les PCRs ont été réalisées sous un volume final de 50 µl contenant 33 µl H₂O, 5 µl 10 × tampon (Qiagen), 2 µl 25 MgCl₂, 5 µl 100 dDNTP, 1 µl d'amorce directe, 1 µl d'amorce reverse, 0.25 µl hotstart Taq (Qiagen) et 2 µl d'ADN. Les PCRs ont été réalisées selon le protocole suivant: 15 minutes d'activation de l'enzyme à 95°C, suivies de 34 cycles comprenant 95°C pendant 30s, 60°C pendant 30s, 72°C pendant 1 minute et 5 minutes d'élongation à 72°C Après contrôle de la taille des amplifiats par électrophorèse sur gel d'agarose, les amplifiats ont été purifiés par addition de 50 µl d'eau distillée et utilisation d'une plaque de purification (Millipore, Molshein, France) agitée pendant 10 minutes. Les solutions de séquençage contiennent 3 µl de tampon (Big Dye V1, Applied Biosystems, Coutaboeuf, France), 5 µl d'eau distillée et 1 µl d'amorce à 3.2 pmol/µl pour un volume final de 11 µl. Les réactions de séquence comprennent une étape initiale de 1 minute à 95°C suivie de 25 cycles de dénaturation comprenant une étape de dénaturation à 96°C pendant 10s, hybridation des amorces à 50°C pendant 5s, et une étape d'élongation à 60°C pendant 3 minutes. Les produits de séquençage ont été purifiés sur plaque de sephadex (Amersham biosciences, Uppsala, Sweden) centrifugée à 720 g pendant 3 minutes et les produits de séquençage purifiés ont été déposés sur une plaque de séquençage Micro Amp Optical 96 well reaction plate (Applied Biosystems). L'électrophorèse de séquençage a été réalisée sur une séquenceur automatique 3100 genetic analyser (Applied Biosystems), les séquences ont été éditées par le Auto assembler program (Applied Biosystems), et alignées par CLUSTAL W (http://pbil.ibcp.fr). Ces séquences déterminées expérimentalement par les inventeurs pour 20 isolats de *Mycobacterium tuberculosis* et les séquences déposées dans GenBank pour les deux souches de référence de *Mycobacterium tuberculosis* CDC 1551 et H37 Rv ont permis de constituer une première base de données.

Les inventeurs ont déterminé que, par rapport à la collection des 105 isolats analysés, les 8 "spacers" suivants; MST5, MST6, MST7, MST9, MST10, MST14, MST15 et MST16, ne présentaient que deux variants de séquences chacun, et avaient un très faible pouvoir discriminant puisque chaque "spacer" MST5, MST6, MST7, MST9, MST10, MST14, MST15 et MST16 ségrégeait 17/20 (85%) des isolats dans un seul de deux variants. Ces huit "spacers" n'étant donc pas suffisamment informatifs n'ont plus été utilisés par la suite pour l'analyse de la collection de souches étudiée dans cet exemple. En revanche, les 8 autres "spacers": MST1, MST2, MST3, MST4, MST8, MST11, MST12, et MST13 ont été appliqués sur l'ensemble de la collection de 105 isolats de *M. tuberculosis* (Tableau 3). Ils ont permis de déterminer respectivement 4, 3, 2, 5, 3, 4, 7 et 11 variants de séquences qui, comme montré au tableau 4 ci-après, ont permis d'identifier 32 génotypes correspondant à 32 combinaisons différentes de ces 8 séquences sur l'ensemble des 105 isolats de l'espèce *Mycobacterium tuberculosis* testés et les deux souches de référence.

Les différents variants de séquences pour les 8 zones intergéniques MST1, MST2, MST3, MST4, MST8, MST11, MST12 et MST13, déterminés expérimentalement par amplification et séquençage, sont présentés dans les tableaux 5 à 12 ci-après, en faisant apparaître des mutations ponctuelles en caractères gras, ainsi que des délétions par alignement des séquences. Dans les alignements des tableaux 5 à 12 ci-après, les amorces nucléotidiques mises en oeuvre sont indiquées également en caractères gras aux extrémités des séquences.

Dans le tableau 5, les variants 1 à 4 de MST1 pour M. tuberculosis sont identifiés dans le listage de séquences comme des séquences respectivement SEQ. ID. n°52 à 55, avec variant 1 = SEQ. ID. n°52, variant 2 = SEQ. ID. n°53, variant 3 = SEQ. ID. n°54, variant 4 = SEQ. ID. n°55.

Dans le tableaux 6, les trois variants de MST2 sont identifiés dans le listage de séquences sous les séquences SEQ. ID n°56 à 58 respectivement, avec variant 1 = SEQ. ID. n°56, variant 2 = SEQ. ID. n°57, variant 3 = SEQ. ID. n°58.

Dans le tableau 7, les deux variants de MST3 sont identifiés dans le listage de séquences sous les séquences SEQ. ID n°59 à 60 respectivement, avec variant 1 = SEQ. ID. n°59, variant 2 = SEQ. ID. n°60.

Dans le tableau 8, les cinq variants de MST4 sont identifiés dans le listage de séquences sous les séquences SEQ. ID n°61 à 65 respectivement, avec variant 1 = SEQ. ID. n°61, variant 2 = SEQ. ID. n°62, variant 3 = SEQ. ID. n°63, variant 4 = SEQ. ID. n°64, variant 5 = SEQ. ID. n°65.

Dans le tableau 9, les trois variants de MST8 sont identifiés dans le listage de séquences sous les séquences SEQ. ID n°66 à 68 respectivement, avec variant 1 = SEQ. ID. n°66, variant 2 = SEQ. ID. n°67, variant 3 = SEQ. ID. n°68.

Dans le tableau 10, les quatre variants de MST11 sont identifiés dans le listage de séquences sous les séquences SEQ. ID n°33 à 36 respectivement, avec variant 1 = SEQ. ID. n°33, variant 2 = SEQ. ID. n°34, variant 3 = SEQ. ID. n°35, variant 4 = SEQ. ID. n°36.

Dans le tableau 11, les sept variants de MST12 sont identifiés dans le listage de séquences sous les séquences SEQ. ID n°41 à 47 respectivement, avec variant 1 = SEQ. ID. n°41, variant 2 = SEQ. ID. n°42, variant 3 = SEQ. ID. n°43, variant 4 = SEQ. ID. n°44, variant 5 = SEQ. ID. n°45, variant 6 = SEQ. ID. n°46, variant 7 = SEQ. ID. n°47.

Dans le tableau 12, les onze variants de MST13 sont identifiés dans le listage de séquences sous les séquences SEQ. ID n°69 à 79 respectivement, avec variant 1 = SEQ. ID. n°69, variant 2 = SEQ. ID. n°70, variant 3 = SEQ. ID. n°71, variant 4 = SEQ. ID. n°72, variant 5 = SEQ. ID. n°73, variant 6 = SEQ. ID. n°74, variant 7 = SEQ. ID. n°75, variant 8 = SEQ. ID. n°76, variant 9 = SEQ. ID. n°77, variant 10 = SEQ. ID. n°78, variant 11 = SEQ. ID. n°79.

Dans le tableau 4 ci-après, on indique dans les différentes colonnes pour les différentes zones intergéniques MST1, MST2, MST3, MST4, MST8, MST11, MST12 et MST13, les numéros des variants des séquences des tableaux 5 à 12, et, dans la colonne de droite, on a identifié 32 génotypes différents correspondants à 32 combinaisons différentes de séquences de variants des différentes zones intergéniques MST1, MST2, MST3, MST4, MST8, MST11, MST12 et MST13 des tableaux 5 à 12.

La méthode MST selon l'invention a été comparée à la méthode de référence par analyse des VNTR. On obtient un pouvoir discriminant mesuré par l'index de Hunter-Gaston supérieur pour la méthode MST que pour la méthode VNTR (index de 0.959 and 0.640, respectivement). Au total, ces résultats expérimentaux démontrent que :
1) l'ensemble des spacers prédits par les données bio-informatiques accessibles n'était pas utilisable pour le génotypage des isolats de *Mycobacterium tuberculosis,*
2) il était nécessaire de tester les différents spacers sur une collection d'isolats de l'espèce *Mycobacterium tuberculosis* provenant de différents sources géographiques, ce qui n'était pas anticipé au début de ce travail, et
3) la combinaison de certains spacers qui font l'objet de la présente invention, permettait pour la première fois le génotypage de *Mycobacterium tuberculosis* basé sur l'analyse de séquences génomiques, et
4) la méthode MST permet un génotypage de *Mycobacterium tuberculosis* davantage discriminant que les méthodes antérieures.

### Exemple 2. Identification des différentes espèces du complexe Mycobacterium tuberculosis

Les inventeurs ont montré que l'analyse de certains spacers permettait l'identification des espèces du complexe *Mycobacterium tuberculosis.* Pour ce faire, les inventeurs ont appliqué les 8 spacers présentés dans l'exemple 1 à une collection de souches types des espèces du complexe *Mycobacterium tuberculosis* habituellement responsables des infections chez l'homme, comprenant *Mycobacterium tuberculosis* CIP 103471 et les 105 souches cliniques représentant les différents génotypes décrits pour chaque spacer considéré dans l'exemple 1, *Mycobacterium bovis,* ssp. *bovis* CIP 10505, *Mycobacterium bovis,* souche BCG CIP 105050, *Mycobacterium africanum* CIP 105147 (sous-type I), *Mycobacterium canettii* CIP 105090. *Mycobacterium africanum* sous-type II n'a pas été inclus dans ce test, car il est actuellement considéré comme un variant de l'espèce *Mycobacterium tuberculosis* [Niemann S, Kubica T, Bange FC, Adjei O, Browne EN, Chinbuah MA, Diel R, Gyapong J, Horstmann RD, Joloba ML, Meyer CG, Mugerwa RD, Okwera A, Osei I, Owusu-Darbo E, Schwander SK, Rusch-Gerdes S. The species Mycobacterium africanum in the light of new molecular markers. J Clin Microbiol. 2004 ; 42:3958-62.

Parmi les 8 spacers, seuls les spacers MST 11 et MST 12 permettent de distinguer sans ambiguïté les 5 espèces testées. Les autres spacers présentés dans l'exemple 1 n'ont pas été retenus pour l'identification des espèces à l'intérieur du complexe *M. tuberculosis* car ils ne permettent pas de différencier l'espèce *M. tuberculosis* de *M. africanum, M. bovis* et *M. bovis* ssp. BCG, contrairement aux spacers MST11 et MST12.

Les conditions expérimentales étaient les mêmes que celles rapportées dans l'exemple 1. La figure 1 reproduit à titre d'exemple le profil d'électrophorèse en gel d'agarose à 1,5% des amplifiats obtenus avec les amorces des spacers MST11 et MST12, montrant la différence de taille des amplifiats obtenus avec chacune des ces espèces. Il a été ensuite montré que l'analyse des séquences des spacers MST11 et MST12, pris isolément, permet d'identifier chacune des espèces testées. Il s'agit de la première méthode d'identification des espèces du complexe *Mycobacterium tuberculosis* basée sur l'analyse de séquences. Ces données sont illustrées par les alignements des tableaux 13 et 14 ci-après :

Les séquences des fragments de la zone intergénique MST11 pour les espèces considérées au tableau 13, sont listées dans le listage de séquences dans les séquences SEQ. ID. n°33 à 40, avec
- *M. tuberculosis* variant 1 = SEQ. ID. n°33
- *M. tuberculosis* variant 2 = SEQ. ID. n°34
- *M. tuberculosis* variant 3 = SEQ. ID. n°35
- *M. tuberculosis* variant 4 = SEQ. ID. n°36
- *M. africanum* = SEQ. ID. n°37
- *M. bovis,* ssp *bovis* = SEQ. ID. n°38
- *M. bovis* ssp BCG= SEQ. ID. n°39
- *M. canettii* = SEQ. ID. n°40

Ces alignements du tableau 13 montrent que le spacer MST11 permet la différenciation des espèces du complexe *Mycobacterium tuberculosis* par rapport à la séquence de référence de *Mycobacterium tuberculosis* H37Rv (GenBank accession number AL 123456) par les éléments suivants :
- *M. canettii* présente une substitution du C en A en position 94 et une deuxième substitution du A en G en position 114 ;
- *M. africanum* présente une insertion de 51 paires de bases en position 350 ;
- *M. bovis* présente une insertion de 116 paires de bases en position 326 ;
- *M. bovis* ssp BCG présente une délétion de 58 paires de bases en position 350

Au tableau 14, les séquences des fragments de la zone intergénique MST12 pour les espèces considérées ci-dessus, sont listées dans le listage de séquences dans les séquences SEQ. ID. n°41 à 51, avec
- *M. tuberculosis* variant 1 = SEQ. ID. n°41
- *M. tuberculosis* variant 2 = SEQ. ID. n°42
- *M*. *tuberculosis* variant 3 = SEQ. ID. n°43
- *M. tuberculosis* variant 4 = SEQ. ID. n°44
- *M. tuberculosis* variant 5 = SEQ. ID. n°45
- *M*. *tuberculosis* variant 6 = SEQ. ID. n°46
- *M. tuberculosis* variant 7 = SEQ. ID. n°47
- *M. africanum =* SEQ. ID. n°48
- *M. bovis* ssp *bovis =* SEQ. ID. n°49
- *M. bovis* ssp BCG= SEQ. ID. n°50
- *M. canettii* = SEQ. ID. n°51

Ces alignements du tableau 14 montrent que le spacer MST12 permet la différenciation des espèces du complexe *Mycobacterium tuberculosis* par rapport à la séquence de référence de *Mycobacterium tuberculosis* H37Rv (GenBank accession number AL 123456) par les éléments suivants :
- *M. africanum* : une substitution du C en T en position 76 ;
- *M. bovis* : une délétion de 284 paires de bases en position 236.
- *M. bovis* subsp BCG : une délétion de 206 paire de bases en position 303.
- *M. canettii* : une délétion de 304 paires de bases en position 196 avec une substitution du T en A en position 194.

D'autres expériences ont été conduites avec des souches cliniques des espèces du complexe autres que l'espèce *Mycobacterium tuberculosis,* qui ont confirmées les résultats ci-dessus concernant le caractère discriminant au niveau de l'espèce des séquences des zones intergéniques MST11 et MST12.

**Tableau 1 : Position des 16 "spacers" sur le génome de M. tuberculosis H37Rv et les gènes encadrant**

| spacer | Position | Gène en amont | Gène en aval |
|---|---|---|---|
| MST1 | 71487-71795 | Conserved transmembrane protéine "Rv0064" | Conserved hypothetical protein "Rv0065"similaire à Rv06090 |
| MST2 | 206690-207028 | mce1F (Mce Family protein MCEF1) | Mce-associated membrane protcin "Rv0175" |
| MST3 | 240150-240131 | Conserved hypothetical protein "Rv1443c" | Conserved hypothetical protein "Rv14444c" |
| MST4 | 36463-636767 | qcr B (Ubiquinol cytochrome C reductase subunit B) | Conserved transmembrane proteine "Rv2197c" |
| MST5 | 304084-304375 | GNTR (transcriptional regulatory protein) | Fad E22 (Acyl coA Deshydrogenase FADE22) |
| MST6 | 273108-273711 | Hely (Helicase hely ATP- dependant DNA) | TatC (sec-independent protein translocase transmembrane |
| MST7 | 322829-323360 | Conserved hypothetical protein "Rv2141c" | Conserved hypothetical protein "Rv2142c" |
| MST8 | 416-440 | PbpB(Penicillin-binding membrane protein) | Conserved hypothetical protein "Rv63c" |
| MST9 | 217346-217871 | Conserved integral membrane protein"Rv2673 | Conserved hypothetical protein "Rv2674" |
| MST10 | 250110-250591 | SigB (RNA polymerase SIGMA factor) | IdeR (Iron-dependant repressor and acitivator IDER) |
| MST11 | 235244-235936 | Conserved hypothetical protein "Rv0486" | Conserved integral membrane protein"Rv0488" |
| MST12 | 238575-239212 | scnX3 (putative histidine kinase senx3) | regX3 (sensory transduction protein) |
| D4ST13 | 224126-224680 | conserved hypothetical protein "Rv1728c" | pbp-4 (penicillin-binding protein 4) |
| MST14 | 128029-128677 | whiBt(transcriptional regulatory protein whiB-like) | Two component sensor kinase |
| MST15 | 223496-223514 | conserved hypothetical protein "Rv3304c" | conserved hypothetical protein "Rv3305c" |
| MST16 | 323365-333650 | dap E2 (acetylornithine-deacetylase E2) | conserved hypothetical protein "Rv2142c" |

**Tableau 2 : Séquence des amorces pour l'amplification par PCR et le séquençage des 16 "spacers" présentés dans le Tableau 1.**

| N° | Amorce | Séquences (5'-3') |
|---|---|---|
| SEQ.ID n°1 | 1site242F | GATGGTCTCCCGGCTGAT |
| SEQ.ID n°2 | 1 site588R | GCTGGCCGATCTGCGCGC |
| SEQ.ID n°3 | 2site210F | GCGCCAAGGCCACCGGCCAA |
| SEQ.ID n°4 | 2site540R | GCCCGGCCAGCGGTGAACTGG |
| SEQ.ID n°5 | 8site481F | CTGTGGCAGGCTCCCGGTAG |
| SEQ.ID n°6 | 8site780R | TCGAGGATTCTGGGACTAT |
| SEQ.ID n°7 | 9site301F | GATCAGCTACGGGTTGGCCG |
| SEQ.ID n°8 | 9site720R | ATGGGTTCGCCAGACGGCGAG |
| SEQ.ID n°9 | 10site781F | CGGCCAGCCGCGGCGGACGA |
| SEQ.ID n°10 | 10site1140R | GATTGCGTTGCTGGACGGCCC |
| SEQ.ID n°11 | 10ghisF | CTGTGAGATCGGTGTGCTTT |
| SEQ.ID n°12 | 10ghisR | CTGCTGATCGTGATGCTGAA |
| SEQ.ID n°13 | 11ghisF | GCACCGGATTCAACGTATTC |
| SEQ.ID n°14 | 11ghisR | TAGTAGGGCACTAGCACCTC |
| SEQ.ID n°15 | 12ghisF | CCGCAATCACAAACTTCAATAC |
| SEQ.ID n°16 | 12ghisR | GCTACTTCGACGACGTGTAT |
| SEQ.ID n°17 | 17ghisF | CTTCATGACGTTGGATCGCT |
| SEQ.ID n°18 | 17ghisR | CGCAATGCGACTCGAATTTC |
| SEQ.ID n°19 | 18ghisF | CTTTGGGCGATTTCATCGAG |
| SEQ.ID n°20 | 18ghisR | CTCTTCCTCGAGGTCGTAGAT |
| SEQ.ID n°21 | 3ghisF | AGGTGTTAGAGGTGGTGGAT |
| SEQ.ID n°22 | 3ghisR | AACCAAAGTCATATTGGGATGAG |
| SEQ.ID n°23 | 4ghisF | GTTGATCGAGGCCTATCACG |
| SEQ.ID n°24 | 4ghisR | GAATAGGGCTTGGTCACGTA |
| SEQ.ID n°25 | 8ghisF | CGAGTTCACCGTCCATCATC |
| SEQ.ID n°26 | 8ghisR | GAGACAACGGTCATCGACTT |
| SEQ.ID n°27 | 20ghisF | CATAGTGAGGAGTAACGACTAA |
| SEQ.ID n°28 | 20ghisR | CTGATCATGGTGATCACCGA |
| SEQ.ID n°29 | 21ghisF | TTTGCGGCAACGAGATCTT |
| SEQ.ID n°30 | 21ghisR | GATGCATGTTCGACCCGTA |
| SEQ.ID n°31 | Vn1F | TTAGGCAAGTTCCGAATGACAG |
| SEQ.ID n°32 | Vn2R | CTATAGAGCTAAGAAGTTGTGTCC |

**Tableau 3 : Liste de 105 isolats cliniques de l'espèce Mycobacterium tuberculosis utilisés pour le génotypage par la méthode MST. Un seul isolat par patient. La deuxième colonne indique la nature du prélèvement d'où l'isolat a été cultivé, la troisième colonne indique l'origine géographique du patient.**

| | | |
|---|---|---|
| Tub1 | Expectoration | Afrique sub-saharienne |
| Tub2 | Abcès | Vietnam |
| Tub3 | Aspiration bronchique | Algérie |
| Tub4 | Ganglion | Algérie |
| Tub5 | Ganglion | Algérie |
| Tub6 | Expectoration | Italie |
| Tub7 | Liquide pleural | Italie |
| Tub8 | Aspiration bronchique | Pologne |
| Tub9 | Expectoration | Espagne |
| Tub10 | Abcès | Afrique sub-saharienne |
| Tub11 | Expectoration | Thaïlande |
| Tub12 | Expectoration | Thaïlande |
| Tub13 | Expectoration | Afrique sub-saharienne |
| Tub14 | Expectoration | Algérie |
| Tub15 | Expectoration | Pologne |
| Tub16 | Expectoration | Afrique sub-saharienne |
| Tub17 | Abcès | France |
| Tub18 | Aspiration bronchique | Algérie |
| Tub19 | Ganglion | France |
| Tub20 | Expectoration | Thaïlande |
| Tub21 | Expectoration | France |
| Tub22 | Expectoration | France |
| Tub23 | Expectoration | Algérie |
| Tub24 | Ganglion | France |
| Tub25 | Expectoration | Vietnam |
| Tub26 | Expectoration | Espagne |
| Tub27 | Expectoration | Algérie |
| Tub28 | Expectoration | Tunisie |
| Tub29 | Expectoration | France |
| Tub30 | Expectoration | France |
| Tub31 | Liquide pleural | France |
| Tub32 | Expectoration | France |
| Tub33 | Expectoration | France |
| Tub34 | Expectoration | Algérie |
| Tub35 | Expectoration | Philippines |
| Tub36 | Expectoration | Algérie |
| Tub37 | Ganglion | France |
| Tub38 | Ganglion | Turquie |
| Tub39 | Expectoration | Algérie |
| Tub40 | Expectoration | Afrique sub-saharienne |
| Tub41 | Ganglion | Afrique sub-saharienne |
| Tub42 | Expectoration | Algérie |
| Tub43 | Expectoration | France |
| Tub44 | Expectoration | Tunisie |
| Tub45 | Expectoration | France |
| Tub46 | Ganglion | Thaïlande |
| Tub47 | Expectoration | France |
| Tub48 | Expectoration | France |
| Tub49 | Expectoration | Afrique sub-saharienne |
| Tub50 | Expectoration | France |
| Tub51 | Expectoration | France |
| Tub52 | Expectoration | Kurdistan |
| Tub53 | Expectoration | Comores |
| Tub54 | Expectoration | Comores |
| Tub55 | Expectoration | Comores |
| Tub56 | Expectoration | Comores |
| Tub57 | Expectoration | France |
| Tub58 | Expectoration | France |
| Tub59 | Expectoration | France |
| Tub60 | Expectoration | Comores |
| Tub61 | Expectoration | Comores |
| Tub62 | Expectoration | Comores |
| Tub63 | Expectoration | Comores |
| Tub64 | Expectoration | France |
| Tub65 | Expectoration | Tunisie |
| Tub66 | Expectoration | Tunisie |
| Tub67 | Expectoration | Vietnam |
| Tub68 | Expectoration | Vietnam |
| Tub69 | Ganglion | Afrique sub-saharienne |
| Tub70 | Ganglion | Afrique sub-saharienne |
| Tub71 | Ganglion | Afrique sub-saharienne |
| Tub72 | Expectoration | Afrique sub-saharienne |
| Tub73 | Expectoration | Afrique sub-saharienne |
| Tub74 | Expectoration | Afrique sub-saharienne |
| Tub75 | Expectoration | Vietnam |
| Tub76 | Expectoration | Vietnam |
| Tub77 | Expectoration | Algérie |
| Tub78 | Aspiration bronchique | Algérie |
| Tub79 | Expectoration | Algérie |
| Tub80 | Ganglion | Algérie |
| Tub81 | Expectoration | Phillipine |
| Tub82 | Expectoration | Tunisie |
| Tub83 | Expectoration | Tunisie |
| Tub84 | Expectoration | Tunisie |
| Tub85 | Expectoration | France |
| Tub86 | Expectoration | France |
| Tub87 | Expectoration | France |
| Tub88 | Expectoration | France |
| Tub89 | Expectoration | France |
| Tub90 | Expectoration | France |
| Tub91 | Expectoration | France |
| Tub92 | Expectoration | France |
| Tub93 | Expectoration | France |
| Tub94 | Expectoration | Kurdistan |
| Tub95 | Expectoration | Thaïlande |
| Tub96 | Expectoration | Pologne |
| Tub97 | Expectoration | France |
| Tub98 | Expectoration | France |
| Tub99 | Expectoration | France |
| Tub100 | Expectoration | France |
| Tub101 | Expectoration | France |
| Tub102 | Expectoration | France |
| Tub103 | Expectoration | France |
| Tub104 | Expectoration | France |
| Tub105 | Expectoration | France |

**Tableau 4 : Résultats du génotypage de 105 isolats de l'espèce Mycobacterium tuberculosis par la méthode MST.**

| MST | | | | | | | | | |
|---|---|---|---|---|---|---|---|---|---|
| Souche | 1 | 2 | 3 | 4 | 8 | 11 | 12 | 13 | Génotypes |
| Tub1 | 1 | 1 | 1 | 1 | 1 | 1 | 1 | 2 | 1 |
| Tub10 | 1 | 1 | 1 | 1 | 1 | 1 | 1 | 2 | 1 |
| Tub13 | 1 | 1 | 1 | 1 | 1 | 1 | 1 | 2 | 1 |
| Tub16 | 1 | 1 | 1 | 1 | 1 | 1 | 1 | 2 | 1 |
| Tub40 | 1 | 1 | 1 | 1 | 1 | 1 | 1 | 2 | 1 |
| Tub41 | 1 | 1 | 1 | 1 | 1 | 1 | 1 | 2 | 1 |
| Tub49 | 1 | 1 | 1 | 1 | 1 | 1 | 1 | 2 | 1 |
| Tub71 | 1 | 1 | 1 | 1 | 1 | 1 | 1 | 2 | 1 |
| Tub72 | 1 | 1 | 1 | 1 | 1 | 1 | 1 | 2 | 1 |
| Tub73 | 1 | 1 | 1 | 1 | 1 | 1 | 1 | 2 | 1 |
| Tub74 | 1 | 1 | 1 | 1 | 1 | 1 | 1 | 2 | 1 |
| Tub75 | 1 | 1 | 1 | 1 | 1 | 1 | 1 | 2 | 1 |
| Tub76 | 1 | 1 | 1 | 1 | 1 | 1 | 1 | 2 | 1 |
| Tub2 | 1 | 1 | 1 | 1 | 1 | 1 | 1 | 5 | 2 |
| Tub77 | 1 | 1 | 1 | 1 | 1 | 1 | 1 | 5 | 2 |
| Tub78 | 1 | 1 | 1 | 1 | 1 | 1 | 1 | 5 | 2 |
| Tub25 | 1 | 1 | 1 | 1 | 3 | 1 | 1 | 5 | 14 |
| Tub69 | 1 | 1 | 1 | 1 | 3 | 1 | 1 | 5 | 14 |
| Tub70 | 1 | 1 | 1 | 1 | 3 | 1 | 1 | 5 | 14 |
| Tub20 | 1 | 1 | 1 | 1 | 1 | 1 | 2 | 3 | 12 |
| Tub46 | 1 | 1 | 1 | 1 | 1 | 1 | 2 | 3 | 12 |
| Tub11 | 3 | 1 | 1 | 1 | 1 | 1 | 1 | 2 | 7 |
| Tub12 | 3 | 1 | 1 | 1 | 1 | 1 | 1 | 2 | 7 |
| Tub91 | 3 | 1 | 1 | 1 | 1 | 1 | 1 | 2 | 7 |
| Tub35 | 1 | 1 | 1 | 5 | 1 | 1 | 2 | 4 | 20 |
| Tub20 | 1 | 1 | 1 | 5 | 1 | 1 | 2 | 4 | 20 |
| Tub52 | 1 | 1 | 1 | 1 | 1 | 4 | 1 | 1 | 26 |
| Tub90 | 1 | 1 | 1 | 1 | 1 | 4 | 1 | 1 | 26 |
| Tub8 | 1 | 1 | 1 | 1 | 1 | 3 | 1 | 3 | 6 |
| Tub90 | 1 | 1 | 1 | 1 | 1 | 3 | 1 | 3 | 6 |
| Tub15 | 1 | 1 | 2 | 1 | 1 | 2 | 1 | 1 | 9 |
| Tub6 | 1 | 1 | 1 | 1 | 1 | 3 | 1 | 1 | 5 |
| Tub7 | 1 | 1 | 1 | 1 | 1 | 3 | 1 | 1 | 5 |
| Tub17 | 3 | 1 | 1 | 1 | 1 | 1 | 1 | 1 | 10 |
| Tub32 | 3 | 1 | 1 | 1 | 1 | 1 | 1 | 1 | 10 |
| Tub33 | 3 | 1 | 1 | 1 | 1 | 1 | 1 | 1 | 10 |
| Tub101 | 1 | 1 | 1 | 1 | 1 | 1 | 1 | 1 | 19 |
| Tub102 | 1 | 1 | 1 | 1 | 1 | 1 | 1 | 1 | 19 |
| Tub103 | 1 | 1 | 1 | 1 | 1 | 1 | 1 | 1 | 19 |
| Tub48 | 1 | 1 | 1 | 3 | 1 | 1 | 1 | 1 | 25 |
| Tub87 | 1 | 1 | 1 | 3 | 1 | 1 | 1 | 1 | 25 |
| Tub88 | 1 | 1 | 1 | 3 | 1 | 1 | 1 | 1 | 25 |
| Tub89 | 1 | 1 | 1 | 3 | 1 | 1 | 1 | 1 | 25 |
| Tub93 | 1 | 1 | 1 | 3 | 1 | 1 | 1 | 1 | 25 |
| Tub94 | 1 | 1 | 1 | 3 | 1 | 1 | 1 | 1 | 25 |
| Tub104 | 1 | 1 | 1 | 3 | 1 | 1 | 1 | 1 | 25 |
| Tub105 | 1 | 1 | 1 | 3 | 1 | 1 | 1 | 1 | 25 |
| Tub64 | 1 | 1 | 1 | 1 | 1 | 1 | 1 | 4 | 30 |
| Tub165 | 1 | 1 | 1 | 1 | 1 | 1 | 1 | 4 | 30 |
| Tub43 | 2 | 1 | 2 | 2 | 1 | 1 | 2 | 3 | 22 |
| Tub45 | 1 | 1 | 1 | 1 | 1 | 2 | 1 | 1 | 24 |
| Tub29 | 1 | 1 | 1 | 1 | 1 | 2 | 1 | 1 | 18 |
| Tub30 | 1 | 1 | 1 | 1 | 1 | 2 | 1 | 1 | 18 |
| Tub95 | 1 | 1 | 1 | 1 | 1 | 2 | 1 | 1 | 18 |
| Tub96 | 1 | 1 | 1 | 1 | 1 | 2 | 1 | 1 | 18 |
| Tub21 | 1 | 1 | 1 | 1 | 1 | 2 | 1 | 1 | 18 |
| Tub22 | 1 | 1 | 1 | 1 | 1 | 2 | 1 | 1 | 18 |
| Tub19 | 1 | 1 | 2 | 1 | 1 | 2 | 1 | 1 | 11 |
| Tub24 | 1 | 1 | 2 | 1 | 1 | 2 | 1 | 1 | 11 |
| Tub47 | 1 | 1 | 2 | 1 | 1 | 2 | 1 | 1 | 11 |
| Tub50 | 1 | 1 | 2 | 1 | 1 | 2 | 1 | 1 | 11 |
| Tub51 | 1 | 1 | 2 | 1 | 1 | 2 | 1 | 1 | 11 |
| Tub58 | 1 | 1 | 2 | 1 | 1 | 2 | 1 | 1 | 11 |
| Tub59 | 1 | 1 | 2 | 1 | 1 | 2 | 1 | 1 | 11 |
| Tub97 | 1 | 1 | 2 | 1 | 1 | 2 | 1 | 1 | 11 |
| Tub98 | 1 | 1 | 2 | 1 | 1 | 2 | 1 | 1 | 11 |
| Tub99 | 1 | 1 | 2 | 1 | 1 | 2 | 1 | 1 | 11 |
| Tub38 | 1 | 1 | 1 | 1 | 1 | 1 | 2 | 3 | 21 |
| Tub28 | 1 | 2 | 2 | 1 | 1 | 2 | 3 | 6 | 17 |
| Tub44 | 4 | 1 | 1 | 1 | 1 | 1 | 1 | 3 | 23 |
| Tub66 | 4 | 1 | 1 | 1 | 1 | 1 | 1 | 3 | 23 |
| Tub67 | 4 | 1 | 1 | 1 | 1 | 1 | 1 | 3 | 23 |
| Tub68 | 4 | 1 | 1 | 1 | 1 | 1 | 1 | 3 | 23 |
| Tub84 | 4 | 1 | 1 | 1 | 1 | 1 | 1 | 3 | 23 |
| Tub85 | 4 | 1 | 1 | 1 | 1 | 1 | 1 | 3 | 23 |
| Tub86 | 4 | 1 | 1 | 1 | 1 | 1 | 1 | 3 | 23 |
| Tub3 | 1 | 1 | 1 | 1 | 2 | 1 | 2 | 1 | 15 |
| Tub79 | 1 | 1 | 1 | 1 | 1 | 1 | 2 | 1 | 3 |
| Tub80 | 1 | 1 | 1 | 1 | 1 | 1 | 2 | 1 | 3 |
| Tub81 | 1 | 1 | 1 | 1 | 1 | 1 | 2 | 1 | 3 |
| Tub82 | 1 | 1 | 1 | 1 | 1 | 1 | 2 | 1 | 3 |
| Tub27 | 1 | 3 | 1 | 1 | 1 | 1 | 4 | 7 | 16 |
| Tub14 | 1 | 1 | 2 | 1 | 1 | 1 | 1 | 2 | 8 |
| Tub42 | 1 | 1 | 2 | 1 | 3 | 1 | 1 | 2 | 8 |
| Tub4 | 1 | 1 | 1 | 1 | 1 | 1 | 1 | 2 | 4 |
| Tub5 | 1 | 1 | 1 | 1 | 1 | 1 | 1 | 2 | 4 |
| Tub36 | 1 | 1 | 1 | 1 | 1 | 1 | 1 | 2 | 4 |
| Tub34 | 1 | 1 | 1 | 1 | 1 | 1 | 1 | 2 | 4 |
| Tub39 | 1 | 1 | 1 | 1 | 1 | 1 | 1 | 1 | 4 |
| Tub23 | 1 | 1 | 2 | 1 | 1 | 1 | 5 | 8 | 13 |
| Tub63 | 1 | 1 | 2 | 1 | 1 | 1 | 6 | 9 | 29 |
| Tub62 | 1 | 1 | 1 | 1 | 2 | 1 | 1 | 1 | 28 |
| Tub61 | 1 | 1 | 1 | 1 | 2 | 1 | 1 | 1 | 28 |
| Tub60 | 1 | 1 | 1 | 1 | 2 | 1 | 1 | 1 | 28 |
| Tub53 | 4 | 1 | 1 | 4 | 2 | 1 | 1 | 1 | 27 |
| Tub54 | 4 | 1 | 1 | 4 | 2 | 1 | 1 | 1 | 27 |
| Tub55 | 4 | 1 | 1 | 4 | 2 | 1 | 1 | 1 | 27 |
| Tub56 | 4 | 1 | 1 | 4 | 2 | 1 | 1 | 1 | 27 |
| Souche H37Rv | 3 | 2 | 1 | 2 | 3 | 3 | 5 | 10 | 31 |
| Souche CDC1551 | 3 | 1 | 1 | 5 | 1 | 4 | 7 | 11 | 32 |

**Tableau 5 : Variants MST1 pour M. tuberculosis : 4 variants**

| | |
|---|---|
| V. 1 | **GATGGTCTCCCGGCTGAT**CCTGGGCTGGCAGTGCTGGCGAGCGCCTGTTCCAGGATCTCGATTGCCTCGGCCTTGTC |
| V. 2 | **GATGGTCTCCCGGCTGAT**CCTGGGCTGGCAGTGCTGGCGAGCGCCTGTTCCAGGATCTCGATTGCCTCGGCCTTGTC |
| V. 4 | **GATGGTCTCCCGGCTGAT**CCTGGGCTGGCAGTGCTGACGAGCGCCTGTTCCAGGATCTCGATTGCCTCGGCCTTGTC |
| V. 3 | **GATGGTCTCCCGGCTGAT**CCTGGGCTGGCAGTGCTGGCGAGCGCCTGTTCCAGGATCTCGATTGCCTCGGCCTTGTC |
| V. 1 | CGGCCCCGCACCCCTTCGATGAGCTTGGTGCGCATATACGTCTGCAGCGACTGCCCCGC |
| V. 2 | GGCCCCGCACCCCTTCGATGAGCTTGGTGCGCATATACGTTTGCAGCGACTGCCCCGC |
| V. 4 | CGGCCCCGCACCCCTTCGATGAGCTTGGTGCGCATATACGTCTGCAGCGACTGCCCCGC |
| V. 3 | CGGCCCCGCACCCCTTCGATGAGCTTGGTGCGCATATACGTCTGCAGCGACTGCCCCGC |
| V. 1 | GCGGTGGCGCGCCGTCGATAGGTTTCGGCGACATCTTCGGGCAAATCCCGAACTTGAAT |
| V. 2 | GCGGTGGCGCGCCGTCGATAGGTTTCGGCGACATCTTCGGGCAAATCCCGAACTTGAAT |
| V. 4 | GCGGTGGCGCGCCGTCGATAGGTTTCGGCGACATCTTCGGGCAAATCCCGAACTTGAAT |
| V. 3 | GCGGTGGCGCGCCGTCGATAGGTTTCGGCGACATCTTCGGGCAAATCCCGAACTTGAAT |
| V. 1 | GTAGCCATAGCGCTATTCTAGCGTCAGATTAGCGCCAGAACAGCGCTCGGAGTTCACCG |
| V. 2 | GTAGCCATAGCGCTATTCTAGCGTCAGATTAGCGCCAGAACAGCGCTCGGAGTTCACCG |
| V. 4 | GTAGCCATAGCGCTATTCTAGCGTCAGATTAGCGCCAGAACAGCGCTCGGAGTTCACCG |
| V. 3 | GTAGCCATAGCG----------------------------------------------------------------------------------GAGTTCACCG |
| V. 1 | GCTAAGAATGCCGGCTTATCCACCGGGCGTTTCGGCGGCATCGATGGCCTTCTCTAACCG |
| V. 2 | GCTAAGAATGCCGGCTTATCCACCGGGCGTTTCGGCGGCATCGATGGCCTTCTCTAACCG |
| V. 4 | GCTAAGAATGCCGGCTTATCCACCGGGCGTTTCGGCGGCATCGATGGCCTTCTCTAACCG |
| V. 3 | GCTAAGAATGCCGGCTTATCCACCGGGCGTTTCGGCGGCATCGATGGCCTTCTCTAACCG |
| V. 1 | GTCGAGCACC**GCGCGCAGATCGGCCAGC** |
| V. 2 | GTCGAGCACC**GCGCGCAGATCGGCCAGC** |
| V. 4 | GTCGAGCACC**GCGCGCAGATCGGCCAGC** |
| V. 3 | GTCGAGCACC**GCGCGCAGATCGGCCAGC** |

**Tableau 6 : Variants MST2 pour M. tuberculosis : 3 variants**

| | |
|---|---|
| V. 1 | **GCGCCAAGGCCACCGGCCAA**CGCGAGCAGCGTCGCCGCAATGCCAACAAGCCAGCCTCGCCGCAACCGCGACGGGCGCCG |
| V. 3 | **GCGCCAAGGCCACCGGCCAA**CGCGAGCAGCGTCGCCGCAATGCCAACAAGCCAGCCTCGCCGCAACCGCGACGGGCGCCG |
| V. 2 | **GCGCCAAGGCCACCGGCCAA**CGCGAGCAGCGTCGCCGCAATGCCAACAAGCCAGCCTCGCCGCAACCGCGACGGGCGCCG |
| V. 1 | CTCGACGAGGGCCTGTTCTGGGCACGCGTCCTCGCCGAGCTCTCCGGCGTCAGATTCCGC |
| V. 3 | CTCGACGAGGGCCTGTTCTGGGCACGCGTCCTCGCCGAGCTCTCCGGCGTCAGATTCCGC |
| V. 2 | CTCGACGAGGGCCTGTTCTGGGCACGCGTCCTCGCCGAGCTCTCCGGCGTCAGATTCCGC |
| V. 1 | CGAATCCGCCGCCTTCACCTGCGGGCCAGTCTGGTCGGCTCCGGCGTCAGATTCCGCCGA |
| V. 3 | CGAATCCGCCGCCTTCACCTGCGGGCCAGTCTGGTCGGCTCCGGCGTCAGATTCCGCCGA |
| V. 2 | CGAATCCGCCGCCTTCACCT------------------------------------------------------------------------------------- |
| V. 1 | ATCCGCCGCCTTCACCTCCGTAGACGACGACTTATTCGCATCGGCAGGGTTCAGCTGGCC |
| V. 3 | ATCCGCCGCCTTCACCTCCGTAGACGACGACTTATTCGCATCGGCAGGGTTCAGCTGGCC |
| V. 2 | -----------------------------------CCGTAGACGACGACTTATTCGCATCGGCAGGGTTCAGCTGGCC |
| V. 1 | GGCGCCAGCATCTCCTTCCATCCGTCGTCTCCTGTTGTGCTCGAGTTTGCGACGGCGTAC |
| V. 3 | GGCGCCAGCATCTCCTTCCATCCGTCGTCTCCTGTTGTGCTCGAGTTTGCGACCGCGTAC |
| V. 2 | GGCGCCAGCATCTCCTTCCATCCGTCGTCTCCTGTTGTGCTCGAGTTTGCGACGGCGTAC |
| V. 1 | TTGACACCATCCGGCCCC**ACCAGTTCACCGCTGGCCGCGC** |
| V. 3 | TTGACACCATCCGGCCCC**ACCAGTTCACCGCTGGCCGGGC** |
| V. 2 | TTGACACCATCCGGCCCC**ACCAGTTCACCGCTGGCCGGGC** |

**Tableau 7 : Variants MST3 pour M. tuberculosis : 2 variants**

| | |
|---|---|
| V. 1 | **CTGTGGCAGGCTCCCGGTAGCCA**CCGGCCTCGCCGTATTCGTCGGCATTTGGTTCGTCGCGTCTCCGTAAGGCGTTTCAG |
| V. 2 | **CTGTGGCAGGCTCCCGGTAG**CCACCGGCCTCGCCGTATTCGTCGGCATTTGGTTCGTCGCGTCTCCGTAAGGCGTTTCAG |
| V. 1 | GGCCTTCGAGGTCGCGCTTCCCCACCCGAAGACACCGAGTGCGGCGCCTGCGACCACGA |
| V. 2 | GGCCTTCGAGGTCGCGCTTCCCCACCCGAAGACACCGAGTGCGGCGCCTGCGACCACGA |
| V. 1 | TGACCGAGAGAAAAGTCAGCGGCCCTGACAGAGCAGCTGCGCGGAATCACTAGCTTCGCG |
| V. 2 | TGACCGAGAGAAAAGTCAGCGGCCCTGACAGAGCAGCTGCGCGGAATCAGTAGCTTCGCG |
| V. 1 | GCAGCGCCGCGACCATCGCCTCGACCTGGGCCCGGGTGAGAACCGTGATGCTGGAGTCTG**ATAGTCCCAGAATCCTCGA** |
| V. 2 | GCAGCGCCGCGACCATCGCCTCGACCTGGTCCCGGGTGAGAACCGTGATGCTGGAGTCT**GATAGTCCCAGAATCCTCGA** |

**Tableau 8 : Variants MST4 pour M. tuberculosis : 5 variants**

| | |
|---|---|
| V. 1 | **GATCAGCTACGGGTTGGCCG**GGGTGGTCACCGCGGGCATGCCGGTGATCGAGTGGCTATACGCTCGGCAGCTGCGCCGGG |
| V. 2 | **GATCAGCTACGGGTTGGCCGG**GGTGGTCACCGCGGGCATGCCGGTGATCGAGTGGCTATACGCTCGGCAGCTGCGCCGGG |
| V. 3 | **GATCAGCTACGGGTTGGCCG**GGGTGGTCACCGCGGGCATGCCGGTGATCGAGTGGCTATACGCTCGGCAGCTGCGCCGGG |
| V. 4 | **GATCAGCTACGGGTTGGCCG**GGGTGGTCACCGCGGGCATGCCGGTGATCGAGTGGCTATACGCTCGGCAGCAGCTGCGCCGGG |
| V. 5 | **GATCAGCTACGGGTTGGCCG**GGGTGGTCACCGCGGGCATGCCGGTGATCGAGTGGCTATACGCTCGGCAGCTGCGCCGGG |
| V. 1 | TGGTGGCGCCCCAGTCCAGTTAGCCGGGCTGTGCTTGATTTGCCCGGTTCCTTCCCGGGT |
| V. 2 | TGGTGGCGCCCCAGTCCAGTTAGCCGGGCTGTGCTTGATTTGCCCGGTTCCTTCCCGGGT |
| V. 3 | TGGTGGCGCCCCAGTCCAGTTAGCCGGGCTGTGCTTGATTTGCCCGGTTCCTTCCCGGGT |
| V.4 | TGGTGGCGCCCCAGTCCAGTTAGCCGGGCTGTGCTTGATTTGCCCGGTTCCTTCCCGGGT |
| V.5 | TGGTGGCGCCCCAGTCCAGTTAGCCGGGCTGTGCTTGATTTGCCCGGTTCCTTCCCGGGT |
| V. 1 | CGTGCCGACCCGGGTCGTCGCCGGGCTAGTG---------------------------------------------------------- |
| V.2 | CGTGCCGACCCGGGTCGTCGCCGGGCTGTGCTTGATTTGCCCGGTTCCTTCCCGGGTCGT |
| V. 3 | CGTGCCGACCCGGGTCGTCGCCGGGCTGTGCTTGATTTGCCCGGTTCCTTCCCGGGTCGT |
| V. 4 | CGTGCCGACCCGGGTCGTCGCCGGGCTGTGCTTGATTTGCCCGGTTCCTTCCCGGGTCGT |
| V. 5 | CGTGCCGACCCGGGTCGTCGCCGGGCTGTGCTTGATTTGCCCGGTTCCTTCCCGGGTCGT |
| V. 1 | -------------------------------------------------------------------------------------------------------------------------------- |
| V. 2 | GCCGACCCGGGTCGTCGCCGGGCTAGTG------------------------------------------------------------------- |
| V. 3 | GCCGACCCGGGTCGTCGCCGGGCTGTGCTTGATTTGCCCGGTTCCTTCCCGGGTCGTGCC |
| V. 4 | GCCGACCCGGGTCGTCGCCGGGCTGTGCTTGATTTGCCCGGTTCCTTCCCGGGTCGTGCC |
| V. 5 | GCCGACCCGGGTCGTCGCCGGGCTGTGCTTGATTTGCCCGGTTCCTTCCCGGCTCGTGCC |
| V. 1 | -------------------------------------------------------------------------------------------------------------------------------- |
| V. 2 | -------------------------------------------------------------------------------------------------------------------------------- |
| V. 3 | GACCCGGGTCGTCGCCGGGCTGTGCTTGATTTGCCCGGTTCCTTCCCGGGTCGTGCCGAC |
| V. 4 | GACCCGGGTCGTCGCCGGGCTGTGCTTGATTTGCCCGGTTCCTTCCCGGGTCGTGCCGAC |
| V. 5 | GACCCGGGTCGTCGCCGGGCTAGTG------------------------------------------------------------------------- |
| V. 1 | -------------------------------------------------------------------------------------------------------------------------------- |
| V. 2 | -------------------------------------------------------------------------------------------------------------------------------- |
| V. 3 | CCGGGTCGTCGCCGGGCTAGTG-------------------------------------------------------------------------------- |
| V. 4 | CCGGGTCGTCGCCGGGCTGTGCTTGATTTGCCCGGTTCCTTCCCGGGTCGTGCCGACCCG |
| V. 5 | -------------------------------------------------------------------------------------------------------------------------------- |
| V. 1 | --------------------------------------------**CTCGCCGTCTGGCGAACCCAT** |
| V. 2 | --------------------------------------------**CTCGCCGTCTGGCGAACCCAT** |
| V. 3 | --------------------------------------------**CTCGCCGTCTGGCGAACCCAT** |
| V. 4 | GGTCGTCGCCGGGCTAGTG**CTCGCCGTCTGGCGAACCCAT** |
| V. 5 | --------------------------------------------**CTCGCCGTCTGGCGAACCCAT** |

**Tableau 9 : Variants MST8 pour M. tuberculosis : 3 variants**

| | |
|---|---|
| V. 1 | **CCGCAATCACAAACTTCAATA**CCCCGCACCTCCGACCAGCTCAGCACGACTTCACGAATCCCAGACCTGCGACACCGTCGGC |
| V. 2 | **CCGCAATCACAAACTTCAATA**CCCCGCACCTCCGACCAGCTCAGCACGACTTCACGAATCCCAGACCTGCGACACCGTAGGC |
| V. 3 | **CCGCAATCACAAACTTCAATA**CCCCGCACCTCCGACCAGCTCAGCACAACTTCACGAATCCCAGACCTGCGACACCGTCGGC |
| V. 1 | AGGGCTTTCGATCCTATAACAATCTGGAAACAGGATGTCGCACTTTCCTTAAAAGAGCTT |
| V. 2 | AGGGCTTTCGATCCTATAACAATCTGGAAACAGGATGTCGCACTTTCCTTAAAAGAGCTT |
| V. 3 | AGGGCTTTCGATCCTATAACAATCTGGAAACAGGATGTCGCACTTTCCTTAAAAGAGCTT |
| V. 1 | CCGCCAACCCGATCGTCAGCGCGCACATGTTGCGCAAAAGTTGTTGGAGCCGAAACGAAC |
| V. 2 | CCCCCAACCCGATCGTCAGCGCGCACATGTTGCGCAAAAGTTGTTGGAGCCGAAACGAAC |
| V. 3 | CCGCCAACCCGATCGTCAGCGCGCACATGTTGCGCAAAAGTTGTTGGAGCCGAAACGAAC |
| V. 1 | CGGCGCGCCCCGTTACCGGCGCCGCCGCCCTAGGTGGCCTGCAAGACCAAAGGAGGCCCG |
| V. 2 | CGGCGCGCGCCGTTACCGGCGCCGCCGCCCTAGGTGGCCTGCAAGACCAAAGGAGGCCCG |
| V. 3 | CGGCGCGCGCCGTTACCGGCGCCGCCGCCCTAGGTGGCCTGCAAGACCAAAGGAGGCCCG |

**Tableau 10 : Variants MST11 pour M. tuberculosis : 4 variants**

| | |
|---|---|
| V. 1 | **AGGTGTTAGAGGTGGTGGA**TTCGGACTTCAATGCGTTGTTGGAGTTGGGATTTCGGTCGTCGATTCAACGAGAGTGGCAG |
| V. 2 | **AGGTGTTAGAGGTGGTGGA**TTCGGACTTCAATGCGTTGTTGGAGTTGGGATTTCGGTCGTCGATTCAACGAGAGTGGCAG |
| V. 3 | **AGGTGTTAGAGGTGGTGGA**TTCGGACTTCAATGCGTTGTTGGAGTTGGGATTTCCGTCGTCGATTCAACGAGAGTGGCAG |
| V. 4 | **AGGTGTTAGAGGTGGTGGA**TTCGGACTTCAATGCGTTGTTGGAGTTGGGATTTCGGTCGTCGATTCAACGAGAGTGGCAG |
| V. 1 | TGGCGGTTATCTCGCGGTGAGTCGCTGCAGAACCTGCAGGCCTTCGCTCACTTACGCCCG |
| V. 2 | TGGCGGTTATCTCGCGGTGAGTCGCTGCAGAACCTGCAGGCCTTCGCTCACTTACGCCTG |
| V. 3 | TGGCGGTTATCTCGCGGTGAGTCGCTGCAGAACCTGCAGGCCTTCGCTCACTTACGCC-- |
| V. 4 | TGGCGGTTATCTCGCGGTGAGTCGCTGCAGAACCTGCAGGCCTTCGCTCACTTACGCC-- |
| V. 1 | ACGACGATCCAGACCCCGCAGCGCGATGAGAAGCAGTTGGGCGGTTAGCTCGACCCCCAC |
| V. 2 | ACGACGATGCAGAGCGCGCAGCGCGATGAGAAGGAGTTGGGCGGTTAGGTCGAGCCCGAC |
| V. 3 | --------------------------------------------------------------------------------------------------------------------------CGAC |
| V. 4 | ---------------------------------------------------------------------------------------------------------------------------------- |
| V. 1 | GGATCGGGTGACAGCGGGCCGTTTTCGCGCTGCATCAGCCAGCCCGCGATGTTGTGGAAC |
| V. 2 | GGATCGGGTGACAGCGGGCCGTTTTCGCGCTGCATCAGCCAGCCCGCGATGTTGTGGAAC |
| V. 3 | GGATCGGGTGACAGCGGGACGTTTTCGCGCTGCATCAGCCAGCCCGCGATGTTGTGGAAC |
| V. 1 | AGCGGGGCGGCCGAGTGCCCAGGCGCGCCGTCGGAGTTGCGCGCCGGGTTGTCCAACATG |
| V. 2 | AGCGGGGCGGCCGAGTGCCCAGGCGCGCCGTCGGAGTTGCGCGCCGGGTTGTCCAACATG |
| V. 3 | AGCGGGGCGGCCGAGTGCCCAGGCGCGCCGTCGGAGTTGCGCGCCGGGTTGTCCAACATG |
| V. 1 | ATGCCGATCACGTAGCGGGGATTGTCGGCAGTGGCGATTCCGGCGAAGGTGATCC**AATACACGTCGTCGAAGTAGC** |
| V. 2 | ATGCCGATCACGTAGCGGGGATTGTCGGCAGTGGCGATTCCGGCGAAGGTGATCC**AATACACGTCGTCGAAGTAGC** |
| V. 3 | ATGCCGATCACGTAGCGGGGATTGTCGGCAGTGGCGATTCCGGCGAAGGTGATCC**AATACACGTCGTCGAAGTAGC** |
| V. 1 | GACGATGCAGAGCGCGCAGCGCGATGAGAAGGAGTTGGGCGGTTAGGTCGAGCCCGACGA |
| V. 2 | GACGATGCAGAGCGCGCAGCGCGATGAGAAGGAGTTGGGCGGTTAGGTCGAGCCCGACGA |
| V. 3 | GACGATGCAGAGCGCGCAGCGCGATGAGAAGGAGTTGGGCGGTTAGGTCGAGCCCCACGA |
| V. 4 | ----------------------------------------------------------------------------------------------------------------------CGACGA |
| V. 1 | CGATGCAGAGCGCGCACCGCGATGAGAAGGAGTTGGGCGCTTAGGTCGAGCCCGACGACG |
| V. 2 | CGATGCAGAGCGCGCACCGCGATGAGAAGGAGTTGGGCGGTTAGGTCGAGCCCGACGACG |
| V. 3 | CGATGCAGACCGCGCAGCGCCATGAGAAGGAGTTGGGCGGTTAGGTCGAGCCCGACGACG |
| V. 4 | CGATGCAGAGCGCGCACCGCGATGAGAAGGAGTTGGGCGGTTAGGTCCAGCCCGACGACG |
| V. 1 | ATGCAGAGCGCGCAGCGCGATGAGAAATAGCACTCGTGGAGGTCAAGACGCCCGCCGGTG |
| V. 2 | ATGCAGAGCGCGCAGCGCGATGAGAAATAGCACTCGTGGAGGTCAAGACGCCCGCCGGTG |
| V. 3 | ATGCAGAGCGCGCAGCGCGATGAGAAATAGCACTCGTGGAGGTCAAGACGCCCGCCGGTG |
| V. 4 | ATGCAGAGCGCGCAGCGCGATGAGAAATAGCACTCGTGGAGGTCAAGACGCCCGCCGGTG |
| V. 1 | ATGGGCTGGTGGCGCTCACCCCGTTCCGGACTCAGAAATTCGCGATCACAATTTGCGCGT |
| V. 2 | ATGGGCTGGTGGCGCTCACCCCGTTCCGGACTCAGAAATTCGCGATCACAATTTGCGCGT |
| V. 3 | ATGGGCTGGTGGCGCTCACCCCGTTCCGGACTCAGAAATTCGCGATCACAATTTGCGCGT |
| V. 4 | ATGGGCTGGTGGCGCTCACCCCGTTCCGGACTCAGAAATTCGCGATCACAATTTGCGCGT |
| V. 1 | TCAAGTCATTGGCATGCATGTGATGGTTTAGCGTTCCGCTGTGCCTCTTCAGGTGTTTGT |
| V. 2 | TCAAGTCATTGGCATGCATGTGATGGTTTAGCGCTCCGCTGTGCCTCTTCAGGTGTTTGT |
| V. 3 | TCAAGTCATTGGCATGCATGTGATGGTTTAGCGTTCCGCTGTGCCTCTTCAGGTGTTTGT |
| V. 4 | TCAAGTCATTGGCATGCATGTGATGGTTTAGCGTTCCGCTGTGCCTCTTCAGGTGTTTGT |
| V. 1 | CGGCTTCGTTGCCATGATGACGCTCAAGGTCGCGATCGGCCCGCAAAACGCATTTGTCCT |
| V. 2 | CGGCTTCGTTGCCATGATGACGCTCAAGGTCGCGATCGGCCCGCAAAACGCATTTGTCCT |
| V. 3 | CGGCTTCGTTGCCATGATGACGCTCAAGGTCGCGATCGGCCCGCAAAACGCATTTGTCCT |
| V. 4 | CGGCTTCGTTGCCATGATGACGCTCAAGGTCGCGATCGGCCCGCAAAACGCATTTGTCCT |
| V. 1 | GCGCCAAGGAATTAGGCGAGAATACGTGCTGGTCATTGTGGCGCTGTGCGGGATCGCTGA |
| V. 2 | GCGCCAAGGAATTAGGCGAGAATACGTGCTGGTCATTGTGGCGCTGTGCGGGATCGCTGA |
| V. 3 | GCGCCAAGGAATTAGGCGAGAATACGTGCTGGTCATTGTGGCGCTGTGCGGGATCGCTGA |
| V. 4 | GCGCCAAGGAATTAGGCGAGAATACGTGCTGGTCATTGTGGCGCTGTGCGGGATCGCTGA |
| V. 1 | TGGGGCACTGATTGCCGCGGGCGTTGGCGGCTTCGCTGCGCTGATTCACG**CTCATCCCAATATGACTTTGGTT** |
| V. 2 | TGGGGCACTGATTGCCGCGGGCGTTGGCGGCTTCGCTGCGCTGATTCACG**CTCATCCCAATATGACTTTGGTT** |
| V. 3 | TGGGGCACTGATTGCCGCGGGCGTTGGCGGCTTCGCTGCGCTGATTCACG**CTCATCCCAATATGACTTTGGTT** |
| V. 4 | TGGGGCACTGATTGCCGCGGGCGTTGGCGGCTTCGCTGCGCTGATTCACG**CTCATCCCAATATGACTTTGGTT** |

**Tableau 11 : Variants MST12 pour M. tuberculosis : 7 variants**

| | |
|---|---|
| V. 1 | **GTTGATCGAGGCCTATCACG**ACGACGAGCGACCCGAGCAGGCGCGAGAGCCCGAACTGCGGTCAAACAGGTCACAACGAG |
| V. 2 | **GTTGATCGAGGCCTATCACG**ACGACGAGCGACCCGAGCAGGCGCGAGAGCCCGAACTGCGGTCAAACAGGTCACAACGAG |
| V. 3 | **GTTGATCGAGGCCTATCACG**ACGACGAGCGACCCGAGCAGGCGCGACAGCCCGAACTGCGGTCAAACAGGTCACAACGAG |
| V. 4 | **GTTGATCGAGGCCTATCACG**ACGACGAGCGACCCGAGCAGGCGCGAGAGCCCGAACTGCGGTCAAACAGGTCACAACGAG |
| V. 5 | **GTTGATCGAGGCCTATCACG**ACGACGAGCGACCCGAGCAGGCGCGAGAGCCCGAACTGCGGTCAAACAGGTCACAACGAG |
| V. 6 | **GTTGATCGAGGCCTATCACG**ACGACGAGCGACCCGAGCAGGCGCGAGAGCCCGAACTGCGGTCAAACAGGTCACAACGAG |
| V. 7 | **GTTGATCGAGGCCTATCACG**ACGACGAGCGACCCGAGCAGGCGCGAGAGCCCGAACTGCGGTCAAACAGGTCACAACGAG |
| V. 1 | AGGAAGAGCTGAGCCGATGACCTGCGCCGACGACGATGCAGAGCGTAGCGATGAGGTGGG |
| V. 2 | AGGAAGAGCTGAGCCGATGACCTGCGCCGACGACGATGCAGAGCGTAGCGATGAGGTGGG |
| V. 3 | AGGAAGAGCTGAGCCGATGACCTGCGCCGACGACGATGCAGAGCGTAGCGATGAGGTGGG |
| V. 4 | AGGAAGAGCTGAGCCGATGACCTGCGCCGACGACGATGCAGAGCGTAGCGATGAGGTGGG |
| V. 5 | AGGAAGAGCTGAGCCGATGACCTGCGCCGACGACGATGCAGAGCGTAGCGATGAGGTGGG |
| V. 6 | AGGAAGAGCTGAGCCGATGACCTGCGCCGACGACGATGCAGAGCGTAGCGATGAGGTGGG |
| V. 7 | AGGAAGAGCTGAGCCGATGACCTGCGCCGACGACGATGCAGAGCGTAGCGATGAGG--------- |
| V. 1 | GGCACCACCCGCTTGCGGGGGAGAGTGGCGCTGATGACCTGCGCCGACGACGATGCAGAG |
| V. 2 | GGCACCACCCGCTTGCGGGGGAGAGTGGCGCTGATGACCTGCGCCGACGACGATGCAGAG |
| V. 3 | GGCACCACCCGCTTGCGGGGGAGAGTGGCGCTGATGACCTGCGCCGACGACGATGCAGAG |
| V. 4 | GGCACCACCCGCTTGCGGGGGAGAGTGGCGCTGATGACCTGCGCCGACGACGATGCAGAG |
| V. 5 | GGCACCACCCGCTTGCGGGGGAGAGTGGCGCTGATGACCTGCGCCGACGACGATGCAGAG |
| V. 6 | TGCACCACCCGCTTGCGGGGGAGAGTGGCGCTGATGACCTGCGCCGACGACGATGCAGAG |
| V. 7 | ---------------------------------------------------------------------------------------------------------------------------------- |
| V. 1 | CGTAGCGATGAGGTGGGGGCACCACCCGCTTGCGGGGGAGAGTGGCGCTGATCACCTGCG |
| V.2 | CGTAGCGATGAGGTGGGGGCACCACCCGCTTGCGGGGGAGAGTGGCGCTGATGACCTGCG |
| V.3 | CGTAGCGATGAGGTGGGGGCACCACCCGCTTGCGGGGGAGAGTGGCGCTGATGACCTGCG |
| V.4 | CGTAGCGATGAGGTGGGGGCACCACCCGCTTGCGGGGGAGAGTGGCGCTGATGACCTGCG |
| V. 5 | CGTAGCGATGAGGTGGGGGCACCACCCGCTTGCGGGGGAGAGTGGCGCTGATGACCTGCG |
| V. 6 | CGTAGCGATGAGGTGGGTGCACCACCCGCTTGCGGGGGAGAGTGGCGCTGATGACCTGCG |
| V. 7 | ---------------------------------------------------------------------------------------------------------------------------------- |
| V. 1 | CCGACGACGATGCAGAGCGTAGCGATGAGGTGGGGGCACCACCCGCTTGCGGGGGAGAGT |
| V. 2 | CCGACGACGATGCAGAGCGTAGCGATGAGGTGGGGGCACCACCCGCTTGCGGGGGAGAGT |
| V. 3 | CCGACGACGATGCAGAGCGTAGCGATGAGGTGGGGGCACCACCCGCTTGCGGGGGAGAGT |
| V. 4 | CCGACGACGATGCAGAGCGTAGCGATGAGGTGGGGGCACCACCCGCTTGCGGGGGAGAGT |
| V. 5 | CCGACGACGATGCAGAGCGTAGCGATGAGG----------------------------------------------------------------- |
| V. 6 | CCGACGACGATGCAGAGCGTAGCGATGAGG----------------------------------------------------------------- |
| V. 7 | ---------------------------------------------------------------------------------------------------------------------------------- |
| V. 1 | GGCGCTGATGACCTGCGCCGACGACGATGCAGAGCGTAGCGATGAGGTGGGGGCACCACC |
| V. 2 | GGCGCTGATGACCTGCGCCGACGACGATGCAGAGCGTAGCGATGAGGTGGGGGCACCACC |
| V. 3 | GGCGCTGATGACCTGCGCCGACGACGATGCAGAGCGTAGCGATGAGG-------------------------- |
| V. 4 | GGCGCTGATGACC----------------------------------------------------------------------------------------------------- |
| V. 5 | --------------------------------------------------------------------------------------------------------------------------------- |
| V. 6 | --------------------------------------------------------------------------------------------------------------------------------- |
| V. 7 | --------------------------------------------------------------------------------------------------------------------------------- |
| V. 1 | CGCTTGCGGGGGAGAGTGGCGCTGATGACCTGCGCCGACGACGATGCAGAGCGTAGCGAT |
| V. 2 | CGCTTGCGGGGGAGAGTGGCGCTGATGACCTGCGCCGACGACGATGCAGAGCGTAGCGAT |
| V. 3 | ---------------------------------------------------------------------------------------------------------------------------------- |
| V. 4 | ---------------------------------------------------------------------------------------------------------------------------------- |
| V. 5 | ---------------------------------------------------------------------------------------------------------------------------------- |
| V. 6 | ---------------------------------------------------------------------------------------------------------------------------------- |
| V. 7 | ---------------------------------------------------------------------------------------------------------------------------------- |
| V. 1 | GAGGTGGGGGCACCACCCGCTTGCGGGGGAGAGTGGCGCTGATGACCTGCGCCGACGACG |
| V. 2 | GAGG------------------------------------------------------------------------------------------------------------------------- |
| V. 3 | -------------------------------------------------------------------------------------------------------------------------------- |
| V. 4 | -------------------------------------------------------------------------------------------------------------------------------- |
| V. 5 | -------------------------------------------------------------------------------------------------------------------------------- |
| V. 6 | -------------------------------------------------------------------------------------------------------------------------------- |
| V. 7 | -------------------------------------------------------------------------------------------------------------------------------- |
| V. 1 | ATGCAGAGCGTAGCGATGAGGAGGAGTGGCGCTGATGACCAGTGTGTTGATTGTGGAGGA |
| V. 2 | ---------------------------------------------AGGAGTGGCGCTGATGACCAGTGTGTTGATTGTGGAGGA |
| V. 3 | ---------------------------------------------AGGAGTGGCGCTGATGACCAGTGTGTTCATTGTGGAGGA |
| V. 4 | --------------------------------------------------------------------------------------AGTGTGTTGATTGTGGAGGA |
| V. 5 | ---------------------------------------------AGGAGTGGCGCTGATGACCAGTGTGTTGATTGTGGAGGA |
| V. 6 | ---------------------------------------------AGGAGTGGCGCTGATGACCAGTGTGTTGATTGTGGAGGA |
| V. 7 | ---------------------------------------------AGGAGTGGCGCTGATGACCAGTGTGTTGATTGTGGAGGA |
| V. 1 | CGAGGAGTCGCTGGCCGATCCGCTGGCGTTTCTGCTGCGCAAGGAGGGCTTTGAGGCCAC |
| V. 2 | CGAGGAGTCGCTGGCCGATCCGCTGGCGTTTCTGCTGCGCAAGGAGGGCTTTGAGGCCAC |
| V. 3 | CGAGGAGTCGCTGGCCGATCCGCTGGCGTTTCTGCTGCGCAAGGAGGGCTTTGAGGCCAC |
| V. 4 | CGAGGAGTCGCTGGCCGATCCGCTGGCGTTTCTGCTGCGCAAGGAGGGCTTTGAGGCCAC |
| V. 5 | CGAGGAGTCGCTGGCCGATCCGCTGGCGTTTCTGCTGCGCAAGGAGGGCTTTGAGGCCAC |
| V. 6 | CGAGGAGTCGCTGGCCGATCCGCTGGCGTTTCTGCTGCGCAAGGAGGGCTTTGAGGCCAC |
| V. 7 | CGAGGAGTCGCTGGCCGATCCGCTGGCGTTTCTGCTGCGCAAGGAGGGCTTTGAGGCCAC |
| V. 1 | GGTGGTGACCGATGGTCCGGCAGCTCTCGCCGAGTTCGACCGGGCCGGCGCCGACATCGT |
| V. 2 | GGTGGTGACCGATGGTCCGGCAGCTCTCGCCGAGTTCGACCGGGCCGGCGCCGACATCGT |
| V. 3 | GGTGGTGACCGATGGTCCGGCAGCTCTCGCCGAGTTCGACCGGGCCGGCGCCGACATCGT |
| V. 4 | GGTGGTGACCGATGGTCCGGCAGCTCTCGCCGAGTTCGACCGGGCCGGCGCCGACATCGT |
| V. 5 | GGTGGTGACCGATGGTCCGGCAGCTCTCGCCGAGTTCGACCGGGCCGGCGCCGACATCGT |
| V. 6 | GGTGGTGACCGATGGTCCGGCAGCTCTCGCCGAGTTCGACCGGGCCGGCGCCGACATCGT |
| V. 7 | GGTGGTGACCGATGGTCCGGCAGCTCTCGCCGAGTTCGACCGGGCCGGCGCCGACATCGT |
| V. 1 | CCTGCTCGATCTGATGCTGCCTGGGATGTCGGGTACCGATGTATGCAAGCAGTTGCGCGC**TACGTGACCAAGCCCTATTC** |
| V. 2 | CCTGCTCGATCTGATGCTGCCTGGGATGTCGGGTACCGATGTATGCAAGCAGTTGCGCGC**TACGTGACCAAGCCCTATTC** |
| V. 3 | CCTGCTCGATCTGATGCTGCCTGGGATGTCGGGTACCGATGTATGCAAGCAGTTGCGCGC**TACGTGACCAAGCCCTATTC** |
| V. 4 | CCTGCTCGATCTGATGCTGCCTGGGATGTCGGGTACCGATGTATGCAAGCAGTTGCGCGC**TACGTGACCAAGCCCTATTC** |
| V. 5 | CCTGCTCGATCTGATGCTGCCTGGGATGTCGGGTACCGATGTATGCAAGCAGTTGCGCGC**TACGTGACCAAGCCCTATTC** |
| V. 6 | CCTGCTCGATCTGATGCTGCCTGGGATGTCGGGTACCGATGTATGCAAGCAGTTGCGCGC**TACGTGACCAAGCCCTATTC** |
| V. 7 | CCTGCTCGATCTGATGCTCCCTGGGATGTCGGGTACCGATGTATGCAAGCAGTTGCGCGC**TACGTGACCAAGCCCTATTC** |

**Tableau 12 : Variants MST13 pour M. tuberculosis : 11 variants**

| | |
|---|---|
| V. 1 | **CGAGTTCACCGTCCATCATC**TTGGTGAACAAATCCAGCCCCACCGCACGGACCAGGGGTTCGGCGAACGGATCGTTGATC |
| V. 7 | **CGAGTTCACCGTCCATCATC**TTGGTGAACAAATCCAGCCCCACCGCACGGACCAGGGGTTCGGCGAACGGATCGTTGATC |
| V. 3 | **CGAGTTCACCGTCCATCATC**TTGGTGAACAAATCCAGCCCCACCGCACGGACCAGGGGTTCGGCGAACGGATCGTTGATC |
| V. 6 | **CGAGTTCACCGTCCATCATC**TTGGTGAACAAATCCAGCCGCACCGCACGGACCAGGGGTTCGGCGAACGGATCGTTGATC |
| V. 4 | **CGAGTTCACCGTCCATCATC**TTGGTGAACAAATCCAGCCCCACCGCACGGACCAGGGGTTCGGCGAACGGATCGTTGATC |
| V. 11 | **CGAGTTCACCGTCCATCATC**TTGGTGAACAAATCCAGCCCCACCGCACGGACCAGGGGTTCGGCGAACGGATCGTTGATC |
| V. 8 | **CGAGTTCACCGTCCATCATC**TTGGTGAACAAATCCAGCCCCACCGCACGGACCAGGGGTTCGGCGAACGGATCGTTGATC |
| V. 9 | **CCAGTTCACCGTCCATCATC**TTGGTGAACAAATCCAGCCCCACCGCACGGACCAGGGGTTCGGCGAACGGATCGTTGATC |
| V. 5 | **CGAGTTCACCGTCCATCATC**TTGGTGAACAAATCCAGCCCCACCGCACGGACCAGGGGTTCGGCGAACGGATCGTTGATC |
| V. 10 | **CGAGTTCACCGTCCATCATC**TTGGTGAACAAATCCAGCCCCACCGCACGGACCAGCGGTTCGGCGAACGGATCGTTGATC |
| V. 2 | **CGAGTTCACCGTCCATCATC**TTGGTGAACAAATCCAGCCCCACCGCACGGACCAGGGGTTCGGCGAACGGATCGTTGATC |
| V. 1 | AAACCGCGCGGATCCTTGGTGGCCAGCGCACGCCCGACCGCGACAATGGTCGCGGTGACG |
| V. 7 | AAACCGCGCGGATCCTTGGTGGCCAGCGCACGCCCGACCGCGACAATGGTCGCGGTGACG |
| V. 3 | AAACCGCGCGGATCCTTGGTGGCCAGCGCACGCCCGACCGCGACAATGGTCGCGGTGACG |
| V. 6 | AAACCGCGCGGATCCTTGGTGGCCAGCGCACGCCCGACCGCGACAATGGTCGCGGTGACG |
| V. 4 | AAACCGCGCGGATCCTTGGTGGCCAGCGCACGCCCGACCGCGACAATGGTCGCGGTGACG |
| V. 11 | AAACCGCGCGGATCCTTGGTGGCCAGCGCACGCCCGACCGCGACAATGGTCGCGGTGACG |
| V. 8 | AAACCGCGCGGATCCTTGGTGGCCAGCGCACGCCCGACCGCGACAATGGTCGCGGTGACG |
| V. 9 | AAACCGCGCGGATCCTTGGTGGCCAGCGCACGCCCGACCGCGACAATGGTCGCGGTGACG |
| V. 5 | AAACCGCGCGGATCCTTGGTGGCCAGCGCACGCCCGACCGCGACAATGGTCGCGGTGACG |
| V. 10 | AAACCGCGCGGATCCTTGGTGGCCAGCGCACGCCCGACCGCGACAATGGTCGCGGTGACG |
| V. 2 | AAACCGCGCGGATCCTTGGTGGCCAGCGCACGCCCGACCGCGACAATGGTCGCGGTGACG |
| V. 1 | CCGACGCTAGACGTCAGATCCCAGTTGTCGTCGTCGGTGCGGGCCACCAGCCCACCCTAG |
| V. 7 | CCGACGCTAGACGTCAGATCCCAGTTGTCGTCGTCGGTGCGGGCCACCAGCCCACCCTAG |
| V. 3 | CCGACGCTAGACGTCAGATCCCAGTTGTCGTCGTCGGTGCGGGCCACCAGCCCACCCTAG |
| V. 6 | CCGACGCTAGACGTCAGATCCCAGTTGTCGTCGTCGGTGCGGGCCACCAGCCCACCCTAG |
| V. 4 | CCGACGCTAGACGTCAGATCCCAGTTGTCGTCGTCGGTGCGGGCCACCAGCCCACCCTAG |
| V. 11 | CCGACGCTAGACGTCAGATCCCAGTTGTCGTCGTCGGTGCGGGCCACCAGCCCACCCTAG |
| V. 8 | CCGACGCTAGACGTCAGATCCCAGTTGTCGTCGTCGGTGCGGGCCACCAGCCCACCCTAG |
| V. 9 | CCGACGCTAGACGTCAGATCCCAGTTGTCGTCGTCGGTGCGGGCCACCAGCCCACCCTAG |
| V. 5 | CCGACGCTAGACGTCAGATCCCAGTTGTCGTCGTCGGTGCGGGCCACCAGCCCACCCTAG |
| V. 10 | CCGACGCTAGACGTCAGATCCCAGTTGTCGTCGTCGGTGCGGGCCACCAGCCCACCCTAG |
| V. 2 | CCGACGCTAGACGTCAGATCCCAGTTGTCGTCGTCGGTGCGGGCCACCAGCCCACCCTAG |
| V. 1 | TCTGATTGCCCGGTTCCTCCTCGCGCCGCAAACGGCGCGCATCGTCACCGGGCGTAGTCT |
| V. 7 | TCTGATTGCCCGGTTCCTCCTCGCGCCGCAAACGGCGCGCATCGTCACCGGGCGTAGTCT |
| V. 3 | TCTGATTGCCCGGTTCCTCCTCGCGCCGCAAACGGCGCGCATCGTCACCGGGCGTCGTCT |
| V. 6 | TCTGATTGCCCGGTTCCTCCTCGCGCCGCAAACGGCGCGCATCGTCACCGGGCGTAGTCT |
| V. 4 | TCTGATTGCCCGGTTCCTCCTCGCGCCGCAAACGGCGCGCATCGTCACCGGGCGTCGTCT |
| V. 11 | TCTGATTGCCCGGTTCCTCCTCGCGCCGCAAACGGCGCGCATCGTCACCGGGCGTCGTCT |
| V. 9 | TCTGATTGCCCGGTTCCTCCTCGCGCCGCAAACGGCGCGCATCGTCACCGGGCGTAGTCT |
| V. 9 | TCTGATTGCCCGGTTCCTCCTCGCGCCGCAAACGGCGCGCATCGTCACCGGGCGTAGTCT |
| V. 5 | TCTGATTGCCCGGTTCCTCCTCGCGCCGCAAACGGCGCGCATCGTCACCGGGCGTCGTCT |
| V. 10 | TCTGATTGCCCGGTTCCTCCTCGCGCCGCAAACGGCGCGCATCGTCACCGGGCGTCGTCT |
| V. 2 | TCTGATTGCCCGGTTCCTCCTCGCGCCGCAAAC---------------------------------------------------------- |
| V. 1 | GATTGCCCGGTTCCTCCTCGCGCCGCAAACGGCGCGCATCGTCACCGGGCGTAGTCTGAT |
| V. 7 | GATTGCCCGGTTCCTCCTCGCGCCGCAAACGGCGCGCATCGTCACCGGGCGTAGTCTGAT |
| V. 3 | GATTGCCCGGTTCCTCCTCGCGCCGCAAACGGCGCGCATCGTCACCGGGCGTCGTCTGAT |
| V. 6 | GATTGCCCGGTTCCTCCTCGCGCCGCAAACGGCGCGCATCGTCACCGGGCGTAGTCTGAT |
| V. 4 | GATTGCCCGGTTCCTCCTCGCGCCGCAAACGGCGCGCATCGTCACCGGGCGT---------------- |
| V. 11 | GATTGCCCGGTTCCTCCTCGCGCCGCAAACGGCGCGCATCGTCACCGGGCGT--------------- |
| V. 8 | GATTGCCCGGTTCCTCCTCGCGCCGCAAACGGCGCGCATCGTCACCGGGCGT---------------- |
| V. 9 | GATTGCCCGGTTCCTCCTCGCGCCGCAAACGGCGCGCATCGTCACCGGGCGTAGTCTGAT |
| V. 5 | GATTGCCCGGTTCCTCCTCGCGCCGCAAACGGCGCGCATCGTCACCGGGCGTC-------------- |
| V. 10 | GATTGCCCGGTTCCTCCTCGCGCCGCAAAC--------------------------------------------------------------- |
| V. 2 | -------------------------------------------------------------------------------------------------------------------------------- |
| V. 1 | TGCCCGGTTCCTCCTCGCGCCGCAAACGGCGCGCATCGTCACCGGGCGT----- |
| V. 7 | TGCCCGGTTCCTCCTCGCGCCGCAAACGGCGCGCATCGTCACCGGGCGTAGTCTGATTGC |
| V. 3 | TGCCCGGTTCCTCCTCGCGCCGCAAACGGCGCGCATCGTCACCGGGCGTAGTCTGATTGC |
| V. 6 | TGCCCGGTTCCTCCTCGCGCCGCAAACGGCGCGCATCGTCACCGGGCGT----------------------- |
| V. 4 | -------------------------------------------------------------------------------------------------------------------------------- |
| V. 11 | ------------------------------------------------------------------------------------------------------------------------------ |
| V. 8 | ------------------------------------------------------------------------------------------------------------------------------- |
| V. 9 | TGCCCGGTTCCTCCTCGCGCCGCAAACGGCGCGCATCGTCACCGGGCGTAGTCTGATTGC |
| V. 5 | ------------------------------------------------------------------------------------------------------------------------------- |
| V. 10 | ------------------------------------------------------------------------------------------------------------------------------ |
| V. 12 | ------------------------------------------------------------------------------------------------------------------------------ |
| V. 1 | ---------------------------------------------------------------------------------------------------CGTCTGATTGCCCG |
| V. 7 | CCGGTTCCTCCTCGCGCCGCAAACGGCGCGCATCGTCACCGGGCGTCGTCTGATTGCCCG |
| V. 3 | -------------------------------------------------------------------------------------------------------------------------------- |
| V. 6 | -------------------------------------------------------------------------------------------------------------------------------- |
| V. 4 | -------------------------------------------------------------------------------------------------------------------------------- |
| V. 11 | ------------------------------------------------------------------------------------------------------------------------------- |
| V. 8 | -------------------------------------------------------------------------------------------------------------------------------- |
| V. 9 | CCGGTTCCTCCTCGCGCCGCAAACGGCGCGCATCGTCACCGGGCGTAGTCTGATTGCCCG |
| V. 5 | -------------------------------------------------------------------------------------------------------------------------------- |
| | V. 10 ------------------------------------------------------------------------------------------------------------------------------- |
| V. 2 | -------------------------------------------------------------------------------------------------------------------------------- |
| V. 1 | GTTCCTCCTCGCGCCGCAAACGGCGCGCATCGTCACCGGGCGTCGTCTGATTGCCCGGTT |
| V. 7 | GTTCCTCCTCGCGCCGCAAACGGCGCGCATCGTCACCGGGCGTCGTCTGATTGCCCGGTT |
| V. 3 | --------------------------------------------------------------------------------------------CGTCTGATTGCCCGGTT |
| V. 6 | --------------------------------------------------------------------------------------------CGTCTGATTGCCCGGTT |
| V. 4 | --------------------------------------------------------------------------------------------CGTCTGATTGCCCGGTT |
| V. 11 | -------------------------------------------------------------------------------------------CGTCTGATTGCCCGGTT |
| V. 8 | --------------------------------------------------------------------------------------------AGTCTGATTGCCCGGTT |
| V. 9 | GTTCCTCCTCGCGCCGCAAACGGCGCGCATCGTCACCGGGCGTCGTCTGATTGCCCGGTT |
| V. 5 | -------------------------------------------------------------------------------------------------------------------------------- |
| V. 10 | ------------------------------------------------------------------------------------------------------------------------------ |
| V. 2 | ------------------------------------------------------------------------------------------------------------------------------- |
| V. 1 | CCTCCTCGCGCCGCAAACGGCGCGCATCGTCACCGGGCGTC--------------------------------------- |
| V. 7 | CCTCCTCGCGCCGCAAACGGCGCGCATCGTCACCGGGCGTC--------------------------------------- |
| V. 3 | CCTCCTCGCGCCGCAAACGGCGCGCATCGTCACCGGGCGTC--------------------------------------- |
| V. 6 | CCTCCTCGCGCCGCAAACGGCGCGCATCGTCACCGGGCGTC--------------------------------------- |
| V. 4 | CCTCCTCGCGCCGCAAACGGCGCGCATCGTCACCGGGCGTC--------------------------------------- |
| V. 11 | CCTCCTCGCGCCGCAAACGGCGCGCATCGTCACCGGGCGTC------------------------------------- |
| V. 8 | CCTCCTCGCGCCGCAAACGGCGCGCATCGTCACCGGGCGTC-------------------------------------- |
| V. 9 | CCTCCTCGCGCCGCAAACGGCGCGCATCGTCACCGGGCGTGTCT**GATTGCCCGGTTCCTC** |
| V. 5 | --------------------------------------------------------------------------------------------------------------------------------- |
| V. 10 | -------------------------------------------------------------------------------------------------------------------------------- |
| V. 2 | ---------------------------------------------------------------------------------------------------------------------------------- |
| V. 1 | ---------------------------------------------------------------------------------GTCTGATTGCCCGGTTCCTCCTCG |
| V. 7 | ---------------------------------------------------------------------------------GTCTGATTGCCCGGTTCCTCCTCG |
| V. 3 | ---------------------------------------------------------------------------------GTCTGATTGCCCGGTTCCTCCTCG |
| V. 6 | ---------------------------------------------------------------------------------GTCTGATTGCCCGGTTCCTCCTCG |
| V. 4 ----- | ----------------------------------------------------------------------------GTCTGATTGCCCGGTTCCTCCTCG |
| V. 11 | --------------------------------------------------------------------------------CTCTGATTGCCCGGTTCCTCCTCG |
| V. 8 | ----------------------------------------------------------------------------------GTCTGATTGCCCGGTTCCTCCTCG |
| V. 9 | **CTCGCGCCGCAAACGGCGCGCATCGTCACCGGGCGT**GTCTGATTGCCCGGTTCCTCCTCG |
| V. 5 | -----------------------------------------------------------------------------------GTCTGATTGCCCGGTTCCTCCTCG |
| V. 10 | ----------------------------------------------------------------------------------------------------------------------------------- |
| V. 2 | ------------------------------------------------------------------------------------------------------------------------------------- |
| V. 1 | CGCCCCAAACCAAGCCGGCTGGTGCTGTGCTATTGGCGTCGGAACAGACGGCCGTGCTGG |
| V. 7 | CGCCGCAAACCAAGCCGGCTGGTGCTGTGCTATTGGCGTCGGAACAGACGGCCGTGCTGG |
| V. 3 | CGCCGCAAACCAAGCCGGCTGGTGCTGTGCTATTGGCGTCGGAACAGACGGCCGTGCTGG |
| V. 6 | CGCCGCAAACCAAGCCGGCTGGTGCTGTGCTATTGGCGTCGGAACAGACGGCCGTGCTGG |
| V. 4 | CGCCGCAAACCAAGCCGGCTGGTGCTGTGCTATTGGCGTCGGAACAGACGGCCGTGCTGG |
| V. 11 | CGCCGCAAACCAAGCCGGCTGGTGCTGTGCTATTGGCGTCGGAACAGACGGCCGTGCTGG |
| V. 8 | CGCCGCAAACCAAGCCGGCTGGTGCTGTGCTATTGGCGTCGGAACAGACGGCCGTGCTGG |
| V. 9 | CGCCGCAAACCAAGCCGGCTGGTGCTGTGCTATTGGCGTCGGAACAGACGGCCGTGCTGG |
| V. 5 | CGCCGCAAACCAAGCCGGCTGGTGCTGTGCTATTGGCGTCGGAACAGACGGCCGTGCTGG |
| V. 10 | --------------------CAAGCCGGCTGGTGCTGTGCTATTGGCGTCGGAACAGACGGCCGTGCTGG |
| V. 2 | ----------------------CAAGCCGGCTGGTGCTGTGCTATTGGCGTCGGAACAGACGGCCGTGCTGG |
| V. 1 | TACAGAACCAGGCGATGTTGCCGTCCGGCCCGCGCACGAAGTTGGAGCGACTGCCGGTG |
| V. 7 | TACAGAACCAGGCGATGTTGCCGTCCGGCCCGCGCACCAAGTTGGAGCGACTGCCGGTG |
| V. 3 | TACAGAACCAGGCGATGTTGCCGTCCGGCCCGCGCACGAAGTTGGAGCGACTGCCGGTG |
| V. 6 | TACAGAACCAGGCGATGTTGCCGTCCGGCCCGCGCACGAAGTTGGAGCGACTGCCGGTG |
| V. 4 | TACAGAACCAGGCGATGTTGCCGTCCGGCCCGCGCACGAAGTTGGAGCGACTGCCGGTG |
| V. 11 | TACAGAACCAGGCGATGTTGCCGTCCGGCCCGCGCACGAAGTTGGAGCGACTGCCGGTG |
| V. 8 | TACAGAACCAGGCGATGTTGCCGTCCGGCCCGCTCACGAAGTTGGAGCGACTGCCGGTG |
| V. 9 | TACAGAACCAGGCGATGTTGCCGTCCGGCCCGCGCACGAAGTTGGAGCGACTGCCGGTG |
| V. 5 | ACAGAACCAGGCGATGTTGCCGTCCGGCCCGCGCACGAAGTTGGAGCGACTGCCGGTG |
| V. 10 | TACAGAACCAGGCGATGTTGCCGTCCGGCCCGCGCACGAAGTTGGAGCGACTGCCGGTG |
| V. 2 | ACAGAACCAGGCGATGTTGCCGTCCGGCCCGCGCACGAAGTTGGAGCGACTGCCGGTG |
| V. 1 | GCTTGTTGTCGGGTCCAAGGTCGAGCCCATAGTCGGGCCGATAGAAGGCGAGGCCCAGA |
| V. 7 | GCTTGTTGTCGGGTCCAAGGTCGAGCCCATAGTCGGGCCGATAGAAGGCGAGGCCCAGA |
| V. 3 | GCTTGTTGTCGGGTCCAAGGTCGAGCCCATAGTCGGGCCGATAGAAGGCGAGGCCCAGA |
| V. 6 | GCTTGTTGTCGGGTCCAAGGTCGAGCCCATAGTCGGGCCGATAGAAGGCGAGGCCCAGA |
| V. 4 | GCTTGTTGTCGGGTCCAAGGTCGAGCCCATAGTCGGGCCGATAGAAGGCGAGGCCCAGA |
| V. 11 | GCTTGTTGTCGGGTCCAAGGTCGAGCCCATAGTCGGGCCGATAGAAGGCGAGGCCCAGA |
| V. 8 | GCTTGTTGTCGGGTCCAAGGTCGAGCCCATAGTCGGGCCGATAGAAGGCGAGGCCCAGA |
| V. 9 | GCTTGTTGTCGGGTCC-------------------------------------------------------------------------------------------- |
| V. 5 | GCTTGTTGTCGGGTCCAAGGTCGAGCCCATAGTCGGGCCGATAGAAGGCGAGGCCCAGA |
| V. 10 | GCTTGTTGTCGGGTCCAAGGTCGAGCCCATAGTCGGGCCGATAGAAGGCGAGGCCCAGA |
| V. 2 | GCTTGTTGTCGGGTCCAAGGTCGAGCCCATAGTCGGGCCGATAGAAGGCGAGGCCCAGA |
| V. 1 | TGGCGCTGTTTTGGCCATCCGGGTTGGCATCGTCGGTGCTCATGCTTCCAGCAAGC**AAGTCGATGACCGTTGTCTC** |
| V. 7 | TGGCGCTGTTTTGGCCATCCGGGTTGGCATCGTCGCTGCTCATGCTTCCAGCAAGC**AAGTCGATGACCGTTGTCTC** |
| V. 3 | GGCGCTGTTTTGGCCATCCGGGTTGGCATCGTCGGTGCTCATGCTTCCAGCAAGC**AAGTCGATGACCGTTGTCTC** |
| V. 6 | TGGCGCTGTTTTGGCCATCCGGGTTGGCATCGTCGGTGCTCATGCTTCCAGCAAGC**AAGTCGATGACCGTTGTCTC** |
| V. 4 | GGCGCTGTTTTGGCCATCCGGGTTGGCATCGTCGGTGCTCATGCTTCCAGCAAGC**AAGTCGATGACCGTTGTCTC** |
| V. 11 | TGGCGCTGTTTTGCCCATCCGGGTTGGCATCGTCTGTGCTCATGCTTCCAGCAAGC**AAGTCGATGACCGTTGTCTC** |
| V. 8 | GGCGCTGTTTTGGCCATCCGGGTTGGCATCGTCGGTGCTCATGCTTCCAGCAAGC---------------------------------------------- |
| V. 9 | ----------------------------------------------------------------------------------------------------------------------**AAGTCGATGACCGTTGTCTC** |
| V. 5 | TGGCGCTGTTTTGGCCATCCGGGTTGGCATCGTCGGTGCTCATGCTTCCACCAAGC**AAGTCGATGACCGTTGTCTC** |
| V. 10 | TGGCGCTGTTTTGGCCATCCGGGTTGGCATCGTCGGTGCTCATGCTTCCAGCAAGC**AAGTCGATGACCGTTGTCTC** |
| V. 2 | GGCGCTGTTTTGGCCATCCGGGTTGGCATCGTCGGTGCTCATGCTTCCAGCAAGC**AAGTCGATGACCGTTGTCTC** |

**Tableau 13 : identification des espèces par l'analyse de la séquence du spacer MST11 :**

| |
|---|
| *M. tuberculosis* 1 |
| **AGGTGTTAGAGGTGGTGGAT**TCGGACTTCAATGCGTTGTTGGAGTTGGGATTTCGGTCGTCGATTCAACGAGAGTGGCAG |
| *M. tuberculosis* 2 |
| **AGGTGTTAGAGGTGGTGGAT**TCGGACTTCAATGCGTTGTTCGAGTTGGGATTTCGGTCGTCGATTCAACGAGAGTGGCAG |
| *M. bovis* |
| **AGGTGTTAGAGGTGGTGGAT**TCGGACTTCAATGCGTTGTTCGAGTTGGGATTTCGGTCGTCGATTCAACGAGAGTGGCAG |
| *M. africanum* |
| **AGGTGTTAGAGGTGGTGGAT**TCGGACTTCAATGCGTTGTTGGAGTTGGGATTTCGGTCGTCGATTCAACGAGAGTGGCAG |
| *M. bovis* ssp BCG |
| **AGGTGTTAGAGGTGGTGGAT**TCGGACTTCAATGCGTTGTTGGAGTTGGGATTTCGGTCGTCGATTCAACGAGAGTGGCAG |
| *M. tuberculosis* 3 |
| **AGGTGTTAGAGGTGGTGGAT**TCGGACTTCAATGCGTTGTTGGAGTTGGGATTTCGGTCGTCGATTCAACGAGAGTGGCAG |
| *M*. *canettii* |
| **AGGTGTTAGAGGTGGTGGAT**TCGGACTTCAATGCGTTGTTGGAGTTGGGATTTCGGTCGTCGATTCAACGAGAGTGGCAG |
| *M. tuberculosis* 4 |
| **AGGTGTTAGAGGTGGTGGAT**TCGGACTTCAATGCGTTGTTGGAGTTGGGATTTCGGTCGTCGATTCAACGAGAGTGGCAG |
| *M*. *tuberculosis* 1 |
| TGGCGGTTATCTCGCGGTGAGTCGCTGCAGAACCTGCAGGCCTTCGCTCACTTACGCCCG |
| *M. tuberculosis* 2 |
| TGGCGGTTATCTCGCGGTGAGTCGCTGCAGAACCTGCAGGCCTTCGCTCACTTACGCCTG |
| *M. bovis* |
| TGGCGGTTATCTCGCGGTGAGTCGCTGCAGAACCTGCAGGCCTTCGCTCACTTACGCCCG |
| *M*. *africanum* |
| TGGCGGTTATCTCGCGGTGAGTCGCTGCAGAACCTGCAGGCCTTCGCTCACTTACGCCCG |
| *M. bovis ssp* BCG |
| TGGCGGTTATCTCGCGGTGAGTCGCTGCAGAACCTGCAGGCCTTCGCTCACTTACGCCCG |
| *M. tuberculosis* 3 |
| TGGCGGTTATCTCGCGGTGAGTCGCTGCAGAACCTGCAGGCCTTCGCTCACTTACGCCCG |
| *M. canettii* |
| TGGCGGTTATCTCGCGGTGAGTCGCTGCAGAACATGCAGGCCTTCGCTCACTTGCGCCCG |
| *M. tubercu*/*osis* 4 |
| TGGCGGTTATCTCGCGGTGAGTCGCTGCAGAACCTGCAGGCCTTCGCTCACTTACGCCCG |
| *M. tuberculosis* 1 |
| ACGACGATGCAGAGCGCGCAGCGCGATGAGAAGGAGTTGGGCGGTTAGGTCGAGCCCGAC |
| *M. tuberculosis* 2 |
| ACGACGATGCAGAGCGCGCAGCGCGATGAGAAGGAGTTGGGCGGTTAGGTCGAGCCCGAC |
| *M. bovis* |
| ACGACGATGCAGAGCGCGCAGCGCGATGAGAAGGAGTTGGGCGGTTAGGTCGAGCCCGAC |
| *M. africanum* |
| ACGACGATGCAGAGCGCGCAGCGCGATGAGAAGGAGTTGGGCGGTTAGGTCGAGCCCGAC |
| *M. bovis ssp* BCG |
| ACGACGATGCAGAGCGCGCAGCGCGATGAGAAGGAGTTGGGCGGTTAGGTCGAGCCCGAC |
| *M. tuberculosis* 3 |
| ACGACGATGC.AGAGCGCGCAGCGCGATGAGAAGGAGTTGGGCGGTTAGGTCGAGCCCGAC |
| *M. canettii* |
| ACGACGATGCAGAGCGCGCAGCGCGATGAGAAGGAGTTGGGCGGTTAGGTCGAGCCCGAC |
| *M. tuberculosis* 4 |
| ACGACGATGCAGAGCGCGCAGCGCGATGAGAAGGAGTTGGGCGGTTAGGTCGAGCCCGAC |
| *M. tuberculosis* 1 |
| GACGATGCAGAGCGCGCAGCGCGATGAGAAGGAGTTGGGCGGTTAGGTCGAGCCCGACGA |
| *M. tuberculosis* 2 |
| GACGATGCAGAGCGCGCAGCGCGATGAGAAGGAGTTGGGCGGTTAGGTCGAGCCCGACGA |
| *M. bovis* |
| GACGATGCAGAGCGCGCAGCGCGATGAGAAGGAGTTGGGCGGTTAGGTCGAGCCCGACGA |
| *M. africanum* |
| GACGATGCAGAGCGCGCAGCGCGATGAGAAGGAGTTGGGCGGTTAGGTCGAGCCCGACGA |
| *M. bovis* ssp BCG |
| GACGATGCAGAGCGCGCAGCGCGATGAGAAGGAGTTGGGCGGTTAGGTCGAGCCCGACGA |
| *M. tuberculosis* 3 |
| GACGATGCAGAGCGCGCAGCGCGATGAGAAGGAGTTGGGCGGTTAGGTCGAGCCCGACGA |
| *M. canettii* |
| GACGATGCAGAGCGCGCAGCGCGATGAGAAGGAGTTGGGCGGTTAGGTCGAGCCCGACGA |
| *M. tuberculosis* 4 |
| GACGATGCAGAGCGCGCAGCGCGATGAGAA----------------------------------------------------------------- |
| *M. tuberculosis* 1 |
| CGATGCAGAGCGCGCAGCGCGATGAGAAGGAGTTGGGCGGTTAGGTCGAGCCCGACGACG |
| *M. tuberculosis* 2 |
| CGATGCAGAGCGCGCAGCGCGATGAGAAGGAGTTGGGCGGTTAGGTCGAGCCCGACGACG |
| *M. bovis* |
| CGATGCAGAGCGCGCAGCGCGATGAGAAGGAGTTGGGCGGTTAGGTCGAGCCCGACGACG |
| *M. africanum* |
| CGATGCAGAGCGCGCAGCGCGATGAGAAGGAGTTGGGC-GGTTAGGTCGAGCCCGACGACG |
| *M. bovis* ssp BCG |
| CGATGCAGAGCGCGCAGCGCGATGAGAAGGAGTTGGGCGGTTAGGTCGAGCCCGACGACG |
| M*. tuberculosis* 3 |
| CGATGCAGAGCGCGCAGCGCGATGAGAA---------------------------------------------------------------------- |
| *M. canettii* |
| CGATGCAGAGCGCGCAGCGCGATGAGAA----------------------------------------------------------------------- |
| *M. tuberculosis* 4 |
| ------------------------------------------------------------------------------------------------------------------------------------ |
| *M. tuberculosis* 1 |
| ATGCAGAGCGCGCAGCGCGATGAGAA------------------------------------------------------------------------ |
| *M. tuberculosis* 2 |
| ATGCAGAGCGCGCAGCGCGATGAGAA------------------------------------------------------------------------- |
| *M. bovis* |
| ATGCAGAGCGCGCAGCGCGATGAGAAGGAGTTGGGCGGTTAGGTCGAGCCCGACGACGAT |
| *M. africanum* |
| ATGCAGAGCGCGCAGCGCGATGAGAAGGAGTTGGGfGGTTAGGTCGAGC------------------------- |
| *M. bovis ssp BCG* |
| ATGCAGAGCGCGCAGCGCGATGAGAAGGAGTTGGGCGGTTAGGTCGAGC------------------------ |
| *M. tuberculosis* 3 |
| ----------------------------------------------------------------------------------------------------------------------------------- |
| *M. canettii* |
| ------------------------------------------------------------------------------------------------------------------------------------ |
| *M. tuberculosis* 4 |
| ------------------------------------------------------------------------------------------------------------------------------------ |
| *M. tuberculosis* 1 |
| --------------------------------------------------------------------------------------------------------------------------------- |
| *M. tuberculosis* 2 |
| --------------------------------------------------------------------------------------------------------------------------------- |
| *M. bovis* |
| GCAGAGCGCGCAGCGCGATGAGAAGGAGTTGGGCGGTTAGGTCGAGCCCGACGACGATGC |
| *M. africanum* |
| ------------------------------------------------------------------------------------------TCGAGCCCGACGACGATGC |
| *M. bovis* ssp BCG |
| ---------------------------------------------------------------------------------------------------------CGACGACGATGC |
| *M. tuberculosis* 3 |
| ----------------------------------------------------------------------------------------------------------------------------------- |
| *M. canettii* |
| ------------------------------------------------------------------------------------------------------------------------------------ |
| *M. tuberculosis* 4 |
| ------------------------------------------------------------------------------------------------------------------------------------ |
| *M*. *tuberculosis* 1 |
| -------------------------------------------------ATAGCACTCGTGGAGGTCAAGACGCCCGCCGGTGATG |
| *M. tuberculosis* 2 |
| -------------------------------------------------ATAGCACTCGTGGAGGTCAAGACGCCCGCCGGTGATG |
| *M. bovis* |
| AGAAGCGCGCAGCGCGATGAGAAATAGCACTCGTGGAGGTCAAGACGCCCGCCGGTGATG |
| *M. africanum* |
| AGAGCCGCGCAGCGCGATGAGAAATAGCACTCGTGGAGGTCAAGACGCCCGCCGGTGATG |
| *M. bovis ssp* BCG |
| AGAG-CGCGCAGCGCGATGAGAAATAGCACTCGTGGAGGTCAAGACGCCCGCCGGTGATG |
| *M. tuberculosis* 3 |
| -------------------------------------------------ATAGCACTCGTGGAGGTCAAGACGCCCGCCGGTGATG |
| *M. canettii* |
| -------------------------------------------------ATAGCACTCGTGGAGGTCAAGACGCCCGCCGGTGATG |
| *M. tuberculosis* 4 |
| -------------------------------------------------ATAGCACTCGTGGAGGTCAAGACGCCCGCCGGTGATG |
| *M. tuberculosis* 1 |
| GGCTGGTGGCGCTCACCCCGTTCCGGACTCAGAAATTCGCGATCACAATTTGCGCGTTCA |
| *M. tuberculosis* 2 |
| GGCTGGTGGCGCTCACCCCGTTCCGGACTCAGAAATTCGCGATCACAATTTGCGCGTTCA |
| *M. bovis* |
| GGCTGGTGGCGCTCACCCCGTTCCGGACTCAGAAATTCGCGATCACAATTTGCGCGTTCA |
| *M. africanum* |
| GGCTGGTGGCGCTCACCCCGTTCCGGACTCAGAAATTCGCGATCACAATTTGCGCGTTCA |
| *M*. *bovis* ssp BCG |
| GGCTGGTGGCGCTCACCCCGTTCCGGACTCAGAAATTCGCGATCACAATTTGCGCGTTCA |
| *M. tuberculosis* 3 |
| GGCTGGTGGCGCTCACCCCGTTCCGGACTCAGAAATTCGCGATCACAATTTGCGCGTTCA |
| *M. canettii* |
| GGCTGGTGGCGCTCACCCCGTTCCGGACTCAGAAATTCGCGATCACAATTTGCGCGTTCA |
| *M. tuberculosis* 4 |
| GGCTGGTGGCGCTCACCCCGTTCCGGACTCAGAAATTCGCGATCACAATTTGCGCGTTCA |
| *M. tuberculosis* 1 |
| AGTCATTGGCATGCATGTGATGGTTTAGCGTTCCGCTGTGCCTCTTCAGGTGTTTGTCGG |
| *M. tuberculosis* 2 |
| AGTCATTGGCATGCATGTGATGGTTTAGCGTTCCGCTGTGCCTCTTCAGGTGTTTGTCGG |
| *M. bovis* |
| AGTCATTGGCATGCATGTGATGGTTTAGCGTTCCGCTGTGCCTCTTCAGGTGTTTGTCGG |
| *M*. *africanum* |
| AGTCATTGGCATGCATGTGATGGTTTAGCGTTCCGCTGTGCCTCTTCAGGTGTTTGTCGG |
| *M. bovis ssp* BCG |
| AGTCATTGGCATGCATGTGATGGTTTAGCGTTCCGCTGTGCCTCTTCAGGTGTTTGTCGG |
| *M. tuberculosis* 3 |
| AGTCATTGGCATGCATGTGATGGTTTAGCGTTCCGCTGTGCCTCTTCAGGTGTTTGTCGG |
| *M. canettii* |
| AGTCATTGGCATGCATGTGATGGTTTAGCGTTCCGCTGTGCCTCTTCAGGTGTTTGTCGG |
| *M. tuberculosis* 4 |
| AGTCATTGGCATGCATGTGATGGTTTAGCGTTCCGCTGTGCCTCTTCAGGTGTTTGTCGG |
| *M. tuberculosis* 1 |
| CTTCGTTGCCATGATGACGCTCAAGGTCGCGATCGGCCCGCAAAACGCATTTGTCCTGCG |
| *M. tuberculosis* 2 |
| CTTCCTTGCCATGATGACGCTCAAGGTCGCGATCGGCCCGCAAAACGCATTTGTCCTGCG |
| *M. bovis* |
| CTTCGTTGCCATGATGACGCTCAAGGTCGCGATCGGCCCGCAAAACGCATTTGTCCTGCG |
| *M. africanum* |
| CTTCGTTGCCATGATGACGCTCAAGGTCGCGATCGGCCCGCAAAACGCATTTGTCCTGCG |
| *M. bovis ssp* BCG |
| CTTCGTTGCCATGATGACGCTCAAGGTCGCGATCGGCCCGCAAAACGCATTTGTCCTGCG |
| *M. tuberculosis* 3 |
| CTTCGTTGCCATGATGACGCTCAAGGTCGCGATCGGCCCGCAAAACGCATTTGTCCTGCG |
| *M. canettii* |
| CTTCGTTGCCATGATGACGCTCAAGGTCGCGATCGGCCCGCAAAACGCATTTGTCCTGCG |
| *M. tuberculosis* 4 |
| CTTCGTTGCCATGATGACGCTCAAGGTCGCGATCGGCCCGCAAAACGCATTTGTCCTGCG |
| *M. tuberculosis* 1 |
| CCAAGGAATTAGGCGAGAATACGTGCTGGTCATTGTGGCGCTGTGCGGGATCGCTGATGG |
| *M. tuberculosis* 2 |
| CCAAGGAATTAGGCGAGAATACGTGCTGGTCATTGTGGCGCTGTGCGGGATCGCTGATGG |
| *M. bovis* |
| CCAAGGAATTAGGCGAGAATACGTGCTGGTCATTGTGGCGCTGTGCGGGATCGCTGATGG |
| *M. africanum* |
| CCAAGGAATTAGGCGAGAATACGTGCTG TCATTGTGGCGCTGTGCGGGATCGCTGATGG |
| *M. bovis* ssp BCG |
| CCAAGGAATTAGGCGAGAATACGTGCTGGTCATTGTGGCGCTGTGCGGGATCGCTGATGG |
| *M. tuberculosis* 3 |
| CCAAGGAATTAGGCGAGAATACGTGCTGGTCATTGTGGCGCTGTGCGGGATCGCTGATGG |
| *M. canettii* |
| CCAAGGAATTAGGCGAGAATACGTGCTGGTCATTGTGGCGCTGTGCGGGATCGCTGATGG |
| *M. tuberculosis* 4 |
| CCAAGGAATTAGGCGAGAATACGTGCTGGTCATTGTGGCGCTGTGCGGGATCGCTGATGG |
| *M. tuberculosis* 1 |
| GGCACTGATTGCCGCGGGCGTTGGCGGCTTCGCTGCGCTGATTCACGC**TCATCCCAATATGACTTTGGTT** |
| *M. tuberculosis 2* |
| GGCACTGATTGCCGCGGGCGTTGGCGGCTTCGCTGCGCTGATTCACGC**TCATCCCAATATGACTTTGGTT** |
| *M. bovis* |
| GGCACTGATTGCCGCGGGCGTTGGCGGCTTCGCTGCGCTGATTCACGC**TCATCCCAATATGACTTTGGTT** |
| *M. africanum* |
| GGCACTGATTGCCGCGGGCGTTGGCGGCTTCGCTGCGCTGATTCACGC**TCATCCCAATATGACTTTGGTT** |
| *M. bovis ssp* BCG |
| GGCACTGATTGCCGCGGGCGTTGGCGGCTTCGCTGCGCTGATTCACGC**TCATCCCAATATGACTTTGGTT** |
| *M. tuberculosis 3* |
| GGCACTGATTGCCGCGGGCGTTGGCGGCTTCGCTGCGCTGATTCACGC**TCATCCCAATATGACTTTGGTT** |
| *M. canettii* |
| GGCACTGATTGCCGCGGGCGTTGGCGGCTTCGCTGCGCTGATTCACGC**TCATCCCAATATGACTTTGGTT** |
| *M. tuberculosis 4* |
| GGCACTGATTGCCGCGGGCGTTGGCGGCTTCGCTGCGCTGATTCACGC**TCATCCCAATATGACTTTGGTT** |

**Tableau 14 : Identification des espèces par l'analyse de la séquence du spacer MST12**

| |
|---|
| *M. tuberculosis* 1 |
| **GTTGATCGAGGCCTATCACG**ACGACGAGCGACCCGAGCAGGCGCGAGAGCCCGAACTGCGGTCAAACAGGTCACAACGAG |
| *M*. *tuberculosis* 2 |
| **GTTGATCGAGGCCTATCACG**ACGACGAGCGACCCGAGCAGGCGCGAGAGCCCGAACTGCGGTCAAACAGGTCACAACGAG |
| *M tuberculosis* 3 |
| **GTTGATCGAGGCCTATCACG**ACGACGAGCGACCCGAGCAGGCGCGAGAGCCCGAACTGCGGTCAAACAGGTCACAACGAG |
| *M*. *africanum* |
| **GTTGATCGAGGCCTATCACG**ACGACGAGCGACCCGAGCAGGCGCGAGAGCCCGAACTGCGGTCAAACAGGTCACAACGAG |
| *M*. *tuberculosis* 4 |
| **GTTGATCGAGGCCTATCACG**ACGACGAGCGACCCGAGCAGGCGCGAGAGCCCGAACTGCGGTCAAACAGGTCACAACGAG |
| *M. bovis* ssp BCG |
| **GTTGATCGAGGCCTATCACG**ACGACGAGCGACCCGAGCAGGCGCGAGAGCCCGAACTGCGGTCAAACAGGTCACAACGAG |
| *M. tuberculosis* 5 |
| **GTTGATCGAGGCCTATCACGACG**ACGAGCGACCCGAGCAGGCGCGAGAGCCCGAACTGCGGTCAAACAGGTCACAACGAG |
| *M. tuberculosis* 7 |
| **GTTGATCGAGGCCTATCACG**ACGACGAGCGACCCGAGCAGGCGCGAGAGCCCGAACTGCGGTCAAACAGGTCACAACGAG |
| *M. bovis* |
| **GTTGATCGAGGCCTATCACG**ACGACGAGCGACCCGAGCAGGCGCGAGAGCCCGAACTGCGGTCAAACAGGTCACAACGAG |
| *M. canettii* |
| **GTTGATCGAGGCCTATCACG**ACGACGAGCGACCCGAGCAGGCGCGAGAGCCCGAACTGCGGTCAAACAGGTCACAACGAG |
| *M. tuberculosis* 6 |
| **GTTGATCGAGGCCTATCACG**ACGACGAGCGACCCGAGCAGGCGCGAGAGCCCGAACTGCGGTCAAACAGGTCACAACGAG |
| *M. tuberculosis* 1 |
| AGGAAGAGCTGAGCCGATGACCTGCGCCGACGACGATGCAGAGCGTAGCGATGAGGTGGG |
| *M*. *tuberculosis* 2 |
| AGGAAGAGCTGAGCCGATGACCTGCGCCGACGACGATGCAGAGCGTAGCGATGAGGTGGG |
| *M. tuberculosis* 3 |
| AGGAAGAGCTGAGCCGATGACCTGCGCCGACGACGATGCAGAGCGTAGCGATGAGGTGGG |
| *M. africanum* |
| AGGAAGAGCTGAGCTGATGACCTGCGCCGACGACGATGCAGAGCGTAGCGATGAGGTGGG |
| *M. tuberculosis* 4 |
| AGGAAGAGCTGAGCCGATGACCTGCGCCGACGACGATGCAGAGCGTAGCGATGAGGTGGG |
| *M. bovis ssp* BCG |
| AGGAAGAGCTGAGCCGATGACCTGCGCCGACGACGATGCAGAGCGTAGCGATGAGGTGGG |
| *M. tuberculosis* 5 |
| AGGAAGAGCTGAGCCGATGACCTGCGCCGACGACGATGCAGAGCGTAGCGATGAGGTGGG |
| *M. tuberculosis* 7 |
| AGGAAGAGCTGAGCCGATGACCTGCGCCGACGACGATGCAGAGCGTAGCGATGAGGTGGG |
| *M. bovis* |
| AGGAAGAGCTGAGCCGATGACCTGCGCCGACGACGATGCAGAGCGTAGCGATGAGGTGGG |
| *M. canettii* |
| AGGAAGAGCTGAGCCGATGACCTGCGCCGACGACGATGCAGAGCGTAGCGATGAGGTGGG |
| *M. tuberculosis* 6 |
| AGGAAGAGCTGAGCCGATGACCTGCGCCGACGACGATGCAGAGCGTAGCGATGAGGAGG- |
| *M. tuberculosis* 1 |
| GGCACCACCCGCTTGCGGGGGAGAGTGGCGCTGATGACCTGCGCCGACGACGATGCAGAG |
| *M. tuberculosis* 2 |
| GGCACCACCCGCTTGCGGGGGAGAGTGGCGCTGATGACCTGCGCCGACGACGATGCAGAG |
| *M. tuberculosis* 3 |
| GGCACCACCCGCTTGCGGGGGAGAGTGGCGCTGATGACCTGCGCCGACGACGATGCAGAG |
| *M*. *africanum* |
| GGCACCACCCGCTTGCGGGGGAGAGTGGCGCTGATGACCTGCGCCGACGACGATGCAGAG |
| *M. tuberculosis* 4 |
| GGCACCACCCGCTTGCGGGGGAGAGTGGCGCTGATGACCTGCGCCGACG_{A}CGATGCAGAG |
| *M. bovis* ssp BCG |
| GGCACCACCCGCTTGCGGGGGAGAGTGGCGC_{T}GATGACCTGCGCCGACGACGATGCAGAG |
| *M. tuberculosis* 5 |
| GGCACCACCCGCTTGCGGGGGAGAGTGGCGCTGATGACCTGCGCCGACGACGATGCAGAG |
| *M. tuberculosis* 7 |
| TGCACCACCCGCTTGCGGGGGAGAGTGGCGCTGATGACCTGCGCCGACGACGATGCAGAG |
| *M. bovis* |
| GGCACCACCCGCTTGCGGGGGAGAGTGGCGCTGATGACCTGCGCCGACGACGATGCAGAG |
| *M. canettii* |
| GGCACCACCCGCTTGCGGGGGAGAGTGGCGCTGATGACCTGCGCCGACGACGATGCAGAG |
| *M. tuberculosis* 6 |
| ---------------------------------------------------------------------------------------------------------------------------------- |
| *M*. *tuberculosis* 1 |
| CGTAGCGATGAGGTGGGGGCACCACCCGCTTGCGGGGGAGAGTGGCGCTGATGACCTGCG |
| *M. tuberculosis* 2 |
| CGTAGCGATGAGGTGGGGGCACCACCCGCTTGCGGGGGAGAGTGGCGCTGATGACCTGCG |
| *M. tuberculosis* 3 |
| CGTAGCGATGAGGTGGGGGCACCACCCGCTTGCGGGGGAGAGTGGCGCTGATGACCTGCG |
| *M. africanum* |
| CGTAGCGATGAGGTGGGGGCACCACCCGCTTGCGGGGGAGAGTGGCGCTGATGACCTGCG |
| *M. tuberculosis* 4 |
| CGTAGCGATGAGGTGGGGGCACCACCCGCTTGCGGGGGAGAGTGGCGCTGATGACCTGCG |
| *M. bovis* ssp BCG |
| CGTAGCGATGAGGTGGGGGCACCACCCGCTTGCGGGGGAGAGTGGCGCTGATGACCTGCG |
| *M. tuberculosis* 5 |
| CGTAGCGATGAGGTGGGGGCACCACCCGCTTGCGGGGGAGAGTGGCGCTGATGACCTGCG |
| *M. tuberculosis* 7 |
| CGTAGCGATGAGGTGGGTGCACCACCCGCTTGCGGGGGAGAGTGGCGCTGATGACCTGCG |
| *M. bovis* |
| CGTAGCGATGAGGTGGGGGCACCACCCGCTTGCGGGGGAGAGTGGCGCTGATGACC------ |
| *M. canettii* |
| CGTAGCGATGAGGAGG---------------------------------------------------------------------------------------------- |
| *M. tuberculosis* 6 |
| --------------------------------------------------------------------------------------------------------------------------------- |
| *M. tuberculosis* 1 |
| CCGACGACGATGCAGAGCGTAGCGATGAGGTGGGGGCACCACCCCCTTGCGGGGGAGAGT |
| *M. tuberculosis* 2 |
| CCCACGACGATGCAGAGCGTAGCGATGAGGTGGGGGCACCACCCGCTTGCGGGGGAGAGT |
| *M. tuberculosis* 3 |
| CCGACGACGATGCAGAGCGTAGCGATGAGGTGGGGGCACCACCCGCTTGCGGGGGAGAGT |
| *M. africanum* |
| CCGACGACGATGCAGAGCGTAGCGATGAGGTGGGGGCACCACCCGCTTGCGGGGGAGAGT |
| *M*. *tuberculosis* 4 |
| CCGACGACGATGCAGAGCGTAGCGATGAGGTGGGGGCACCACCCGCTTGCGGGGGAGAGT |
| *M. bovis ssp* BCG |
| CCGACGACGATGCAGAGCGTAGCGATGAGGTGGGGGCACCACCCGCTTGCGGGGGAGAGT |
| *M. tuberculosis* 5 |
| CCGACGACGATGCAGAGCGTAGCGATGAGGAGG----------------------------------------------------------- |
| *M. tuberculosis* 7 |
| CCGACGACGATGCAGAGCGTAGCGATGAGGAGG----------------------------------------------------------- |
| *M. bovis* |
| ----------------------------------------------------------------------------------------------------------------------------------- |
| *M. canettii* |
| ----------------------------------------------------------------------------------------------------------------------------------- |
| *M. tuberculosis* 6 |
| ----------------------------------------------------------------------------------------------------------------------------------- |
| *M*. *tuberculosis* 1 |
| GGCGCTGATGACCTGCGCCGACGACGATGCAGAGCGTAGCGATGAGGTGGGGGCACCACC |
| *M. tuberculosis* 2 |
| GGCGCTGATGACCTGCGCCGACGACGATGCAGAGCGTAGCGATGAGGTGGGGGCACCACC |
| *M. tuberculosis* 3 |
| GGCGCTGATGACCTGCGCCGACGACGATGCAGAGCGTAGCGATGAGG---------------------------- |
| *M. africanum* |
| GGCACTGATGACCTGCGCCGACGACGATGCAGAGCGTAGCGATGAGG----------------------------- |
| *M. tuberculosis* 4 |
| GGC---------------------------------------------------------------------------------------------------------------------------- |
| *M. bovis* ssp BCG |
| GGC---------------------------------------------------------------------------------------------------------------------------- |
| *M. tuberculosis* 5 |
| ----------------------------------------------------------------------------------------------------------------------------------- |
| *M. tuberculosis* 7 |
| ------------------------------------------------------------------------------------------------------------------------------------ |
| *M. bovis* |
| ------------------------------------------------------------------------------------------------------------------------------- |
| *M. canettii* |
| -------------------------------------------------------------------------------------------------------------------------------- |
| *M. tuberculosis* 6 |
| -------------------------------------------------------------------------------------------------------------------------------- |
| *M. tuberculosis* 1 |
| CGCTTGCGGGGGAGAGTGGCGCTGATGACCTGCGCCGACGACGATGCAGAGCGTAGCGAT |
| *M. tuberculosis* 2 |
| CGCTTGCGGGGGAGAGTGGCGCTGATGACCTGCGCCGACGACGATGCAGAGCGTAGCGAT |
| *M. tuberculosis* 3 |
| ---------------------------------------------------------------------------------------------------------------------------------- |
| *M. africanum* |
| --------------------------------------------------------------------------------------------------------------------------------- |
| *M*. *tuberculosis* 4 |
| ---------------------------------------------------------------------------------------------------------------------------------- |
| *M. bovis* ssp BCG |
| ------------------------------------------------------------------------------------------------------------------------------- |
| *M. tuberculosis* 5 |
| -------------------------------------------------------------------------------------------------------------------------------- |
| *M. tuberculosis* 7 |
| -------------------------------------------------------------------------------------------------------------------------------- |
| *M. bovis* |
| --------------------------------------------------------------------------------------------------------------------------------- |
| *M. canettii* |
| ---------------------------------------------------------------------------------------------------------------------------------- |
| *M. tuberculosis* 6 |
| ---------------------------------------------------------------------------------------------------------------------------------- |
| *M. tuberculosis* 1 |
| GAGGTGGGGGCACCACCCGCTTGCGGGGGAGAGTGGCGCTGATGACCTGCGCCGACGACG |
| *M. tuberculosis* 2 |
| GAGG-------------------------------------------------------------------------------------------------------------------------- |
| *M. tuberculosis* 3 |
| -------------------------------------------------------------------------------------------------------------------------------------- |
| *M. africanum* |
| -------------------------------------------------------------------------------------------------------------------------------------- |
| *M. tuberculosis* 4 |
| --------------------------------------------------------------------------------------------------------------------------------------- |
| *M. bovis* ssp BCG |
| ---------------------------------------------------------------------------------------------------------------------------------------- |
| *M. tuberculosis* 5 |
| ----------------------------------------------------------------------------------------------------------------------------------------- |
| *M. tuberculosis* 7 |
| ---------------------------------------------------------------------------------------------------------------------------------------- |
| *M. bovis* |
| ---------------------------------------------------------------------------------------------------------------------------------------- |
| *M. canettii* |
| ---------------------------------------------------------------------------------------------------------------------------------------- |
| *M*. *tuberculosis* 6 |
| -------------------------------------------------------------------------------------------------------------------------------- |
| *M*. *tuberculosis* 1 |
| ATGCAGAGCGTAGCGATGAGGAGGAGTGGCGCTGATGACCAGTGTGTTGATTGTGGAGGA |
| *M. tuberculosis* 2 |
| ---------------------------------------------AGGAGTGGCGCTGATGACCAGTGTGTTGATTGTGGAGGA |
| *M. tuberculosis* 3 |
| ----------------------------------------------AGGAGTGGCGCTGATGACCAGTGTGTTGATTGTGGAGGA |
| *M. africanum* |
| ---------------------------------------------AGGAGTGGCGCTGATGACCAGTGTGTTGATTGTGGAGGA |
| *M. tuberculosis* 4 |
| ------------------------------------------------------------------GCTGATGACCAGTGTGTTGATTGTGCAGGA |
| *M. bovis ssp* BCG |
| -----------------------------------------------------------------GCTGATGACCAGTGTGTTGATTGTGGAGGA |
| *M*. *tuberculosis* 5 |
| ----------------------------------------------------AGTGGCGCTGATGACCAGTGTGTTGATTGTGGAGGA |
| *M. tuberculosis* 7 |
| ---------------------------------------------------AGTGGCGCTGATGACCAGTGTGTTGATTGTGGAGGA |
| *M. bovis* |
| -------------------------------------------------------------------------------------AGTGTGTTGATTGTGGAGGA |
| *M. canettii* |
| ---------------------------------------------------AGTGGCGCTGATGACCAGTGTGTTGATTGTGGAGGA |
| *M. tuberculosis* 6 |
| ----------------------------------------------------AGTGGCGCTGATGACCAGTGTGTTGATTGTGGAGGA |
| *M. tuberculosis* 1 |
| CGAGGAGTCGCTGGCCGATCCGCTGGCGTTTCTGCTGCGCAAGGAGGGCTTTGAGGCCAC |
| *M. tuberculosis* 2 |
| CGAGGAGTCGCTGGCCGATCCGCTGGCGTTTCTGCTGCGCAAGGAGGGCTTTGAGGCCAC |
| *M. tuberculosis* 3 |
| CGAGGAGTCGCTGGCCGATCCGCTGGCGTTTCTGCTGCGCAAGGAGGGCTTTGAGGCCAC |
| *M. africanum* |
| CGAGGAGTCGCTGGCCGATCCGCTGGCGTTTCTGCTGCGCAAGGAGGGCTTTGAGGCCAC |
| *M. tuberculosis* 4 |
| CGAGGAGTCGCTGGCCGATCCGCTGGCGTTTCTGCTGCGCAAGGAGGGCTTTGAGGCCAC |
| *M. bovis* ssp BCG |
| CGAGGAGTCGCTGGCCGATCCGCTGACGTTTCTGCTGCGCAAGGAGGGCTTTGAGGCCAC |
| *M. tuberculosis* 5 |
| CGAGGAGTCGCTGGCCGATCCGCTGGCGTTTCTGCTGCGCAAGGAGGGCTTTGAGGCCAC |
| *M. tuberculosis* 7 |
| CGAGGAGTCGCTGGCCGATCCGCTGGCGTTTCTGCTGCGCAAGGAGGGCTTTGAGGCCAC |
| *M. bovis* |
| CGAGGAGTCGCTGGCCGATCCGCTGACGTTTCTGCTGCGCAAGGAGGGCTTTGAGGCCAC |
| *M. canettii* |
| CGAGGAGTCGCTGGCCGATCCGCTGGCGTTTCTGCTGCGCAAGGAGGGCTTTGAGGCCAC |
| *M. tuberculosis* 6 |
| CGAGGAGTCGCTGGCCGATCCGCTGGCGTTTCTGCTGCGCAAGGAGGGCTTTGAGGCCAC |
| *M. tuberculosis* 1 |
| GGTGGTGACCGATGGTCCGGCAGCTCTCGCCGAGTTCGACCGGGCCGGCGCCGACATCGT |
| *M. tuberculosis* 2 |
| GGTGGTGACCGATGGTCCGGCAGCTCTCGCCGAGTTCGACCGGGCCGGCGCCGACATCGT |
| *M. tuberculosis* 3 |
| GGTGGTGACCGATGGTCCGGCAGCTCTCGCCGAGTTCGACCGGGCCGGCGCCGACATCGT |
| *M. africanum* |
| GGTGGTGACCGATGGTCCGGCrIGCTCTCGCCGAGTTCGACCGGGCCGGCGCCGACATCGT |
| *M. tuberculosis* 4 |
| GGTGGTGACCGATGGTCCGGCAGCTCTCGCCGAGTTCGACCGGGCCGGCGCCGACATCGT |
| *M. bovis* ssp BCG |
| GGTGGTGACCGATGGTCCGGCAGCTCTCGCCGAGTTCGACCGGGCCGGCGCCGACATCGT |
| *M. tuberculosis* 5 |
| GGTGGTGACCGATGGTCCGGCAGCTCTCGCCGAGTTCGACCGGGCCGGCGCCGACATCGT |
| *M. tuberculosis* 7 |
| GGTGGTGACCGATGGTCCGGCAGCTCTCGCCGAGTTCGACCGGGCCGGCGCCGACATCGT |
| *M. bovis* |
| GGTGGTGACCGATGGTCCGGCAGCTCTCGCCGAGTTCGACCGGGCCGGCGCCGACATCGT |
| *M. canettii* |
| GGTGGTGACCGATGGTCCGGCAGCTCTCGCCGAGTTCGACCGGGCCGGCGCCGACATCGT |
| *M. tuberculosis 6* |
| GGTGGTGACCGATGGTCCGGCAGCTCTCGCCGAGTTCGACCGGGCCGGCGCCGACATCGT |
| *M. tubererculoris* 1 |
| CCTGCTCGATCTGATGCTGCCTGGGATGTCGGGTACCGATGTATGCAAGCAGTTGCGCGC |
| *M. tuberculosis* 2 |
| CCTGCTCGATCTGATGCTGCCTGGGATGTCGGGTACCGATGTATGCAAGCAGTTGCGCGC |
| *M. tuberculosis* 3 |
| CCTGCTCGATCTGATGCTGCCTGGGATGTCGGGTACCGATGTATGCAAGCAGTTGCGCGC |
| *M. africanum* |
| CCTGCTCGATCTGATGCTGCCTGGGATGTCGGGTACCGATGTATGCAAGCAGTTGCGCGC |
| *M. tuberculosis* 4 |
| CCTGCTCGATCTGATGCTGCCTGGGATGTCGGGTACCGATGTATGCAAGCAGTTGCGCGC |
| *M. bovis ssp* BCG |
| CCTGCTCGATCTGATGCTGCCTGGGATGTCGGGTACCGATGTATGCAAGCAGTTGCGCGC |
| *M. tuberculosis* 5 |
| CCTGCTCGATCTGATGCTGCCTGGGATGTCGGGTACCGATGTATGCAAGCAGTTGCGCGC |
| *M. tuberculosis* 7 |
| CCTGCTCGATCTGATGCTGCCTGGGATGTCGGGTACCGATGTATGCAAGCAGTTGCGCGC |
| *M. bovis* |
| CCTGCTCGATCTGATGCTGCCTGGGATGTCGGGTACCGATGTATGCAAGCAGTTGCGCGC |
| *M. canettii* |
| CCTGCTCGATCTGATGCTGCCTGGGATGTCGGGTACCGATGTATGCAAGCAGTTGCGCGC |
| *M.* tuberculosis 6 |
| CCTGCTCGATCTGATGCTGCCTGGGATGTCGGGTACCGATGTATGCAAGCAGTTGCGCGC |
| *M. tuberculosis* 1 |
| TCGGTCCAGCGTTCCGGTGATCATGGTGACCGCCCGGGATAGCGAGATCGACAAGGTGGT |
| *M. tuberculosis* 2 |
| TCGGTCCAGCGTTCCGGTGATCATGGTGACCGCCCGGGATAGCGAGATCGACAAGGTGGT |
| *M. tuberculosis* 3 |
| TCGGTCCAGCGTTCCGGTGATCATGGTGACCGCCCGGGATAGCGAGATCGACAAGGTGGT |
| *M. africanum* |
| TCGGTCCAGCGTTCCGCGTGATCATGGTGACCGCCCGGGATAGCGAGATCGACAAGGTGGT |
| *M. tuberculosis* 4 |
| TCGGTCCAGCGTTCCGGTGATCATGGTGACCGCCCGGGATAGCGAGATCGACAAGGTGGT |
| *M. bovis* ssp BCG |
| TCGGTCCAGCGTTCCGGTGATCATGGTGACCGCCCGGGATAGCGAGATCGACAAGGTGGT |
| *M. tuberculosis* 5 |
| TCGGTCCAGCGTTCCGGTGATCATGGTGACCGCCCGGGATAGCGAGATCGACAAGGTGGT |
| *M. tuberculosis 7* |
| TCGGTCCAGCGTTCCGGTGATCATGGTGACCGCCCGGGATAGCGAGATCGACAAGGTGGT |
| *M. bovis* |
| TCGGTCCAGCGTTCCGGTGATCATGGTGACCGCCCGGGATAGCGAGATCGACAAGGTGGT |
| *M. canettii* |
| TCGGTCCAGCGTTCCGGTGATCATGGTGACCGCCCGGGATAGCGAGATCGACAAGGTGGT |
| *M. tuberculosis* 6 |
| TCGGTCCAGCGTTCCGGTGATCATGGTGACCGCCCGGGATAGCGAGATCGACAAGGTGGT |
| *M. tuberculosis* 1 |
| CGGCCTGGAGCTGGGCGCTGACGACTACGTGACCAAGCCCTATTC |
| *M. tuberculosis* 2 |
| CGGCCTGGAGCTGGGCGCTGACGACTACGTGACCAAGCCCTATTC |
| *M. tuberculosis* 3 |
| CGGCCTGGAGCTGGGCGCTGACGACTACGTGACCAAGCCCTATTC |
| *M. africanum* |
| CGGCCTGGAGCTGGGCGCTGACGACTACGTGACCAAGCCCTATTC |
| *M. tuberculois* 4 |
| CGGCCTGGAGCTGGGCGCTGACGACTACGTGACCAAGCCCTATTC |
| *M. bovis* ssp BCG |
| CGGCCTGGAGCTGGGCGCTGACCGACTACGTGACCAAGCCCTATTC |
| *M. tuberculosis* 5 |
| CGGCCTGGAGCTGGGCGCTGACGACTACGTGACCAAGCCCTATTC |
| *M. tuberculosis* 7 |
| CGGCCTGGAGCTGGGCGCTGACGACTACGTGACCAAGCCCTATTC |
| *M. bovis* |
| CGGCCTGGAGCTGGGCGCTGACGACTACGTGACCAAGCCCTATTC |
| *M. canettii* |
| CGGCCTGGAGCTGGGCGCTGACGACTACGTGACCAAGCCCTATTC |
| *M*. *tuberculosis* 6 |
| CGGCCTGGAGCTGGGCGCTGACGACTACGTGACCAAGCCCTATTC |

## Revendications

1. Procédé d'identification de l'espèce d'une bactérie du complexe *Mycobacterium tuberculosis* dans un échantillon biologique contenant des acides nucléiques d'une dite bactérie dans lequel on effectue les étapes suivantes dans lesquelles:
1) on réalise l'amplification enzymatique de type PCR dans l'acide nucléique dudit échantillon, d'au moins 1 fragment d'acide nucléique d'une zone intergénique choisie parmi l'une des 2 zones intergéniques MST11 ou MST12 du génome de la dite bactérie à l'aide d'un couple d'amorce apte à amplifier ledit fragment, ledit couple d'amorces étant constitué par des oligonucléotides de séquences comprenant au moins 12 nucléotides consécutifs, de préférence au moins 18, inclus de préférence encore consistant dans les séquences SEQ. ID. n°21 à 24 du listage de séquences et leurs séquences complémentaires, avec les couples d'amorces 5', 3', tirés des séquences suivantes pour les fragments suivants :
- pour le fragment de la zone intergénique MST11 :
· amorce 5'= SEQ. ID. n°21
· amorce 3'= SEQ. ID. n°22 ;
- pour le fragment de la zone intergénique MST12 :
· amorce 5'= SEQ. ID. n°23
· amorce 3'= SEQ. ID. n°24
2) on réalise le séquençage du fragment amplifié à l'étape 1), et
3) on identifie l'espèce, la sous espèce ou le sous type de ladite bactérie, en comparant la séquence obtenue à l'étape 2) avec les séquences d'une banque de données des séquences d'au moins ledit fragment d'acide nucléique d'une dite zone intergénique choisie parmi les 2 dites zones intergéniques MST11 et MST12, respectivement des différentes espèces, sous-espèces et sous-types de bactéries du complexe *Mycobacterium tuberculosis.*

2. Procédé selon la revendication 1, **caractérisé en ce que** à l'étape 3), lesdites séquences constituant ladite banque ont été déterminées par amplification et séquençage de dit(s) fragment(s) de zone(s) intergénique(s) des différentes bactéries d'une collection de différents isolats de bactéries de différentes espèces, sous espèces et sous types de bactéries du complexe *Mycobacterium tuberculosis* dont les espèces, sous espèces et sous types et, le cas échéant, génotypes, sont connus.

3. Procédé selon la revendication 1 ou 2, **caractérisé en ce qu'**on identifie une espèce, sous espèce ou sous type de bactérie parmi les bactéries *Mycobacterium tuberculosis, Mycobacterium bovis ssp bovis, Mycobacterium bovis ssp BCG, Mycobacterium bovis ssp caprae, Mycobacterium africanum sous type I, Mycobacterium canettii, Mycobacterium microti.*

4. Procédé selon la revendication 3, **caractérisé en ce qu'**on identifie une espèce sous espèce ou sous type de bactérie parmi les bactéries *Mycobacterium tuberculosis, Mycobacterium bovis ssp bovis, Mycobacterium bovis ssp BCG, Mycobacterium africanum sous type I, Mycobacterium canettii.*

5. Procédé selon la revendication 4, **caractérisé en ce que**, à l'étape 3), lesdites séquences de ladite banque comprennent les séquences SEQ. ID. n°33 à 51 suivantes du listage de séquences et leurs séquences complémentaires pour les espèces suivantes :
- pour *Mycobacterium tuberculosis* :
· les séquences du fragment de la zone intergénique MST11 consistant dans l'une des séquences SEQ. ID. n°33 à 36,
· les séquences du fragment de la zone intergénique MST12 consistant dans l'une des séquences SEQ. ID. n°41 à 47,
- pour *Mycobacterium africanum* :
· la séquence du fragment de la zone intergénique MST11 consistant dans SEQ. ID. n°37,
· la séquence du fragment de la zone intergénique MST12 consistant dans SEQ. ID. n°48,
- pour *Mycobacterium bovis ssp bovis* :
· la séquence du fragment de la zone intergénique MST11 consistant dans SEQ. ID. n°38,
· la séquence du fragment de la zone intergénique MST12 consistant dans SEQ. ID. n°49,
- pour *Mycobacterium bovis ssp BCG* :
· la séquence du fragment de la zone intergénique MST11 consistant dans SEQ. ID. n°39,
· la séquence du fragment de la zone intergénique MST12 consistant dans SEQ. ID. n°50,
- pour *Mycobacterium canettii* :
· la séquence du fragment de la zone intergénique MST11 consistant dans SEQ. ID. n°40,
· la séquence du fragment de la zone intergénique MST12 consistant dans SEQ. ID. n°51,

6. Procédé selon l'une des revendications 1 à 5, dans lequel on identifie le génotype d'une espèce, sous espèce ou sous type donné d'une bactérie parmi les bactéries du complexe *Mycobacterium tuberculosis* **caractérisé en ce que** on réalise les étapes dans lesquelles :
a) à l'étape 1) on réalise l'amplification enzymatique de type PCR dans l'acide nucléique dudit échantillon, d'au moins 8 fragments d'acides nucléiques d'au moins 8 zones intergéniques choisies parmi 16 dites zones intergéniques MST1 à MST16 dont les 2 zones intergéniques MST11 et MST12, à l'aide respectivement des au moins 8 dits couples d'amorces aptes à amplifier les dits fragments des au moins 8 dites zones intergéniques , lesdites amorces étant constituées par des oligonucléotides de séquences comprenant 12 à 35 nucléotides consécutifs, de préférence 18 à 30, inclus ou de préférence encore consistant dans les séquences suivantes du listage de séquences et leurs séquences complémentaires, avec les couples d'amorces 5', 3', tirés des séquences suivantes pour les fragments suivants :
- pour le fragment de la zone intergénique MST1 :
· amorce 5'= SEQ. ID. n°1
· amorce 3'= SEQ. ID. n°2
- pour le fragment de la zone intergénique MST2 :
· amorce 5'= SEQ. ID. n°3
· amorce 3'= SEQ. ID. n°4
- pour le fragment de la zone intergénique MST3 :
· amorce 5'= SEQ. ID. n°5
· amorce 3'= SEQ. ID. n°6
- pour le fragment de la zone intergénique MST4 :
· amorce 5'= SEQ. ID. n°7
· amorce 3'= SEQ. ID. n°8
- pour le fragment de la zone intergénique MST5 :
· amorce 5'= SEQ. ID. n°9
· amorce 3'= SEQ. ID. n°10
- pour le fragment de la zone intergénique MST6 :
· amorce 5'= SEQ. ID. n°11
· amorce 3'= SEQ. ID. n°12
- pour le fragment de la zone intergénique MST7 :
amorce 5'= SEQ. ID. n°13
· amorce 3'= SEQ. ID. n°14
- pour le fragment de la zone intergénique MST8 :
· amorce 5'= SEQ. ID. n°15
· amorce 3'= SEQ. ID. n°16
- pour le fragment de la zone intergénique MST9 :
· amorce 5'= SEQ. ID. n°17
· amorce 3'= SEQ. ID. n°18
- pour le fragment de la zone intergénique MST10 :
· amorce 5'= SEQ. ID. n°19
· amorce 3'= SEQ. ID. n°20
- pour le fragment de la zone intergénique MST11 :
· amorce 5'= SEQ. ID. n°21
· amorce 3'= SEQ. ID. n°22
- pour le fragment de la zone intergénique MST12 :
· amorce 5'= SEQ. ID. n°23
· amorce 3'= SEQ. ID. n°24
- pour le fragment de la zone intergénique MST13 :
· amorce 5'= SEQ. ID. n°25
· amorce 3'= SEQ. ID. n°26
- pour le fragment de la zone intergénique MST14 :
· amorce 5'= SEQ. ID. n°27
· amorce 3'= SEQ. ID. n°28
- pour le fragment de la zone intergénique MST15 :
· amorce 5'= SEQ. ID. n°29
· amorce 3'= SEQ. ID, n°30
- pour le fragment de la zone intergénique MST16 :
· amorce 5'= SEQ. ID. n°31
· amorce 3'= SEQ. ID. n°32
, et
b) à l'étape 2) on réalise le séquençage des fragments amplifiés à l'étape a), et
c) à l'étape 3) on identifie en outre le génotype de l'espèce, sous espèce ou sous type de dite bactérie en comparant les séquences des fragments amplifiés obtenues à l'étape b) avec les séquences d'une banque de données des séquences des au moins 8 dits fragments d'acides nucléiques des au moins 8 dites zones intergéniques choisie parmi 16 dites zones intergéniques des différents génotypes de dites bactéries du complexe *Mycobacterium tuberculosis,* les différents génotypes correspondant aux différentes combinaisons possibles des au moins 8 séquences des au moins 8 dits fragments d'acide nucléique des au moins 8 dites zones intergéniques, les séquences constituant ladite banque ayant été déterminées par amplification et séquençage desdits au moins 8 fragments des au moins 8 dites zones intergéniques des différentes bactéries d'une collection de différents isolats de bactéries de ladite espèce, sous espèce ou sous type connues du complexe Mycobacterium tuberculosis, les différents génotypes correspondant à des combinaisons différentes des au moins 8 séquences desdites au moins 8 zones intergéniques des bactéries de ladite collection.

7. Procédé selon la revendication 6, **caractérisé en ce que** lesdits fragments des au moins 8 zones intergéniques sont les 8 fragments desdites zones intergéniques MST1, MST2, MST3, MST4, MST8, MST11, MST12 et MST13, amplifiables par leurs dits couples d'amorces respectives de séquences tirées des séquences SEQ. ID. n°1 à 16 et 21 à 26, avec:
- pour le fragment de la zone intergénique MST1 :
· amorce 5'= SEQ. ID. n°1
· amorce 3'= SEQ. ID. n°2
- pour le fragment de la zone intergénique MST2 :
· amorce 5'= SEQ. ID. n°3.
· amorce 3'= SEQ. ID. n°4
- pour le fragment de la zone intergénique MST3 :
· amorce 5'= SEQ. ID. n°5
· amorce 3'= SEQ. ID. n°6
- pour le fragment de la zone intergénique MST4 :
· amorce 5'= SEQ. ID. n°7
· amorce 3'= SEQ. ID. n°8
- pour le fragment de la zone intergénique MST8 :
· amorce 5'= SEQ. ID. n°15
· amorce 3'= SEQ. ID. n°16
- pour le fragment de la zone intergénique MST11 :
· amorce 5'= SEQ. ID. n°21
· amorce 3'= SEQ. ID. n°22
- pour le fragment de la zone intergénique MST12 :
· amorce 5'= SEQ. ID. n°23
· amorce 3'= SEQ. ID. n°24
- pour le fragment de la zone intergénique MST13 :
· amorce 5'= SEQ. ID. n°25
· amorce 3'= SEQ. ID. n°26

8. Procédé selon l'une des revendications 6 ou 7, **caractérisé en ce que** lesdites séquences de ladite banque comprennent les séquences choisies parmi SEQ. ID. n°33 à 79 du listage de séquences et leurs séquences complémentaires pour lesdits fragments de zones intergéniques MST1 à MST16 suivants :
- pour le fragment de la zone intergénique MST1, les 4 séquences SEQ. ID. n°52 à 55
- pour le fragment de la zone intergénique MST2, les 3 séquences SEQ. ID. n°56 à 58
- pour le fragment de la zone intergénique MST3, les 2 séquences SEQ. ID. n°59 à 60
- pour le fragment de la zone intergénique MST4, les 5 séquences SEQ. ID. n°61 à 65
- pour le fragment de la zone intergénique MST8, les 3 séquences SEQ. ID. n°66 à 68
- pour le fragment de la zone intergénique MST11, les 4 séquences SEQ. ID. n°33 à 36
- pour le fragment de la zone intergénique MST12, les 7 séquences SEQ. ID. n°41 à 47
- pour le fragment de la zone intergénique MST13, les 11 séquences SEQ. ID. n°69 à 79.

9. Procédé selon l'une des revendications 1 à 8, **caractérisé en ce que** l'on vérifie au préalable que l'échantillon sur lequel on réalise ladite identification, contient effectivement des acides nucléiques d'une dite bactérie du complexe *Mycobacterium tuberculosis.*

10. Oligonucléotide isolé apte à servir d'amorce d'amplification d'un fragment d'acide nucléique de dite zone intergénique choisi parmi les fragments d'acide nucléique des 8 zones intergéniques MST1, MST2, MST3, MST4, MST8, MST11, MST12 et MST13,, utile dans un procédé selon l'une des revendication 1 à 9, **caractérisé en ce qu'**il est constitué d'une séquence consistant dans l'une des séquences SEQ. ID. n°1 à 8, 15-16 et 21 à 26 du listage de séquences et leurs séquences complémentaires, correspondant aux couples d'amorces 5', 3' suivants :
- pour le fragment de la zone intergénique MST1 :
· amorce 5'= SEQ. ID. n°1
· amorce 3'= SEQ. ID. n°2
- pour le fragment de la zone intergénique MST2 :
· amorce 5'= SEQ. ID. n°3
· amorce 3'= SEQ. ID. n°4
- pour le fragment de la zone intergénique MST3 :
· amorce 5'= SEQ. ID. n°5
· amorce 3'= SEQ. ID. n°6
- pour le fragment de la zone intergénique MST4 :
· amorce 5'= SEQ. ID. n°7
· amorce 3'= SEQ. ID. n°8
- pour le fragment de la zone intergénique MST8 :
· amorce 5'= SEQ. ID. n° 15
· amorce 3'= SEQ. ID. n°16
- pour le fragment de la zone intergénique MST11 :
· amorce 5'= SEQ. ID. n°2
· amorce 3'= SEQ. ID. n°22
- pour le fragment de la zone intergénique MST12 :
· amorce 5'= SEQ. ID. n°23
· amorce 3'= SEQ. ID. n°24
- pour le fragment de la zone intergénique MST13 :
· amorce 5'= SEQ. ID. n°25
· amorce 3'= SEQ. ID. n°26

11. Fragment isolé d'acide nucléique de zone intergénique non codante d'une bactérie du complexe *Mycobacterium tuberculosis* utile pour constituer une banque dans un procédé selon l'une des revendications 1 à 9, choisi parmi les fragments d'acides nucléiques des 8 zones intergéniques MST1, MST2, MST3, MST4, MST8, MST11, MST12 et MST13, **caractérisé en ce que** sa séquence est choisie parmi les séquences SEQ. ID. n°33 à 79 du listage de séquences et leurs séquences complémentaires, avec les séquences suivantes pour les espèces suivantes :
- pour l'espèce *Mycobacterium tuberculosis* :
· les séquences du fragment de la zone intergénique MST1, consistant dans les 4 séquences SEQ. ID. n°52 à 55,
· les séquences du fragment de la zone intergénique MST2, consistant dans les 3 séquences SEQ. ID. n°56 à 58,
· les séquences du fragment de la zone intergénique MST3, consistant dans les 2 séquences SEQ. ID. n°59 à 60,
· les séquences du fragment de la zone intergénique MST4, consistant dans les 5 séquences SEQ. ID. n°61 à 65,
· les séquences du fragment de la zone intergénique MST8, consistant dans les 3 séquences SEQ. ID. n°66 à 68,
· les séquences du fragment de la zone intergénique MST11, consistant dans les 4 séquences SEQ. ID. n°33 à 36,
· les séquences du fragment de la zone intergénique MST12, consistant dans les 7 séquences SEQ. ID. n°41 à 47,
· les séquences du fragment de la zone intergénique MST13, consistant dans les 11 séquences SEQ. ID. n°69 à 79,
- pour *Mycobacterium africanum* :
· la séquence du fragment de la zone intergénique MST11 consistant dans SEQ. ID. n°37, et
· la séquence du fragment de la zone intergénique MST12 consistant dans SEQ. ID. n°48,
- pour *Mycobacterium bovis ssp bovis* :
· la séquence du fragment de la zone intergénique MST11 consistant dans SEQ. ID. n°38, et
· la séquence du fragment de la zone intergénique MST12 consistant dans SEQ. ID. n°49,
- pour *Mycobacterium bovis ssp BCG* :
· la séquence du fragment de la zone intergénique MST11 consistant dans SEQ. ID. n°39, et
· la séquence du fragment de la zone intergénique MST12 consistant dans SEQ. ID. n°50,
- pour *Mycobacterium canettii* :
· la séquence du fragment de la zone intergénique MST11 consistant dans SEQ. ID. n°40, et
· la séquence du fragment de la zone intergénique MST12 consistant dans SEQ. ID. n°51.

12. Fragment isolé d'acide nucléique de zone intergénique non codante d'une bactérie du complexe *Mycobacterium tuberculosis* selon la revendication 11, choisi parmi les fragments d'acides nucléiques des 2 dites zones intergéniques MST11 et MST12, **caractérisé en ce que** sa séquence est choisie parmi les séquences SEQ. ID. n°33 à 51 du listage de séquences et leurs séquences complémentaires, avec les séquences suivantes pour les espèces suivantes :
- pour l'espèce *Mycobacterium tuberculosis* :
· les séquences du fragment de la zone intergénique MST11, consistant dans les 4 séquences SEQ. ID. n°33 à 36,
· les séquences du fragment de la zone intergénique MST12, consistant dans les 7 séquences SEQ. ID. n°41 à 47,
- pour *Mycobacterium africanum* :
· la séquence du fragment de la zone intergénique MST11 consistant dans SEQ. ID. n°37, et
· la séquence du fragment de la zone intergénique MST12 consistant dans SEQ. ID. n°48,
- pour *Mycobacterium bovis ssp bovis* :
• la séquence du fragment de la zone intergénique MST11 consistant dans SEQ. ID. n°38, et
• la séquence du fragment de la zone intergénique MST12 consistant dans SEQ. ID. n°49,
- pour *Mycobacterium bovis ssp BCG* :
• la séquence du fragment de la zone intergénique MST11 consistant dans SEQ. ID. n°39, et
• la séquence du fragment de la zone intergénique MST12 consistant dans SEQ. ID. n°50,
- pour *Mycobacterium canettii* :
• la séquence du fragment de la zone intergénique MST11 consistant dans SEQ. ID. n°40, et
• la séquence du fragment de la zone intergénique MST12 consistant dans SEQ. ID. n°51.

## Claims

1. A method for identification of the species of a bacterium of the Mycobacterium tuberculosis complex in a biological sample containing nucleic acids of a said bacterium, in which the following steps are carried out, in which steps:
1) the PCR-type enzymatic amplification of at least one nucleic acid fragment of an intergenic region chosen from one of the two intergenic regions MST11 or MST12 of the genome of said bacterium is carried out, in the nucleic acid of said sample, using a pair of primers capable of amplifying said fragment, said pair of primers being constituted of oligonucleotides having sequences comprising at least 12 consecutive nucleotides, preferably at least 18, included, more preferably consisting, in the sequences SEQ. ID. Nos. 21 to 24 of the sequence listing and the sequences complementary thereto, with the pairs of 5' and 3' primers, drawn from the following sequences for the following fragments:
- for the fragment of the intergenic region MST11:
• 5' primer = SEQ. ID. No. 21
• 3' primer = SEQ. ID. No. 22;
- for the fragment of the intergenic region MST12:
• 5' primer = SEQ. ID. No. 23
• 3' primer = SEQ. ID. No. 24
2) the sequencing of the fragment amplified in step 1) is carried out, and
3) the species, the subspecies or the subtype of said bacterium is identified by comparing the sequence obtained in step 2) with the sequences of a data bank of the sequences of at least said nucleic acid fragment of a said intergenic region chosen from the two said intergenic regions MST11 and MST12, respectively, of the various species, subspecies and subtypes of bacteria of the Mycobacterium tuberculosis complex.

2. The method according to claim 1, **characterized in that**, in step 3), said sequences making up said bank have been determined by amplification and sequencing said fragment(s) of one or more intergenic region(s) of the various bacteria of a collection of various isolates of bacteria of various species, subspecies and subtypes of bacteria of the Mycobacterium tuberculosis complex, the species, subspecies and subtypes and, where appropriate, genotypes of which are known.

3. The method according to claim 1 or claim 2, **characterized in that** a species, subspecies or subtype of a bacterium among the bacteria Mycobacterium tuberculosis, Mycobacterium bovis ssp bovis, Mycobacterium bovis ssp BCG, Mycobacterium bovis ssp caprae, Mycobacterium africanum subtype I, Mycobacterium canettii and Mycobacterium microti is identified.

4. The method according to claim 3, **characterized in that** a species, subspecies or subtype of a bacterium among the bacteria Mycobacterium tuberculosis, Mycobacterium bovis ssp bovis, Mycobacterium bovis ssp BCG, Mycobacterium africanum subtype I and Mycobacterium canettii is identified.

5. The method according to claim 4, **characterized in that**, in step 3), said sequences of said bank comprise the following sequences SEQ. ID. Nos. 33 to 51 of the sequence listing and the sequences complementary thereto, for the following species:
- for Mycobacterium tuberculosis:
• the sequences of the fragment of the intergenic region MST11, consisting in one of the sequences SEQ. ID. Nos. 33 to 36,
• the sequences of the fragment of the intergenic region MST12, consisting in one of the sequences SEQ. ID. Nos. 41 to 47,
- for Mycobacterium africanum:
• the sequence of the fragment of the intergenic region MST11, consisting in SEQ. ID. No. 37,
• the sequence of the fragment of the intergenic region MST12, consisting in SEQ. ID. No. 48,
- for Mycobacterium bovis ssp bovis:
• the sequence of the fragment of the intergenic region MST11, consisting in SEQ. ID. No. 38,
• the sequence of the fragment of the intergenic region MST12, consisting in SEQ. ID. No. 49,
- for Mycobacterium bovis ssp BCG:
• the sequence of the fragment of the intergenic region MST11, consisting in SEQ. ID. No. 39,
• the sequence of the fragment of the intergenic region MST12, consisting in SEQ. ID. No. 50,
- for Mycobacterium canettii:
• the sequence of the fragment of the intergenic region MST11, consisting in SEQ. ID. No. 40,
• the sequence of the fragment of the intergenic region MST12, consisting in SEQ. ID. No. 51.

6. The method according to one of claims 1 to 5, in which the genotype of a given species, subspecies or subtype of a bacterium among the bacteria of the Mycobacterium tuberculosis complex is identified, the method being **characterized in that** the following steps are carried out, in which:
a) in step 1) the PCR-type enzymatic amplification of at least eight nucleic acid fragments of at least eight intergenic regions chosen from 16 said intergenic regions MST1 to MST16, including the two intergenic regions MST11 and MST12, is carried out, in the nucleic acid of said sample, using, respectively, the at least eight said pairs of primers capable of amplifying the said fragments of the at least eight said intergenic regions, said primers being constituted of oligonucleotides having sequences comprising 12 to 35 consecutive nucleotides, preferably 18 to 30, included or more preferably consisting in the following sequences of the sequence listing and the sequences complementary thereto, with the pairs of 5' and 3' primers, drawn from the following sequences for the following fragments:
- for the fragment of the intergenic region MST1:
• 5' primer = SEQ. ID. No. 1
• 3' primer = SEQ. ID. No. 2,
- for the fragment of the intergenic region MST2:
• 5' primer = SEQ. ID. No. 3
• 3' primer = SEQ. ID. No. 4,
- for the fragment of the intergenic region MST3:
• 5' primer = SEQ. ID. No. 5
• 3' primer = SEQ. ID. No. 6,
- for the fragment of the intergenic region MST4:
• 5' primer = SEQ. ID. No. 7
• 3' primer = SEQ. ID. No. 8,
- for the fragment of the intergenic region MST5:
• 5' primer = SEQ. ID. No. 9
• 3' primer = SEQ. ID. No. 10,
- for the fragment of the intergenic region MST6:
• 5' primer = SEQ. ID. No. 11
• 3' primer = SEQ. ID. No. 12,
- for the fragment of the intergenic region MST7:
• 5' primer = SEQ. ID. No. 13
• 3' primer = SEQ. ID. No. 14,
- for the fragment of the intergenic region MST8:
• 5' primer = SEQ. ID. No. 15
• 3' primer = SEQ. ID. No. 16,
- for the fragment of the intergenic region MST9:
• 5' primer = SEQ. ID. No. 17
• 3' primer = SEQ. ID. No. 18,
- for the fragment of the intergenic region MST10:
• 5' primer = SEQ. ID. No. 19
• 3' primer = SEQ. ID. No. 20,
- for the fragment of the intergenic region MST11:
• 5' primer = SEQ. ID. No. 21
• 3' primer = SEQ. ID. No. 22,
- for the fragment of the intergenic region MST12:
• 5' primer = SEQ. ID. No. 23
• 3' primer = SEQ. ID. No. 24,
- for the fragment of the intergenic region MST13:
• 5' primer = SEQ. ID. No. 25
• 3' primer = SEQ. ID. No. 26,
- for the fragment of the intergenic region MST14:
• 5' primer = SEQ. ID. No. 27
• 3' primer = SEQ. ID. No. 28,
- for the fragment of the intergenic region MST15:
• 5' primer = SEQ. ID. No. 29
• 3' primer = SEQ. ID. No. 30,
- for the fragment of the intergenic region MST16:
• 5' primer = SEQ. ID. No. 31
• 3' primer = SEQ. ID. No. 32,
and
b) in step 2), the sequencing of the fragments amplified in step a) is carried out, and
c) in step 3), the genotype of the species, subspecies or subtype of said bacterium is, in addition, identified by comparing the sequences of the amplified fragments obtained in step b) with the sequences of a data bank of the sequences of the at least eight said nucleic acid fragments of the at least eight said intergenic regions chosen from 16 said intergenic regions of the various genotypes of said bacteria of the Mycobacterium tuberculosis complex, the various genotypes corresponding to the various possible combinations of the at least eight sequences of the at least eight said nucleic acid fragments of the at least eight said intergenic regions, the sequences making up said bank having been determined by amplification and sequencing of said at least eight fragments of the at least eight said intergenic regions of the various bacteria of a collection of various isolates of bacteria of said species, subspecies or subtype, that are known, of the Mycobacterium tuberculosis complex, the various genotypes corresponding to different combinations of the at least eight sequences of said at least eight intergenic regions of the bacteria of said collection.

7. The method according to claim 6, **characterized in that** said fragments of the at least eight intergenic regions are the eight fragments of said intergenic regions MST1, MST2, MST3, MST4, MST8, MST11, MST12 and MST13, that can be amplified by their said pairs of respective primers having sequences taken from the sequences SEQ. ID. Nos. 1 to 16 and 21 to 26, with:
- for the fragment of the intergenic region MST1:
• 5' primer = SEQ. ID. No. 1
• 3' primer = SEQ. ID. No. 2,
- for the fragment of the intergenic region MST2:
• 5' primer = SEQ. ID. No. 3
• 3' primer = SEQ. ID. No. 4,
- for the fragment of the intergenic region MST3:
• 5' primer = SEQ. ID. No. 5
• 3' primer = SEQ. ID. No. 6,
- for the fragment of the intergenic region MST4:
• 5' primer = SEQ. ID. No. 7
• 3' primer = SEQ. ID. No. 8,
- for the fragment of the intergenic region MST8:
• 5' primer = SEQ. ID. No. 15
• 3' primer = SEQ. ID. No. 16,
- for the fragment of the intergenic region MST11:
• 5' primer = SEQ. ID. No. 21
• 3' primer = SEQ. ID. No. 22,
- for the fragment of the intergenic region MST12:
• 5' primer = SEQ. ID. No. 23
• 3' primer = SEQ. ID. No. 24,
- for the fragment of the intergenic region MST13:
• 5' primer = SEQ. ID. No. 25
• 3' primer = SEQ. ID. No. 26.

8. The method according to claim 6 or claim 7, **characterized in that** said sequences of said bank comprise the sequences chosen from among SEQ. ID. Nos. 33 to 79 of the sequence listing and the sequences complementary thereto, for said fragments of intergenic regions MST1 to MST16 below:
- for the fragment of the intergenic region MST1, the four sequences SEQ. ID. Nos. 52 to 55,
- for the fragment of the intergenic region MST2, the three sequences SEQ. ID. Nos. 56 to 58,
- for the fragment of the intergenic region MST3, the two sequences SEQ. ID. Nos. 59 to 60,
- for the fragment of the intergenic region MST4, the five sequences SEQ. ID. Nos. 61 to 65,
- for the fragment of the intergenic region MST8, the three sequences SEQ. ID. Nos. 66 to 68,
- for the fragment of the intergenic region MST11, the four sequences SEQ. ID. Nos. 33 to 36,
- for the fragment of the intergenic region MST12, the seven sequences SEQ. ID. Nos. 41 to 47,
- for the fragment of the intergenic region MST13, the 11 sequences SEQ. ID. Nos. 69 to 79.

9. The method according to one of claims 1 to 8, **characterized in that** it is verified beforehand that the sample on which said identification is carried out actually contains nucleic acids of a said bacterium of the Mycobacterium tuberculosis complex.

10. An isolated oligonucleotide capable of serving as a primer for the amplification of a nucleic acid fragment of said intergenic region, chosen from the nucleic acid fragments of the 8 intergenic regions MST1, MST2, MST3, MST4, MST8, MST11, MST12 and MST13, that is of use in a method according to one of claims 1 to 9, the oligonucleotide being **characterized in that** it has a sequence consisting in one of the sequences SEQ. ID. Nos. 1 to 8, 15-16 and 21 to 26 of the sequence listing and the sequences complementary thereto, corresponding to the following pairs of 5' and 3' primers:
- for the fragment of the intergenic region MST1:
• 5' primer = SEQ. ID. No. 1
• 3' primer = SEQ. ID. No. 2,
- for the fragment of the intergenic region MST2:
• 5' primer = SEQ. ID. No. 3
• 3' primer = SEQ. ID. No. 4,
- for the fragment of the intergenic region MST3:
• 5' primer = SEQ. ID. No. 5
• 3' primer = SEQ. ID. No. 6,
- for the fragment of the intergenic region MST4:
• 5' primer = SEQ. ID. No. 7
• 3' primer = SEQ. ID. No. 8,
- for the fragment of the intergenic region MST8:
• 5' primer = SEQ. ID. No. 15
• 3' primer = SEQ. ID. No. 16,
- for the fragment of the intergenic region MST11:
• 5' primer = SEQ. ID. No. 21
• 3' primer = SEQ. ID. No. 22,
- for the fragment of the intergenic region MST12:
• 5' primer = SEQ. ID. No. 23
• 3' primer = SEQ. ID. No. 24,
- for the fragment of the intergenic region MST13:
• 5' primer = SEQ. ID. No. 25
• 3' primer = SEQ. ID. No. 26,

11. An isolated nucleic acid fragment of a noncoding intergenic region of a bacterium of the Mycobacterium tuberculosis complex, that is of use for building up a bank in a method according to one of claims 1 to 9, chosen from the nucleic acid fragments of the 8 intergenic regions MST1, MST2, MST3, MST4, MST8, MST11, MST12 and MST13, the fragment being **characterized in that** it has a sequence chosen from among the sequences SEQ. ID Nos. 33 to 79 of the sequence listing and the sequences complementary thereto, with the following sequences for the following species:
- for the Mycobacterium tuberculosis species:
• the sequences of the fragment of the intergenic region MST1, consisting in the four sequences SEQ. ID. Nos. 52 to 55,
• the sequences of the fragment of the intergenic region MST2, consisting in the three sequences SEQ. ID. Nos. 56 to 58,
• the sequences of the fragment of the intergenic region MST3, consisting in the two sequences SEQ. ID. Nos. 59 to 60,
• the sequences of the fragment of the intergenic region MST4, consisting in the five sequences SEQ. ID. Nos. 61 to 65,
• the sequences of the fragment of the intergenic region MST8, consisting in the three sequences SEQ. ID. Nos. 66 to 68,
• the sequences of the fragment of the intergenic region MST11, consisting in the four sequences SEQ. ID. Nos. 33 to 36,
• the sequences of the fragment of the intergenic region MST12, consisting in the seven sequences SEQ. ID. Nos. 41 to 47,
• the sequences of the fragment of the intergenic region MST13, consisting in the eleven sequences SEQ. ID. Nos. 69 to 79,
- for Mycobacterium africanum:
• the sequence of the fragment of the intergenic region MST11, consisting in SEQ. ID. No. 37, and
• the sequence of the fragment of the intergenic region MST12, consisting in SEQ. ID. No. 48,
- for Mycobacterium bovis ssp bovis:
• the sequence of the fragment of the intergenic region MST11, consisting in SEQ. ID. No. 38, and
• the sequence of the fragment of the intergenic region MST12, consisting in SEQ. ID. No. 49,
- for Mycobacterium bovis ssp BCG:
• the sequence of the fragment of the intergenic region MST11, consisting in SEQ. ID. No. 39, and
• the sequence of the fragment of the intergenic region MST12, consisting in SEQ. ID. No. 50,
- for Mycobacterium canettii:
• the sequence of the fragment of the intergenic region MST11, consisting in SEQ. ID. No. 40, and
• the sequence of the fragment of the intergenic region MST12, consisting in SEQ. ID. No. 51.

12. An isolated nucleic acid fragment of a noncoding intergenic region of a bacterium of the Mycobacterium tuberculosis complex according to claim 11, chosen from the nucleic acid fragments of the 2 intergenic regions MST11 and MST12, the fragment being **characterized in that** it has a sequence chosen from among the sequences SEQ. ID Nos. 33 to 51 of the sequence listing and the sequences complementary thereto, with the following sequences for the following species:
- for the Mycobacterium tuberculosis species:
• the sequences of the fragment of the intergenic region MST11, consisting in the four sequences SEQ. ID. Nos. 33 to 36,
• the sequences of the fragment of the intergenic region MST12, consisting in the seven sequences SEQ. ID. Nos. 41 to 47,
- for Mycobacterium africanum:
• the sequence of the fragment of the intergenic region MST11, consisting in SEQ. ID. No. 37, and
• the sequence of the fragment of the intergenic region MST12, consisting in SEQ. ID. No. 48,
- for Mycobacterium bovis ssp bovis:
• the sequence of the fragment of the intergenic region MST11, consisting in SEQ. ID. No. 38, and
• the sequence of the fragment of the intergenic region MST12, consisting in SEQ. ID. No. 49,
- for Mycobacterium bovis ssp BCG:
• the sequence of the fragment of the intergenic region MST11, consisting in SEQ. ID. No. 39, and
• the sequence of the fragment of the intergenic region MST12, consisting in SEQ. ID. No. 50,
- for Mycobacterium canettii:
• the sequence of the fragment of the intergenic region MST11, consisting in SEQ. ID. No. 40, and
• the sequence of the fragment of the intergenic region MST12, consisting in SEQ. ID. No. 51.

## Patentansprüche

1. Verfahren zur Identifizierung einer Spezies eines Bakteriums des Komplexes *Mycobacterium tuberculosis* in einer biologischen Probe, die Nukleinsäuren eines solchen Bakteriums enthält, in welchem man die folgenden Schritte durchführt, in denen:
1) man die enzymatische Amplifikation vom PCR-Typ aus der Nukleinsäure der Probe von wenigstens einem Nukleinsäurefragment einer intergenischen Zone ausführt, die gewählt ist unter einer der beiden intergenischen Zonen MST11 oder MST12 des Genoms des genannten Bakteriums, mit Hilfe eines Primerpaars, das geeignet ist, dieses Fragment zu amplifizieren, wobei das Primerpaar gebildet ist aus Oligonukleotiden mit Sequenzen, die wenigstens 12, vorzugsweise wenigstens 18 aufeinanderfolgende Nukleotide umfassen und weiterhin vorzugsweise umfaßt sind und liegen in den Sequenzen SEQ. ID. Nr. 21 bis 24 des Sequenzprotokolls, und deren komplementären Sequenzen, mit den 5'-, 3'-Primerpaaren, welche aus den folgenden Sequenzen für die folgenden Fragmente gezogen sind:
- für das Fragment der intergenischen Zone MST11:
• 5'-Primer = SEQ. ID. Nr. 21
• 3'-Primer = SEQ. ID. Nr. 22
- für das Fragment der intergenischen Zone MST12:
• 5'-Primer = SEQ. ID. Nr. 23
• 3'-Primer = SEQ. ID. Nr. 24
2) man die Sequenzierung des in Schritt 1 amplifizierten Fragmentes ausführt und
3) man die Spezies, die Unterspezies oder den Untertyp des Bakteriums, welche/welcher die in Schritt 2) erhaltene Sequenz umfaßt, identifiziert mit den Sequenzen einer Sequenzdatenbank von wenigstens dem Nukleinsäurefragment einer intergenischen Zone, die gewählt ist aus den zwei intergenischen Zonen MST11 und MST12 der unterschiedlichen Spezies, bzw. Unterspezies bzw. Untertypen von Bakterien des Komplexes Mycobacterium tuberculosis.

2. Verfahren nach Anspruch 1, **dadurch gekennzeichnet, daß** bei Schritt 3) die Sequenzen, die die Datenbank bilden, bestimmt worden sind durch Amplifikation und Sequenzierung des Fragments einer intergenischen Zone/der Fragmente von intergenischen Zonen unterschiedlicher Bakterien einer Sammlung von unterschiedlichen Isolaten von Bakterien unterschiedlicher Spezies, Unterspezies und Untertypen von Bakterien des Komplexes *Mycobacterium tuberculosis,* von denen die Spezies, Unterspezies und Untertypen und andernfalls Genotypen bekannt sind.

3. Verfahren nach Anspruch 1 oder 2, **dadurch gekennzeichnet, daß** man eine Spezies, Unterspezies oder einen Untertyp eines Bakteriums unter den Bakterien *Mycobacterium tuberculosis, Mycobacterium bovis ssp bovis, Mycobacterium bovis ssp BCG, Mycobacterium bovis ssp caprae, Mycobacterium africanum Untertyp I, Mycobacterium canettii, Mycobacterium microti* identifiziert.

4. Verfahren nach Anspruch 3, **dadurch gekennzeichnet, daß** man eine Spezies, Unterspezies oder einen Untertyp eines Bakteriums unter den Bakterien *Mycobacterium tuberculosis, Mycobacterium bovis ssp bovis, Mycobacterium bovis ssp BCG, Mycobacterium africanum Untertyp 1, Mycobacterium canettii* identifiziert.

5. Verfahren nach Anspruch 4, **dadurch gekennzeichnet, daß** bei Schritt 3) die Sequenzen der Datenbank die Sequenzen SEQ. ID. Nr. 33 bis 51 gemäß dem Sequenzprotokoll und deren Komplementärsequenzen für die folgenden Spezies umfassen:
- für *Mycobacterium tuberculosis:*
• die Sequenzen des Fragments der intergenischen Zone MST11 bestehend aus einer der Sequenzen SEQ. ID. Nr. 33 bis 36,
• die Sequenzen des Fragments der intergenischen Zone MST12 bestehend aus einer der Sequenzen SEQ. ID. Nr. 41 bis 47,
- für *Mycobacterium africanum*:
• die Sequenz des Fragments der intergenischen Zone MST11 bestehend aus der Sequenz SEQ. ID. Nr. 37,
• die Sequenz des Fragments der intergenischen Zone MST12 bestehend aus der Sequenz SEQ. ID. Nr. 48,
- für *Mycobacterium bovis ssp bovis*:
• die Sequenz des Fragments der intergenischen Zone MST11 bestehend aus der Sequenz SEQ. ID. Nr. 38,
• die Sequenz des Fragments der intergenischen Zone MST12 bestehend aus der Sequenz SEQ. ID. Nr. 49,
- für *Mycobacterium bovis ssp BCG*:
• die Sequenz des Fragments der intergenischen Zone MST11 bestehend aus der Sequenz SEQ. ID. Nr. 39,
• die Sequenz des Fragments der intergenischen Zone MST12 bestehend aus der Sequenz SEQ. ID. Nr. 50,
- für *Mycobacterium canettii*:
• die Sequenz des Fragments der intergenischen Zone MST11 bestehend aus der Sequenz SEQ. ID. Nr. 40,
• die Sequenz des Fragments der intergenischen Zone MST12 bestehend aus der Sequenz SEQ. ID. Nr. 51.

6. Verfahren nach einem der Ansprüche 1 bis 5, bei dem man den Genotyp einer vorgegeben Spezies, Unterspezies oder eines vorgegeben Untertyps von einem Bakterium unter den Bakterien des Komplexes *Mycobacterium tuberculosis* identifiziert, **dadurch gekennzeichnet, daß** man die Schritte ausführt, in denen:
a) bei Schritt 1) man die enzymatische Amplifikation vom PCR-Typ aus der Nukleinsäure der Probe von wenigstens 8 Nukleinsäurefragmenten von wenigstens 8 intergenischen Zonen ausführt, die gewählt sind unter 16 der intergenischen Zonen MST1 bis MST16, darunter die 2 intergenischen Zonen MST11 und MST12, jeweils mit Hilfe der wenigstens 8 Primerpaare, die geeignet sind, diese Fragmente der wenigstens 8 intergenischen Zonen zu amplifizieren, wobei die Primer gebildet sind aus Oligonukleotiden mit Sequenzen, die wenigstens 12 bis 35, vorzugsweise wenigstens 18 bis 30 aufeinanderfolgende Nukleotide umfassen und weiterhin vorzugsweise umfaßt sind und liegen in den folgenden Sequenzen des Sequenzprotokolls, und deren komplementären Sequenzen, mit den 5'-, 3'-Primerpaaren, welche aus den folgenden Sequenzen für die folgenden Fragmente gezogen sind:
- für das Fragment der intergenischen Zone MST1:
• 5'-Primer = SEQ. ID. Nr. 1
• 3'-Primer = SEQ. ID. Nr. 2
- für das Fragment der intergenischen Zone MST2:
• 5'-Primer = SEQ. ID. Nr. 3
• 3'-Primer = SEQ. ID. Nr. 4
- für das Fragment der intergenischen Zone MST3:
• 5'-Primer = SEQ. ID. Nr. 5
• 3'-Primer = SEQ. ID. Nr. 6
- für das Fragment der intergenischen Zone MST4:
• 5'-Primer = SEQ. ID. Nr. 7
• 3'-Primer = SEQ. ID. Nr. 8
- für das Fragment der intergenischen Zone MST5:
• 5'-Primer = SEQ. ID. Nr. 9
• 3'-Primer = SEQ. ID. Nr. 10
- für das Fragment der intergenischen Zone MST6:
• 5'-Primer = SEQ. ID. Nr. 11
• 3'-Primer = SEQ. ID. Nr. 12
- für das Fragment der intergenischen Zone MST7:
• 5'-Primer = SEQ. ID. Nr. 13
• 3'-Primer = SEQ. ID. Nr. 14
- für das Fragment der intergenischen Zone MST8:
• 5'-Primer = SEQ. ID. Nr. 15
• 3'-Primer = SEQ. ID. Nr. 16
- für das Fragment der intergenischen Zone MST9:
• 5'-Primer = SEQ. ID. Nr. 17
• 3'-Primer = SEQ. ID. Nr. 18
- für das Fragment der intergenischen Zone MST10:
• 5'-Primer = SEQ. ID. Nr. 19
• 3'-Primer = SEQ. ID. Nr. 20
- für das Fragment der intergenischen Zone MST11:
• 5'-Primer = SEQ. ID. Nr. 21
• 3'-Primer = SEQ. ID. Nr. 22
- für das Fragment der intergenischen Zone MST12:
• 5'-Primer = SEQ. ID. Nr. 23
• 3'-Primer = SEQ. ID. Nr. 24
- für das Fragment der intergenischen Zone MST13:
• 5'-Primer = SEQ. ID. Nr. 25
• 3'-Primer = SEQ. ID. Nr. 26
- für das Fragment der intergenischen Zone MST14:
• 5'-Primer = SEQ. ID. Nr. 27
• 3'-Primer = SEQ. ID. Nr. 28
- für das Fragment der intergenischen Zone MST15:
• 5'-Primer = SEQ. ID. Nr. 29
• 3'-Primer = SEQ. ID. Nr. 30
- für das Fragment der intergenischen Zone MST16:
• 5'-Primer = SEQ. ID. Nr. 31
• 3'-Primer = SEQ. ID. Nr. 32
und
b) bei Schritt 2) man die Sequenzierung der bei Schritt a) amplifizierten Fragmente ausführt, und
c) bei Schritt 3) man im Übrigen den Genotyp der Spezies, der Unterspezies oder des Untertyps des Bakteriums identifiziert, indem man die Sequenzen der bei Schritt b) erhaltenen amplifizierten Fragmente vergleicht mit den Sequenzen einer Sequenzdatenbank der wenigstens 8 Nukleinsäurefragmente der wenigstens 8 intergenischen Zonen, die gewählt sind unter den 16 intergenischen Zonen der unterschiedlichen Genotypen der Bakterien des Komplexes *Mycobacterium tuberculosis,* wobei die verschiedenen Genotypen den verschiedenen möglichen Kombinationen der wenigstens 8 Sequenzen der wenigstens 8 genannten Nukleinsäurefragmente der wenigstens 8 genannten intergenischen Zonen entsprechen, wobei die Sequenzen, die die Datenbank bilden, bestimmt worden sind durch Amplifizierung und Sequenzierung der wenigstens 8 Fragmente der wenigstens 8 genannten intergenischen Zonen der verschiedenen Bakterien einer Sammlung verschiedener Isolate von Bakterien der Spezies, Unterspezies oder des Untertyps, welche bekannt sind vom Komplex *Mycobacterium tuberculosis,* wobei die verschiedenen Genotypen verschiedenen Kombinationen der 8 Sequenzen der wenigstens 8 intergenischen Zonen der Bakterien dieser Sammlung entsprechen.

7. Verfahren nach Anspruch 6, **dadurch gekennzeichnet, daß** die Fragmente der wenigstens 8 intergenischen Zonen 8 Fragmente der intergenischen Zonen MST1, MST2, MST3, MST4, MST8, MST11, MST12 und MST13 sind, welche durch die jeweiligen Primerpaare von Sequenzen amplifizierbar sind, welche gezogen sind aus den Sequenzen SEQ. ID. Nr. 1 bis 16 und 21 bis 26, mit:
- für das Fragment der intergenischen Zone MST1:
• 5'-Primer = SEQ. ID. Nr. 1
• 3'-Primer = SEQ. ID. Nr. 2
- für das Fragment der intergenischen Zone MST2:
• 5'-Primer = SEQ. ID. Nr. 3
• 3'-Primer = SEQ. ID. Nr. 4
- für das Fragment der intergenischen Zone MST3:
• 5'-Primer = SEQ. ID. Nr. 5
• 3'-Primer = SEQ. ID. Nr. 6
- für das Fragment der intergenischen Zone MST4:
• 5'-Primer = SEQ. ID. Nr. 7
• 3'-Primer = SEQ. ID. Nr. 8
- für das Fragment der intergenischen Zone MST8:
• 5'-Primer = SEQ. ID. Nr. 15
• 3'-Primer = SEQ. ID. Nr. 16
- für das Fragment der intergenischen Zone MST11:
• 5'-Primer = SEQ. ID. Nr. 21
• 3'-Primer = SEQ. ID. Nr. 22
- für das Fragment der intergenischen Zone MST12:
• 5'-Primer = SEQ. ID. Nr. 23
• 3'-Primer = SEQ. ID. Nr. 24
- für das Fragment der intergenischen Zone MST13:
• 5'-Primer = SEQ. ID. Nr. 25
• 3'-Primer = SEQ. ID. Nr. 26

8. Verfahren nach einem der Ansprüche 6 oder 7, **dadurch gekennzeichnet, daß** die Sequenzen der Datenbank die Sequenzen umfassen, welche gewählt sind unter SEQ. ID. Nr. 33 bis 79 des Sequenzprotokolls und deren Komplementärsequenzen für die folgenden Fragmente von intergenischen Zonen MST 1 bis MST 16:
- für das Fragment der intergenischen Zone MST1, die 4 Sequenzen SEQ. ID. Nr. 52 bis 55,
- für das Fragment der intergenischen Zone MST2, die 3 Sequenzen SEQ. ID. Nr. 56 bis 58,
- für das Fragment der intergenischen Zone MST3, die 2 Sequenzen SEQ. ID. Nr. 59 bis 60,
- für das Fragment der intergenischen Zone MST4, die 5 Sequenzen SEQ. ID. Nr. 61 bis 65,
- für das Fragment der intergenischen Zone MST8, die 3 Sequenzen SEQ. ID. Nr. 66 bis 68,
- für das Fragment der intergenischen Zone MST11, die 4 Sequenzen SEQ. ID. Nr. 33 bis 36,
- für das Fragment der intergenischen Zone MST12, die 7 Sequenzen SEQ. ID. Nr. 41 bis 47,
- für das Fragment der intergenischen Zone MST13, die 11 Sequenzen SEQ. ID. Nr. 69 bis 79.

9. Verfahren nach einem der Ansprüche 1 bis 8, **dadurch gekennzeichnet, daß** man zuvor überprüft, daß die Probe, auf der man die Identifizierung ausführen will, wirklich Nukleinsäuren eines genannten Bakteriums des Komplexes *Mycobacterium tuberculosis* enthält.

10. Isoliertes Oligonukleotid, das geeignet ist als Amplifikationsprimer eines Nukleinsäurefragments der intergenischen Zone zu dienen, welches gewählt ist unter den Nukleinsäurefragmenten der 8 intergenischen Zonen MST1, MST2, MST3, MST4, MST8, MST11, MST12 und MST13, welches verwendbar ist in einem Verfahren nach einem der Ansprüche 1 bis 9, **dadurch gekennzeichnet, daß** es aus einer Sequenz gebildet ist, die in den Sequenzen SEQ. ID. 1 bis 8, 15 bis 16 und 21 bis 26 des Sequenzprotokolls und deren Komplementärsequenzen liegt, entsprechend den folgenden 5'-, 3'-Primerpaaren:
- für das Fragment der intergenischen Zone MST1:
· 5'-Primer = SEQ. ID. Nr. 1
· 3'-Primer = SEQ. ID. Nr. 2
- für das Fragment der intergenischen Zone MST2:
· 5'-Primer = SEQ. ID. Nr. 3
· 3'-Primer = SEQ. ID. Nr. 4
- für das Fragment der intergenischen Zone MST3:
· 5'-Primer = SEQ. ID. Nr. 5
· 3'-Primer = SEQ. ID. Nr. 6
- für das Fragment der intergenischen Zone MST4:
· 5'-Primer = SEQ. ID. Nr. 7
· 3'-Primer = SEQ. ID. Nr. 8
- für das Fragment der intergenischen Zone MST8:
· 5'-Primer = SEQ. ID. Nr. 15
· 3'-Primer = SEQ. ID. Nr. 16
- für das Fragment der intergenischen Zone MST11:
· 5'-Primer = SEQ. ID. Nr. 21
· 3'-Primer = SEQ. ID. Nr. 22
- für das Fragment der intergenischen Zone MST12:
· 5'-Primer = SEQ. ID. Nr. 23
· 3'-Primer = SEQ. ID. Nr. 24
- für das Fragment der intergenischen Zone MST13:
· 5'-Primer = SEQ. ID. Nr. 25
· 3'-Primer = SEQ. ID. Nr. 26

11. Isoliertes Nukleinsäurefragment einer nicht kodierenden intergenischen Zone eines Bakterium des Komplexes *Mycobacterium tuberculosis,* welches verwendbar ist, um eine Datenbank in einem Verfahren nach einem der Ansprüche 1 bis 9 zu bilden, gewählt unter den Nukleinsäurefragmenten der 8 intergenischen Zonen MST1, MST2, MST3, MST4, MST8, MST11, MST12 und MST13, **dadurch gekennzeichnet, daß** seine Sequenz gewählt ist unter den Sequenzen SEQ. ID. Nr. 33 bis 79 des Sequenzprotokolls und deren Komplementärsequenzen, mit den folgenden Sequenzen für die folgenden Spezies:
- für die Spezies *Mycobacterium* tuberculosis:
· die Sequenzen des Fragments der intergenischen Zone MST1, bestehend aus den 4 Sequenzen SEQ. ID. Nr. 52 bis 55,
· die Sequenzen des Fragments der intergenischen Zone MST2, bestehend aus den 3 Sequenzen SEQ. ID. Nr. 56 bis 58,
· die Sequenzen des Fragments der intergenischen Zone MST3, bestehend aus den 2 Sequenzen SEQ. ID. Nr. 59 bis 60,
· die Sequenzen des Fragments der intergenischen Zone MST4, bestehend aus den 5 Sequenzen SEQ. ID. Nr. 61 bis 65,
· die Sequenzen des Fragments der intergenischen Zone MST8, bestehend aus den 3 Sequenzen SEQ. ID. Nr. 66 bis 68,
· die Sequenzen des Fragments der intergenischen Zone MST11, bestehend aus den 4 Sequenzen SEQ. ID. Nr. 33 bis 36,
· die Sequenzen des Fragments der intergenischen Zone MST12, bestehend aus den 7 Sequenzen SEQ. ID. Nr. 41 bis 47,
· die Sequenzen des Fragments der intergenischen Zone MST13, bestehend aus den 11 Sequenzen SEQ. ID. Nr. 69 bis 79.
- für *Mycobacterium africanum*:
· die Sequenz des Fragments der intergenischen Zone MST11, bestehend aus der Sequenz SEQ. ID. Nr. 37, und
· die Sequenz des Fragments der intergenischen Zone MST12, bestehend aus der Sequenz SEQ. ID. Nr. 48,
- für *Mycobacterium bovis ssp bovis:*
· die Sequenz des Fragments der intergenischen Zone MST11, bestehend aus der Sequenz SEQ. ID. Nr. 38, und
· die Sequenz des Fragments der intergenischen Zone MST12, bestehend aus der Sequenz SEQ. ID. Nr. 49,
- für *Mycobacterium bovis ssp BCG:*
· die Sequenz des Fragments der intergenischen Zone MST11, bestehend aus der Sequenz SEQ. ID. Nr. 39, und
· die Sequenz des Fragments der intergenischen Zone MST12, bestehend aus der Sequenz SEQ. ID. Nr. 50,
- für *Mycobacterium canettii*:
· die Sequenz des Fragments der intergenischen Zone MST11, bestehend aus der Sequenz SEQ. ID. Nr. 40, und
· die Sequenz des Fragments der intergenischen Zone MST12, bestehend aus der Sequenz SEQ. ID. Nr. 51.

12. Isoliertes Nukleinsäurefragment einer nicht kodierenden intergenischen Zone eines Bakteriums des Komplexes *Mycobacterium tuberculosis* gemäß Anspruch 11, gewählt unter den Nukleinsäurefragmenten der 2 genannten intergenischen Zonen MST11 und MST12, **dadurch gekennzeichnet, daß** seine Sequenz gewählt ist unter den Sequenzen SEQ. ID. Nr. 33 bis 51 des Sequenzprotokolls und deren Komplementärsequenzen, mit den folgenden Sequenzen für die folgenden Spezies:
- für die Spezies *Mycobacterium* tuberculosis:
· die Sequenzen des Fragments der intergenischen Zone MST11, bestehend aus den 4 Sequenzen SEQ. ID. Nr. 33 bis 36,
· die Sequenzen des Fragments der intergenischen Zone MST12, bestehend aus den 7 Sequenzen SEQ. ID. Nr. 41 bis 47,
- für *Mycobacterium africanum*:
· die Sequenz des Fragments der intergenischen Zone MST11, bestehend aus der Sequenz SEQ. ID. Nr. 37, und
· die Sequenz des Fragments der intergenischen Zone MST12, bestehend aus der Sequenz SEQ. ID. Nr. 48,
- für *Mycobacterium bovis ssp bovis:*
· die Sequenz des Fragments der intergenischen Zone MST11, bestehend aus der Sequenz SEQ. ID. Nr. 38, und
· die Sequenz des Fragments der intergenischen Zone MST12, bestehend aus der Sequenz SEQ. ID. Nr. 49,
- für *Mycobacterium bovis ssp BCG*:
· die Sequenz des Fragments der intergenischen Zone MST11, bestehend aus der Sequenz SEQ. ID. Nr. 39, und
· die Sequenz des Fragments der intergenischen Zone MST12, bestehend aus der Sequenz SEQ. ID. Nr. 50,
- für *Mycobacterium canettii*:
· die Sequenz des Fragments der intergenischen Zone MST11, bestehend aus der Sequenz SEQ. ID. Nr. 40, und
· die Sequenz des Fragments der intergenischen Zone MST12, bestehend aus der Sequenz SEQ. ID. Nr. 51.


### SEQUENCE LISTING

<110> UNIVERSITE DE LA MEDITERRANEE (Aix-Marseille II) Centre National de la Recherche Scientifique (CNRS)
<120> Procédé d'Identification moléculaire et génotypage des bactéries du complexe Mycobacterium tuberculosis
<130> H52 437 cas 18 FR
<160> 83
<170> Patent In version 3.1
<210> 1
   <211> 18
   <212> DNA
   <213> Mycobacterium tuberculosis complex
<400> 1
   gatggtctcccggctgat 18
<210> 2
   <211> 18
   <212> DNA
   <213> Mycobacterium tuberculosis complex
<400> 2
   gctggccgatctgcgcgc 18
<210> 3
   <211> 20
   <212> DNA
   <213> Mycobacerium tuberculosis complex
<400> 3
   gcgccaaggccaccggccaa 20
<210> 4 <210> 4
   <211> 21
   <212> DNA
   <213> Mycobacterium tuberculosis complex
<400> 4
   gcccggccagcggtgaactgg 21
<210> 5
   <211> 20
   <212> DNA
   <213> Mycobacterium tuberculosis complex
<400> 5
   ctgtggcaggctcccggtag 20
<210> 6
   <211> 19
   <212> DNA
   <213> Mycobacterium tuberculosis complex
<400> 6
   tcgaggattctgggactat 19
<210> 7
   <211> 20
   <212> DNA
   <213> Mycobacterium tuberculosis complex
<400> 7
   gatcagctacgggttggccg 20
<210> 8
<211> 21
   <212> DNA
   <213> Mycobacterium tuberculosis complex
<400> 8
   atgggttcgccagacggcgag 21
<210> 9
   <211> 20
   <212> DNA
   <213> Mycobacterium tuberculosis complex
<400> 9
   cggccagccgcggcggacga 20
<210> 10
   <211> 21
   <212> DNA
   <213> Mycobacterium tuberculosis complex
<400> 10
   gattgcgttgctggacggccc 21
<210> 11
   <211> 20
   <212> DNA
   <213> Mycobacterium tuberculosis complex
<400> 11
   ctgtgagatcggtgtgcttt 20
<210> 12
   <211> 20
   <212> DNA
   <213> Mycobacterium tuberculosis complex
<400> 12
   ctgctgatcgtgatgctgaa 20
<210> 13
   <211> 20
   <212> DNA
   <213> Mycobacterium tuberculosis complex
<400> 13
   gcaccggattcaacgtattc 20
<210> 14
   <211> 20
   <212> DNA
   <213> Mycobacterium tuberculosis complex
<400> 14
   tagtagggcactagcacctc 20
<210> 15
   <211> 22
   <212> DNA
   <213> Mycobacterium tuberculosis complex
<400> 15
   ccgcaatcacaaacttcaatac 22
<210> 16
   <211> 20
   <212> DNA
   <213> Mycobacterium tuberculosis complex
<400> 16
   gctacttcgacgacgtgtat 20
<210> 17
   <211> 20
   <212> DNA
   <213> Mycobacterium tuberculosis complex
<400> 17
   cttcatgacgttggatcgct 20
- <210> 18
   <211> 20
   <212> DNA
   <213> Mycobacterium tuberculosis complex
<400> 18
   cgcaatgcgactcgaatttc 20
<210> 19
   <211> 20
   <212> DNA
   <213> Mycobacterium tuberculosis complex
<400> 19
   ctttgggcgatttcatcgag 20
<210> 20
   <211> 21
   <212> DNA
   <213> Mycobacterium tuberculosis complex
<400> 20
   ctcttcctcgaggtcgtagat 21
<210> 21
   <211> 20
   <212> DNA
   <213> Mycobacterium tuberculosis complex
<400> 21
   aggtgttagaggtggtggat 20
<210> 22
   <211> 23
   <212> DNA
   <213> Mycobacterium tuberculosis complex
<400> 22
   aaccaaagtcatattgggatgag 23
<210> 23
   <211> 20
   <212> DNA
   <213> Mycobacterium tuberculosis complex
<400> 23
   gttgatcgaggcctatcacg 20
<210> 24
   <211> 20
   <212> DNA
   <213> Mycobacterium tuberculosis complex
<400> 24
   gaatagggcttggtcacgta 20
<210> 25
   <211> 20
   <212> DNA
   <213> Mycobacterium tuberculosis complex
<400> 25
   cgagttcaccgtccatcatc 20
<210> 26
   <211> 20
   <212> DNA
   <213> Mycobacterium tuberculosis complex
<400> 26
   gagacaacggtcatcgactt 20
<210> 27
   <211> 22
   <212> DNA
   <213> Mycobacterium tuberculosis complex
<400> 27
   catagt gaggagtaacgactaa 22
<210> 28
   <211> 20
   <212> DNA
   <213> Mycobacterium tuberculosis complex
<400> 28
   ctgatcatggtgatcaccga 20
<210> 29
   <211> 19
   <212> DNA
   <213> Mycobacterium tuberculosis complex
<400> 29
   tttgcggcaacgagatctt 19
<210> 30
   <211> 19
   <212> DNA
   <213> Mycobacterium tuberculosis complex
<400> 30
   gatgcatgttcgacccgta 19
<210> 31
   <211> 22
   <212> DNA
   <213> Mycobacterium tuberculosis complex
<400> 31
   ttaggcaagttccgaatgacag 22
<210> 32
   <211> 24
   <212> DNA
   <213> Mycobacterium tuberculosis complex
<400> 32
   ctatagagctaagaagttgtgtcc 24
<210> 33
   <211> 693
   <212> DNA
   <213> Mycobacterium tuberculosis
<400> 33
   aggtgttaga ggtggtggat tcggacttca atgcgttgtt ggagttggga tttcggtcgt 60
   cgattcaacg agagtggcag tggcggttat ctcgcggtga gtcgctgcag aacctgcagg 120
   ccttcgctca cttacgcccg acgacgatgc agagcgcgca gcgcgatgag aaggagttgg 180
   gcggttaggt cgagcccgac gacgatgcag agcgcgcagc gcgatgagaa ggagttgggc 240
   ggttaggtcg agcccgacga cgatgcagag cgcgcagcgc gatgagaagg agttgggcgg 300
   ttaggtcgag cccgacgacg atgcagagcg cgcagcgcga tgagaaatag cactcgtgga 360
   ggtcaagacg cccgccggtg atgggctggt ggcgctcacc ccgttccgga ctcagaaatt 420
   cgcgatcaca atttgcgcgt tcaagtcatt ggcatgcatg tgatggttta gcgttccgct 480
   gtgcctcttc aggtgtttgt cggcttcgtt gccatgatga cgctcaaggt cgcgatcggc 540
   ccgcaaaacg catttgtcct gcgccaagga attaggcgag aatacgtgct ggtcattgtg 600
   gcgctgtgcg ggatcgctga tggggcactg attgccgcgg gcgttggcgg cttcgctgcg 660
   ctgattcacg ctcatcccaa tatgactttg gtt 693
<210> 34
   <211> 693
   <212> DNA
   <213> Mycobacterium tuberculosis
<400> 34
   aggtgttaga ggtggtggat tcggacttca atgcgttgtt ggagttggga tttcggtcgt 60
   cgattcaacg agagtggcag tggcggttat ctcgcggtga gtcgctgcag aacctgcagg 120
   ccttcgctca cttacgcctg acgacgatgc agagcgcgca gcgcgatgag aaggagttgg 180
   gcggttaggt cgagcccgac gacgatgcag agcgcgcagc gcgatgagaa ggagttgggc 240
   ggttaggtcg agcccgacga cgatgcagag cgcgcagcgc gatgagaagg agttgggcgg 300
   ttaggtcgag cccgacgacg atgcagagcg cgcagcgcga tgagaaatag cactcgtgga 360
   ggtcaagacg cccgccggtg atgggctggt ggcgctcacc ccgttccgga ctcagaaatt 420
   cgcgatcaca atttgcgcgt tcaagtcatt ggcatgcatg tgatggttta gcgttccgct 480
   gtgcctcttc aggtgtttgt cggcttcgtt gccatgatga cgctcaaggt cgcgatcggc 540
   ccgcaaaacg catttgtcct gcgccaagga attaggcgag aatacgtgct ggtcattgtg 600
   gcgctgtgcg ggatcgctga tggggcactg attgccgcgg gcgttggcgg cttcgctgcg 660
   ctgattcacg ctcatcccaa tatgactttg gtt 693
<210> 35
   <211> 629
   <212> DNA
   <213> Mycobacterium tuberculosis
<400> 35
   aggtgttaga ggtggtggat tcggacttca atgcgttgtt ggagttggga tttcggtcgt 60
   cgattcaacg agagtggcag ggcggttatc tcgcggtgag tcgctgcaga acctgcaggc 120
   cttcgctcac ttacgcccga cgacgatgca gagcgcgcag cgcgatgaga aggagttggg 180
   cggttaggtc gagcccgacg acgatgcaga gcgcgcagcg cgatgagaag gagttgggcg 240
   aggtcgagcc cgacgacgat gcagagcgcg cagcgcgatg agaaatagca ctcgtggagg 300
   tcaagacgcc cgccggtgat ggctggtggc gctcaccccg ttccggactc agaaattcgc 360
   gatcacaatt tgcgcgttca agtcattggc atgcatgtga tggttagcgt tccgctgtgc 420
   ctcttcaggt gtttgtcggc ttcgttgcca tgatgacgct caaggtcgcg atcggcccgc 480
   aaaacgcatt tgtcctgcgc caaggaatta ggcgagaata cgtgctggtc attgtggcgc 540
   tgtgcgggat cgctgatggg gcactgattg ccgcgggcgt tggcggcttc gctgcgctga 600
   ttcacgctca tcccaatatg actttggtt 629
<210> 36
   <211> 575
   <212> DNA
   <213> Mycobacterium tuberculosis
<400> 36
   aggtgttaga ggtggtggat tcggacttca atgcgttgtt ggagttggga tttcggtcgt 60
   cgattcaacg agagtggcag tggcggttat ctcgcggtga gtcgctgcag aacctgcagg 120
   ccttcgctca cttacgcccg acgacgatgc agagcgcgca gcgcgatgag aaggagttgg 180
   gcggttaggt cgagcccgac gacgatgcag agcgcgcagc gcgatgagaa atagcactcg 240
   tggaggtcaa gacgcccgcc ggtgatgggc tggtggcgct caccccgttc cggactcaga 300
   aattcgcgat cacaatttgc gcgttcaagt cattggcatg catgtgatgg tttagcgttc 360
   cgctgtgcct cttcaggtgt ttgtcggctt cgttgccatg atgacgctca aggtcgcgat 420
   cggcccgcaa aacgcatttg tcctgcgcca aggaattagg cgagaatacg tgctggtcat 480
   tgtggcgctg tgggatcgct gatggggcac tgattgccgc gggcgttggc ggcttcgctg 540
   cgctgattca cgctcatccc aatatgactt tggtt 575
<210> 37
   <211> 757
   <212> DNA
   <213> Mycobacterium africanum
<400> 37
   aggt gt t aga ggt ggt ggat t cggact t ca at gcgt t gt t ggagt t ggga t t t cggt cgt 60
   cgat t caacg agagt ggcag t ggcggt t at ct cgcggt ga gt cgct gcag aacct gcagg 120
   ccttcgctca cttacgcccg acgacgatgc agagcgcgca gcgcgatgag aaggagttgg     180
   gcggttaggt cgagcccgac gacgatgcag agcgcgcagc gcgatgagaa ggagttgggc     240
   ggttaggtcg agcccgacga cgatgcagag cgcgcagcgc gatgagaagg agttgggcgg     300
   ttaggtcgag cccgacgacg atgcagagcg cgcagcgcga tgagaaggag ttgggcggtt     360
   aggtcgagct cgagcccgac gacgatgcag agccgcgcag cgcgatgaga aatagcactc     420
   gtggaggtca agacgcccgc cggtgatggg ctggtggcgc tcaccccgtt ccggactcag     480
   aaat t cgcga t cacaat t t g cgcgt t caag t cat t ggcat gcat gt gat g gt t t agcgt t 540
   ccgct gt gcc t ct t caggt g t t t gt cggct t cgt t gccat gat gacgct c aaggt cgcga 600
   t cggcccgca aaacgcat t t gt cct gcgcc aaggaat t ag gcgagaat ac gt gct gt cat 660
   t gt ggcgct g t gcgggat cg ct gat ggggc act gat t gcc gcgggcgt t g gcggct t cgc 720
   t gcgct gat t cacgct cat c ccaat at gac t t t ggt t 757
<210> 38
   <211> 810
   <212> DNA
   <213> Mycobacterium bovis sssp bovis
<400> 38
   aggt gt t aga ggt ggt ggat t cggact t ca at gcgt t gt t ggagt t ggga t t t cggt cgt 60
   cgat t caacg agagt ggcag t ggcggt t at ct cgcggt ga gt cgct gcag aacct gcagg 120
   ccttcgctca cttacgcccg acgacgatgc agagcgcgca gcgcgatgag aaggagttgg     180
   gcggttaggt cgagcccgac gacgatgcag agcgcgcagc gcgatgagaa ggagttgggc     240
   ggttaggtcg agcccgacga cgatgcagag cgcgcagcgc gatgagaagg agttgggcgg     300
   ttaggtcgag cccgacgacg atgcagagcg cgcagcgcga tgagaaggag ttgggcggtt     360
   aggtcgagcc cgacgacgat gcagagcgcg cagcgcgatg agaaggagtt gggcggttag     420
   gtcgagcccg acgacgatgc agaagcgcgc agcgcgatga gaaatagcac tcgtggaggt     480
   caagacgccc gccggtgatg ggctggtggc gctcaccccg ttccggactc agaaattcgc     540
   gat cacaat t t gcgcgt t ca agt cat t ggc at gcat gt ga t ggt t t agcg t t ccgct gt g 600
   cct ct t cagg t gt t t gt cgg ct t cgt t gcc at gat gacgc t caaggt cgc gat cggcccg 660
   caaaacgcat ttgtcctgcg ccaaggaatt aggcgagaat acgtgctggt cattgtggcg     720
   ctgtgcggga tcgctgatgg ggcactgatt gccgcgggcg ttggcggctt cgctgcgctg     780
   at t cacgct c at cccaat at gact t t ggt t 810
<210> 39
   <211> 750
   <212> DNA
   <213> Mycobacterium bovis ssp BCG
<400> 39
   aggtgttaga ggtggtggat tcggacttca atgcgttgtt ggagttggga tttcggtcgt 60
   cgattcaacg agagtggcag tggcggttat ctcgcggtga gtcgctgcag aacctgcagg 120
   ccttcgctca cttacgcccg acgacgatgc agagcgcgca gcgcgatgag aaggagttgg 180
   gcggttaggt cgagcccgac gacgatgcag agcgcgcagc gcgatgagaa ggagttgggc 240
   ggttaggtcg agcccgacga cgatgcagag cgcgcagcgc gatgagaagg agttgggcgg 300
   ttaggtcgag cccgacgacg atgcagagcg cgcagcgcga tgagaaggag ttgggcggtt 360
   aggtcgagcc cgacgacgat gcagagcgcg cagcgcgatg agaaatagca ctcgtggagg 420
   tcaagacgcc cgccggtgat gggctggtgg cgctcacccc gttccggact cagaaattcg 480
   cgatcacaat ttgcgcgttc aagtcattgg catgcatgtg atggtttagc gttccgctgt 540
   gcctcttcag gtgtttgtcg gcttcgttgc catgatgacg ctcaaggtcg cgatcggccc 600
   gcaaaacgca tttgtcctgc gccaaggaat taggcgagaa tacgtgctgg tcattgtggc 660
   gctgtgcggg atcgctgatg gggcactgat tgccgcgggc gttggcggct tcgctgcgct 720
   gattacgctc atcccaatat gactttggtt 750
<210> 40
   <211> 635
   <212> DNA
   <213> Mycobacterium canettii
<400> 40
   aggtgttaga ggtggtggat tcggacttca atgcgttgtt ggagttggga tttcggtcgt 60
   cgattcaacg agagtggcag tggcggttat ctcgcggtga gtcgctgcag aacatgcagg 120
   ccttcgctca cttgcgcccg acgacgatgc agagcgcgca gcgcgatgag aaggagttgg 180
   gcggttaggt cgagcccgac gacgatgcag agcgcgcagc gcgatgagaa ggagttgggc 240
   ggttaggtcg agcccgacga cgatgcagag cgcgcagcgc gatgagaaat agcactcgtg 300
   gaggtcaaga cgcccgccgg tgatgggctg gtggcgctca ccccgttccg gactcagaaa 360
   ttcgcgatca caatttgcgc gttcaagtca ttggcatgca tgtgatggtt tagcgttccg 420
   ctgtgcctct tcaggtgttt gtcggcttcg ttgccatgat gacgctcaag gtcgcgatcg 480
   gcccgcaaaa cgcatttgtc ctgcgccaag gaattaggcg agaatacgtg ctggtcattg 540
   tggcgctgtg cgggatcgct gatggggcac tgattgccgc gggcgttggc ggcttcgctg 600
   cgctgattca cgctcatccc aatatgactt tggtt 635
<210> 41
   <211> 838
   <212> DNA
   <213> Mycobacterium tuberculosis
<400> 41
   gttgatcgag gcctatcacg acgacgagcg acccgagcag gcgcgagagc ccgaactgcg      60
   gtcaaacagg tcacaacgag aggaagagct gagccgatga cctgcgccga cgacgatgca     120
   gagcgtagcg atgaggtggg ggcaccaccc gcttgcggag agtggcgctg atgacctgcg     180
   ccgacgacga tgcagagcgt agcgatgagg tgggggcacc acccgcttgc ggggagatgg     240
   cgctgatgac ctgcgccgac gacgatgcag agcgtagcga tgaggtgggg gcaccacccg     300
   cttgcggggg agagtggcct gatgacctgc gccgacgacg atgcagagcg tagcgatgag     360
   gtgggggcac cacccgcttg cgggggagag tggcgctgat gacctgcgcc gacgacgatg     420
   cagagcgtag cgatgaggtg ggggcaccac ccgcttgcgg gggagagtgg cgctgatgac    480
   ct gcgccgac gacgat gcag agcgt agcga t gaggaggag t ggcgct gat gaccagt gt g 540
   ttgattgtgg aggacgagga gtcgctggcc gatccgctgg cgtttctgct gcgcaaggag     600
   ggctttgagg ccacggtggt gaccgatggt ccggcagctc tcgccgagtt cgaccgggcc     660
   ggcgccgaca tcgtcctgct cgatctgatg ctgcctggga tgtcgggtac cgatgtatgc     720
   aagcagt t gc gcgct cggt c cagcgt t ccg gt gat cat gg t gaccgcccg ggat agcgag 780
   at cgacaagg t ggt cggcct ggagct ggcg ct gacgact a cgt gaccaag ccct at t c 838
<210> 42
   211> 768
   <212> DNA
   <213> Mycobacterium tuberculosis
<400> 42
   gttgatcgag gcctatcacg acgacgagcg acccgagcag gcgcgagagc ccgaactgcg      60
   gtcaaacagg tcacaacgag aggaagagct gagccgatga cctgcgccga cgacgatgca     120
   gagcgtagcg atgaggtggg ggcaccaccc gcttgcgggg gagagtggcg ctgatgacct     180
   gcgccgacga cgatgcagag cgtagcgatg aggtgggggc accacccgct tgcgggggag     240
   agtggcgctg atgacctgcg ccgacgacga tgcagagcgt agcgatgagg tgggggcacc     300
   acccgcttgc gggggagagt ggcgctgatg acctgcgccg acgacgatgc agagcgtagc     360
   gatgaggtgg gggcaccacc cgcttgcggg ggagagtggc gctgatgacc tgcgccgacg     420
   acgatgcaga gcgtagcgat gaggaggagt ggcgctgatg accagtgtgt tgattgtgga     480
   ggacgaggag tcgctggccg atccgctggc gtttctgctg cgcaaggagg gctttgaggc     540
   cacggtggtg accgatggtc cggcagctct cgccgagttc gaccgggccg gcgccgacat     600
   cgt cct gct c gat ct gat gc t gcct gggat gt cgggt acc gat gt at gca agcagt t gcg 660
   cgctcggtcc agcgttccgg tgatcatggt gaccgcccgg gatagcgaga tcgacaaggt     720
   ggt cggcct g gagct gggcg ct gacgact a cgt gaccaag ccct at t c 768
<210> 43
   <211> 691
   <212> DNA
   <213> Mycobacterium tuberculosis
<400> 43
   gttgatcgag gcctatcacg acgacgagcg acccgagcag gcgcgagagc ccgaactgcg 60
   gtcaaacagg tcacaacgag aggaagagct gagccgatga cctgcgccga cgacgatgca 120
   gagcgtagcg atgaggtggg ggcaccaccc gcttgcgggg gagagtggcg ctgatgacct 180
   gcgccgacga cgatgcagag cgtagcgatg aggtgggggc accacccgct tgcgggggag 240
   agtggcgctg atgacctgcg ccgacgacga tgcagagcgt agcgatgagg tgggggcacc 300
   acccgcttgc gggggagagt ggcgctgatg acctgcgccg acgacgatgc agagcgtagc 360
   gatgaggagg agtggcgctg atgaccagtg tgttgattgt ggaggacgag gagtcgctgg 420
   ccgat ccgct ggcgt t t ct g ct gcgcaagg agggct t t ga ggccacggt g gt gaccgat g 480
   gtccggcagc tctcgccgag ttcgaccggg ccggcgccga catcgtcctg ctcgatctga 540
   t gct gcct gg gat gt cgggt accgat gt at gcaagcagt t gcgcgct cgg t ccagcgt t c 600
   cggt gat cat ggt gaccgcc cgggat agcg agat cgacaa ggt ggt cggc ct ggagct gg 660
   gcgct gacga ct acgt gacc aagccct at t c 691
<210> 44 <211> 638 <212> DNA <213> Mycobacterium tuberculosis <400> 44
<210> 45
   <211> 614
   <212> DNA
   <213> Mycobacterium tuberculosis
<400> 45
   gttgatcgag gcctatcacg acgacgagcg acccgagcag gcgcgagagc ccgaactgcg 60
   gtcaaacagg tcacaacgag aggaagagct gagccgatga cctgcgccga cgacgatgca 120
   gagcgtagcg atgaggtggg ggcaccaccc gcttgcgggg gagagtggcg ctgatgacct 180
   gcgccgacga cgatgcagag cgtagcgatg aggtgggggc accacccgct tgcgggggag 240
   agtggcgctg atgacctgcg ccgacgacga tgcagagcgt agcgatgagg aggagtggcg 300
   ctgatgacca gtgtgttgat tgtggaggac gaggagtcgc tggccgatcc gctggcgttt 360
   ctgctgcgca aggagggctt tgaggccacg gtggtgaccg atggtccggc agctctcgcc 420
   gagttcgacc gggccggcgc cgacatcgtc ctgctcgatc tgatgctgcc tgggatgtcg 480
   ggtaccgatg tatgcaagca gttgcgcgct cggtccagcg ttccggtgat catggtgacc 540
   gcccgggata gcgagatcga caaggtggtc ggcctggagc tgggcgctga cgactacgtg 600
   accaagccct attc 614
<210> 46
   <211> 460
   <212> DNA
   <213> Mycobacterium tuberculosis
<400> 46
   gttgatcgag gcctatcacg acgacgagcg acccgagcag gcgcgagagc ccgaactgcg 60
   gtcaaacagg tcacaacgag aggaagagct gagccgatga cctgcgccga cgacgatgca 120
   gagcgtagcg atgaggagga gtggcgctga tgaccagtgt gttgattgtg gaggacgagg 180
   agtcgctggc cgatccgctg gcgtttctgc tgcgcaagga gggctttgag gccacggtgg 240
   tgaccgatgg tccggcagct ctcgccgagt tcgaccgggc cggcgccgac atcgtcctgc 300
   tcgatctgat gctgcctggg atgtcgggta ccgatgtatg caagcagttg cgcgctcggt 360
   ccagcgttcc ggtgatcatg gtgaccgccc gggatagcga gatcgacaag gtggtcggcc 420
   tggagctggg cgctgacgac tacgtgacca agccctattc 460
<210> 47
   <211> 614
   <212> DNA
   <213> Mycobacterium tuberculosis
<400> 47
   gttgatcgag gcctatcacg acgacgagcg acccgagcag gcgcgagagc ccgaactgcg 60
   gtcaaacagg tcacaacgag aggaagagct gagccgatga cctgcgccga cgacgatgca 120
   gagcgt agcg at gaggt ggg t gcaccaccc gct t gcgggg gagagt ggcg ct gat gacct 180
   gcgccgacga cgatgcagag cgtagcgatg aggtgggtgc accacccgct tgcgggggag 240
   agtggcgctg atgacctgcg ccgacgacga tgcagagcgt agcgatgagg aggagtggcg 300
   ct gat gacca gt gt gt t gat t gt ggaggac gaggagt cgc t ggccgat cc gct ggcgt t t 360
   ct gct gcgca aggagggct t t gaggccacg gt ggt gaccg at ggt ccggc agct ct cgcc 420
   gagttcgacc gggccggcgc cgacatcgtc ctgctcgatc tgatgctgcc tgggatgtcg 480
   ggt accgat g t at gcaagca gt t gcgcgct cggt ccagcg t t ccggt gat cat ggt gacc 540
   gcccgggat a gcgagat cga caaggt ggt c ggcct ggagc t gggcgct ga cgact acgt g 600
   accaagccct at t c 614
<210> 48

   <211> 691
   <212> DNA
   <213> Mycobacterium africanum
<400> 48
   gttgatcgag gcctatcacg acgacgagcg acccgagcag gcgcgagagc ccgaactgcg 60
   gtcaaacagg tcacaacgag aggaagagct gagctgatga cctgcgccga cgacgatgca 120
   gagcgtagcg atgaggtggg ggcaccaccc gcttgcgggg gagagtggcg ctgatgacct 180
   gcgccgacga cgatgcagag cgtagcgatg aggtgggggc accacccgct tgcgggggag 240
   agtggcgctg atgacctgcg ccgacgacga tgcagagcgt agcgatgagg tgggggcacc 300
   acccgcttgc gggggagagt ggcactgatg acctgcgccg acgacgatgc agagcgtagc 360
   gatgaggagg agtggcgctg atgaccagtg tgttgattgt ggaggacgag gagtcgctgg 420
   ccgat ccgct ggcgt t t ct g ct gcgcaagg agggct t t ga ggccacggt g gt gaccgat g 480
   gtccggcagc tctcgccgag ttcgaccggg ccggcgccga catcgtcctg ctcgatctga 540
   t gct gcct gg gat gt cgggt accgat gt at gcaagcagt t gcgcgct cgg t ccagcgt t c 600
   cggt gat cat ggt gaccgcc cgggat agcg agat cgacaa ggt ggt cggc ct ggagct gg 660
   gcgct gacga ct acgt gacc aagccct at t c 691
<210> 49
   <211> 561
   <212> DNA
   <213> Mycobacterium bovis ssp bovis
<400> 49
   gttgatcgag gcctatcacg acgacgagcg acccgagcag gcgcgagagc ccgaactgcg      60
   gtcaaacagg tcacaacgag aggaagagct gagccgatga cctgcgccga cgacgatgca     120
   gagcgtagcg atgaggtggg ggcaccaccc gcttgcgggg gagagtggcg ctgatgacct     180
   gcgccgacga cgatgcagag cgtagcgatg aggtgggggc accacccgct tgcgggggag     240
   agtggcgctg atgaccagtg tgttgattgt ggaggacgag gagtcgctgg ccgatccgct     300
   gacgt t t ct g ct gcgcaagg agggct t t ga ggccacggt g gt gaccgat g gt ccggcagc 360
   tctcgccgag ttcgaccggg ccggcgccga catcgtcctg ctcgatctga tgctgcctgg     420
   gat gt cgggt accgat gt at gcaagcagt t gcgcgct cgg t ccagcgt t c cggt gat cat 480
   ggt gaccgcc cgggat agcg agat cgacaa ggt ggt cggc ct ggagct gg gcgct gacga 540
   ct acgt gacc aagccct at t c 561
<210> 50
   <211> 638
   <212> DNA
   <213> Mycobacterium bovis ssp BCG
<400> 50
   gttgatcgag gcctatcacg acgacgagcg acccgagcag gcgcgagagc ccgaactgcg      60
   gtcaaacagg tcacaacgag aggaagagct gagccgatga cctgcgccga cgacgatgca     120
   gagcgtagcg atgaggtggg ggcaccaccc gcttgcgggg gagagtggcg ctgatgacct     180
   gcgccgacga cgatgcagag cgtagcgatg aggtgggggc accacccgct tgcgggggag     240
   agtggcgctg atgacctgcg ccgacgacga tgcagagcgt agcgatgagg tgggggcacc     300
   acccgcttgc gggggagagt ggcgctgatg accagtgtgt tgattgtgga ggacgaggag     360
   tcgctggccg atccgctgac gtttctgctg cgcaaggagg gctttgaggc cacggtggtg     420
   accgatggtc cggcagctct cgccgagttc gaccgggccg gcgccgacat cgtcctgctc     480
   gat ct gat gc t gcct gggat gt cgggt acc gat gt at gca agcagt t gcg cgct cggt cc 540
   agcgttccgg tgatcatggt gaccgcccgg gatagcgaga tcgacaaggt ggtcggcctg     600
   gagct gggcg ct gacgact a cgt gaccaag ccct at t c 638
<210> 51
   <211> 537
   <212> DNA
   <213> Mycobacterium canettii
<400> 51
   gttgatcgag gcctatcacg acgacgagcg acccgagcag gcgcgagagc ccgaactgcg    60
   gtcaaacagg tcacaacgag aggaagagct gagccgatga cctgcgccga cgacgatgca   120
   gagcgtagcg atgaggtggg ggcaccaccc gcttgcgggg gagagtggcg ctgatgacct   180
   gcgccgacga cgatgcagag cgtagcgatg aggaggagtg gcgctgatga ccagtgtgtt   240
   gattgtggag gacgaggagt cgctggccga tccgctggcg tttctgctgc gcaaggaggg   300
   ctttgaggcc acggtggtga ccgatggtcc ggcagctctc gccgagttcg accgggccgg   360
   cgccgacat c gt cct gct cg at ct gat gct gcct gggat g t cgggt accg at gt at gcaa 420
   gcagttgcgc gctcggtcca gcgttccggt gatcatggtg accgcccggg atagcgagat   480
   cgacaaggt g gt cggcct gg agct gggcgc t gacgact ac gt gaccaagc cct at t c 537
<210> 52
   <211> 346
   <212> DNA
   <213> Mycobacterium tuberculosis
<400> 52
   gat ggt ct cc cggct gat gc ct gggct ggc agt gct ggcg agcgcct gt t ccaggat ct c 60
   gattgcctcg gccttgtctc ggccccgcac cccttcgatg agcttggtgc gcatatacgt   120
   ct gcagcgac t gccccgct g cggt ggcgcg ccgt cgat ag gt t t cggcga cat ct t cggg 180
   caaatcccga acttgaatgg tagccatagc gctattctag cgtcagatta gcgccagaac   240
   agcgct cgga gt t caccggc t aagaat gcc ggct t at cca ccgggcgt t t cggcggcat c 300
   gatggccttc tctaaccggt cgagcaccgc gcgcagatcg gccagc                  346
<210> 53
   <211> 346
   <212> DNA
   <213> Mycobacterium tuberculosis
<400> 53
   gat ggt ct cc cggct gat gc ct gggct ggc agt gct ggcg agcgcct gt t ccaggat ct c 60
   gattgcctcg gccttgtctc ggccccgcac cccttcgatg agcttggtgc gcatatacgt   120
   t t gcagcgac t gccccgct g cggt ggcgcg ccgt cgat ag gt t t cggcga cat ct t cggg 180
   caaatcccga acttgaatgg tagccatagc gctattctag cgtcagatta gcgccagaac   240
   agcgct cgga gt t caccggc t aagaat gcc ggct t at cca ccgggcgt t t cggcggcat c 300
   gatggccttc tctaaccggt cgagcaccgc gcgcagatcg gccagc                  346
<210> 54
   <211> 309
   <212> DNA
   <213> Mycobacterium tuberculosis
<400> 54
   gatggtctcc cggctgatgc ctgggctggc agtgctggcg agcgcctgtt ccaggatctc 60
   gattgcctcg gccttgtctc ggccccgcac cccttcgatg agcttggtgc gcatatacgt 120
   ctgcagcgac tgccccgctg cggtggcgcg ccgtcgatag gtttcggcga catcttcggg 180
   caaatcccga acttgaatgg tagccatagc ggagttcacc ggctaagaat gccggcttat 240
   ccaccgggcg tttcggcggc atcgatggcc ttctctaacc ggtcgagcac cgcgcgcaga 300
   tcggccagc 309
<210> 55
   <211> 346
   <212> DNA
   <213> Mycobacterium tuberculosis
<400> 55
   gatggtctcc cggctgatgc ctgggctggc agtgctgacg agcgcctgtt ccaggatctc 60
   gattgcctcg gccttgtctc ggccccgcac cccttcgatg agcttggtgc gcatatacgt 120
   ctgcagcgac tgccccgctg cggtggcgcg ccgtcgatag gtttcggcga catcttcggg 180
   caaatcccga acttgaatgg tagccatagc gctattctag cgtcagatta gcgccagaac 240
   agcgctcgga gttcaccggc taagaatgcc ggcttatcca ccgggcgttt cggcggcatc 300
   gatggccttc tctaaccggt cgagcaccgc gcgcagatcg gccagc 346
<210> 56
   <211> 360
   <212> DNA
   <213> Mycobacterium tuberculosis
<400> 56
   gcgccaaggc caccggccaa cgcgagcagc gtcgccgcaa tgccaacaag ccagcctcgc 60
   cgcaaccgcg acgggcgccg ctcgacgagg gcctgttctg ggcacgcgtc ctcgccgagc 120
   tctccggcgt cagattccgc cgaatccgcc gccttcacct gcgggccagt ctggtcggct 180
   ccggcgtcag attccgccga atccgccgcc ttcacctccg tagacgacga cttattcgca 240
   tcggcagggt tcagctggcc ggcgccagca tctccttcca tccgtcgtct cctgttgtgc 300
   tcgagtttgc gacggcgtac ttgacaccat ccggccccac cagttcaccg ctggccgggc 360
<210> 57
   <211> 303
   <212> DNA
   <213> Mycobacterium tuberculosis
<400> 57
   gcgccaaggc caccggccaa cgcgagcagc gtcgccgcaa tgccaacaag ccagcctcgc      60
   cgcaaccgcg acgggcgccg ctcgacgagg gcctgttctg ggcacgcgtc ctcgccgagc     120
   t ct ccggcgt cagat t ccgc cgaat ccgcc gcct t cacct ccgt agacga cgact t at t c 180
   gcatcggcag ggttcagctg gccggcgcca gcatctcctt ccatccgtcg tctcctgttg     240
   t gct cgagt t t gcgacggcg t act t gacac cat ccggccc caccagt t ca ccgct ggccg 300
   ggc 303
<210> 58
   <211> 360
   <212> DNA
   <213> Mycobacterium tuberculosis
<400> 58
   gcgccaaggc caccggccaa cgcgagcagc gtcgccgcaa tgccaacaag ccagcctcgc      60
   cgcaaccgcg acgggcgccg ctcgacgagg gcctgttctg ggcacgcgtc ctcgccgagc     120
   tctccggcgt cagattccgc cgaatccgcc gccttcacct gcgggccagt ctggtcggct     180
   ccggcgt cag at t ccgccga at ccgccgcc t t cacct ccg t agacgacga ct t at t cgca 240
   tcggcagggt tcagctggcc ggcgccagca tctccttcca tccgtcgtct cctgttgtgc     300
   tcgagtttgc gaccgcgtac ttgacaccat ccggccccac cagttcaccg ctggccgggc     360
<210> 59
   <211> 279
   <212> DNA
   <213> Mycobacterium tuberculosis
<400> 59
   ctgtggcagg ctcccggtag ccaccggcct cgccgtattc gtcggcattt ggttcgtcgc      60
   gtctccgtaa ggcgtttcag tggccttcga ggtcgcgctt ccccacccga agacaccgag     120
   tgcggcgcct gcgaccacga tgaccgagag aaaagtcagc ggccctgaca gagcagctgc     180
   gcggaatcag tagcttcgcg gcagcgccgc gaccatcgcc tcgacctggg cccgggtgag     240
   aaccgt gat g ct ggagt ct g at agt cccag aat cct cga 279
<210> 60
   <211> 279
   <212> DNA
   <213> Mycobacterium tuberculosis
<400> 60
   ctgtggcagg ctcccggtag ccaccggcct cgccgtattc gtcggcattt ggttcgtcgc 60
   gtctccgtaa ggcgtttcag tggccttcga ggtcgcgctt ccccacccga agacaccgag 120
   tgcggcgcct gcgaccacga tgaccgagag aaaagtcagc ggccctgaca gagcagctgc 180
   gcggaatcag tagcttcgcg gcagcgccgc gaccatcgcc tcgacctggt cccgggtgag 240
   aaccgtgatg ctggagtctg atagtcccag aatcctcga 279
<210> 61
   <211> 192
   <212> DNA
   <213> Mycobacterium tuberculosis
<400> 61
   gatcagctac gggttggccg gggtggtcac cgcgggcatg ccggtgatcg agtggctata 60
   cgctcggcag ctgcgccggg tggtggcgcc ccagtccagt tagccgggct gtgcttgatt 120
   tgcccggttc cttcccgggt cgtgccgacc cgggtcgtcg ccgggctagt gctcgccgtc 180
   tggcgaaccc at 192
<210> 62
   <211> 249
   <212> DNA
   <213> Mycobacterium tuberculosis
<400> 62
   gatcagctac gggttggccg gggtggtcac cgcgggcatg ccggtgatcg agtggctata 60
   cgctcggcag ctgcgccggg tggtggcgcc ccagtccagt tagccgggct gtgcttgatt 120
   tgcccggttc cttcccgggt cgtgccgacc cgggtcgtcg ccgggctgtg cttgatttgc 180
   ccggttcctt cccgggtcgt gccgacccgg gtcgtcgccg ggctagtgct cgccgtctgg 240
   cgaacccat 249
<210> 63
   <211> 363
   <212> DNA
   <213> Mycobacterium tuberculosis
<400> 63
   gat cagct ac gggt t ggccg gggt ggt cac cgcgggcat g ccggt gat cg agt ggct at a 60
   cgct cggcag ct gcgccggg t ggt ggcgcc ccagt ccagt t agccgggct gt gct t gat t 120
   t gcccggt t c ct t cccgggt cgt gccgacc cgggt cgt cg ccgggct gt g ct t gat t t gc 180
   ccggttcctt cccgggtcgt gccgacccgg gtcgtcgccg ggctgtgctt gatttgcccg     240
   gt t cct t ccc gggt cgt gcc gacccgggt c gt cgccgggc t gt gct t gat t t gcccggt t 300
   cct t cccggg t cgt gccgac ccgggt cgt c gccgggct ag t gct cgccgt ct ggcgaacc 360
   cat 363
<210> 64
   <211> 420
   <212> DNA
   <213> Mycobacterium tuberculosis
<400> 64
   gat cagct ac gggt t ggccg gggt ggt cac cgcgggcat g ccggt gat cg agt ggct at a 60
   cgct cggcag ct gcgccggg t ggt ggcgcc ccagt ccagt t agccgggct gt gct t gat t 120
   t gcccggt t c ct t cccgggt cgt gccgacc cgggt cgt cg ccgggct gt g ct t gat t t gc 180
   ccggttcctt cccgggtcgt gccgacccgg gtcgtcgccg ggctgtgctt gatttgcccg     240
   gt t cct t ccc gggt cgt gcc gacccgggt c gt cgccgggc t gt gct t gat t t gcccggt t 300
   cct t cccggg t cgt gccgac ccgggt cgt c gccgggct gt gct t gat t t g cccggt t cct 360
   tcccgggtcg tgccgacccg ggtcgtcgcc gggctagtgc tcgccgtctg gcgaacccat     420
<210> 65
   <211> 306
   <212> DNA
   <213> Mycobacterium tuberculosis
<400> 65
   gat cagct ac gggt t ggccg gggt ggt cac cgcgggcat g ccggt gat cg agt ggct at a 60
   cgct cggcag ct gcgccggg t ggt ggcgcc ccagt ccagt t agccgggct gt gct t gat t 120
   t gcccggt t c ct t cccgggt cgt gccgacc cgggt cgt cg ccgggct gt g ct t gat t t gc 180
   ccggttcctt cccgggtcgt gccgacccgg gtcgtcgccg ggctgtgctt gatttgcccg     240
   gt t cct t ccc gggt cgt gcc gacccgggt c gt cgccgggc t agt gct cgc cgt ct ggcga 300
   acccat 306
<210> 66
   <211> 457
   <212> DNA
   <213> Mycobacterium tuberculosis
<400> 66
   ccgcaatcac aaacttcaat acccgcacct ccgaccagct cagcacgact tcacgaatcc   60
   cagacctgcg acaccgtcgg cagggctttc gatcctataa caatctggaa acaggatgtc  120
   gcactttcct taaaagagct tccgccaacc cgatcgtcag cgcgcacatg ttgcgcaaaa  180
   gttgttggag ccgaaacgaa ccggcgcgcg ccgttaccgg cgccgccgcc ctaggtggcc  240
   tgcaagacca aaggaggccc gggatcgggt gacagcgggc cgttttcgcg ctgcatcagc  300
   cagcccgcga tgttgtggaa cagcggggcg gccgagtgcc caggcgcgcc gtcggagttg  360
   cgcgccgggt tgtccaacat gatgccgatc acgtagcggg gattgtcggc agtggcgatt  420
   ccggcgaagg tgatccaata cacgtcgtcg aagtagc                           457
<210> 67
   <211> 457
   <212> DNA
   <213> Mycobacterium tuberculosis
<400> 67
   ccgcaatcac aaacttcaat acccgcacct ccgaccagct cagcacgact tcacgaatcc   60
   cagacctgcg acaccgtagg cagggctttc gatcctataa caatctggaa acaggatgtc  120
   gcactttcct taaaagagct tccgccaacc cgatcgtcag cgcgcacatg ttgcgcaaaa  180
   gttgttggag ccgaaacgaa ccggcgcgcg ccgttaccgg cgccgccgcc ctaggtggcc  240
   tgcaagacca aaggaggccc gggatcgggt gacagcgggc cgttttcgcg ctgcatcagc  300
   cagcccgcga tgttgtggaa cagcggggcg gccgagtgcc caggcgcgcc gtcggagttg  360
   cgcgccgggt tgtccaacat gatgccgatc acgtagcggg gattgtcggc agtggcgatt  420
   ccggcgaagg tgatccaata cacgtcgtcg aagtagc                           457
<210> 68
   <211> 457
   <212> DNA
   <213> Mycobacterium tuberculosis
<400> 68
   ccgcaatcac aaacttcaat acccgcacct ccgaccagct cagcacaact tcacgaatcc   60
   cagacctgcg acaccgtcgg cagggctttc gatcctataa caatctggaa acaggatgtc  120
   gcactttcct taaaagagct tccgccaacc cgatcgtcag cgcgcacatg ttgcgcaaaa  180
   gttgttggag ccgaaacgaa ccggcgcgcg ccgttaccgg cgccgccgcc ctaggtggcc  240
   tgcaagacca aaggaggccc gggatcgggt gacagcggga cgttttcgcg ctgcatcagc  300
   cagcccgcga tgttgtggaa cagcggggcg gccgagtgcc caggcgcgcc gtcggagttg  360
   cgcgccgggt tgtccaacat gatgccgatc acgtagcggg gattgtcggc agtggcgatt  420
   ccggcgaagg tgatccaata cacgtcgtcg aagtagc                           457
<210> 69
   <211> 765
   <212> DNA
   <213> Mycobacterium tuberculosis
<400> 69
   cgagttcacc gtccatcatc ttggtgaaca aatccagccc caccgcacgg accaggggtt 60
   cggcgaacgg atcgttgatc aaaccgcgcg gatccttggt ggccagcgca cgcccgaccg 120
   cgacaatggt cgcggtgacg ccgacgctag acgtcagatc ccagttgtcg tcgtcggtgc 180
   gggccaccag cccaccctag tctgattgcc cggttcctcc tcgcgccgca aacggcgcgc 240
   atcgtcaccg ggcgtagtct gattgcccgg ttcctcctcg cgccgcaaac ggcgcgcatc 300
   gtcaccgggc gtagtctgat tgcccggttc ctcctcgcgc cgcaaacggc gcgcatcgtc 360
   accgggcgtc gtctgattgc ccggttcctc ctcgcgccgc aaacggcgcg catcgtcacc 420
   gggcgtcgtc tgattgcccg gttcctcctc gcgccgcaaa cggcgcgcat cgtcaccggg 480
   cgtcgtctga ttgcccggtt cctcctcgcg ccgcaaacca agccggctgg tgctgtgcta 540
   ttggcgtcgg aacagacggc cgtgctggct acagaaccag gcgatgttgc cgtccggccc 600
   gcgcacgaag ttggagcgac tgccggtggg cttgttgtcg ggtccaaggt cgagcccata 660
   gtcgggccga tagaaggcga ggcccagatt ggcgctgttt tggccatccg ggttggcatc 720
   gtcggtgctc atgcttccag caagcaagtc gatgaccgtt gtctc 765
<210> 70
   <211> 480
   <212> DNA
   <213> Mycobacterium tuberculosis
<400> 70
   cgagttcacc gtccatcatc ttggtgaaca aatccagccc caccgcacgg accaggggtt 60
   cggcgaacgg atcgttgatc aaaccgcgcg gatccttggt ggccagcgca cgcccgaccg 120
   cgacaatggt cgcggtgacg ccgacgctag acgtcagatc ccagttgtcg tcgtcggtgc 180
   gggccaccag cccaccctag tctgattgcc cggttcctcc tcgcgccgca aaccaagccg 240
   gctggtgctg tgctattggc gtcggaacag acggccgtgc tggctacaga accaggcgat 300
   gttgccgtcc ggcccgcgca cgaagttgga gcgactgccg gtgggcttgt tgtcgggtcc 360
   aaggtcgagc ccatagtcgg gccgatagaa ggcgaggccc agattggcgc tgttttggcc 420
   atccgggttg gcatcgtcgg tgctcatgct tccagcaagc aagtcgatga ccgttgtctc 480
<210> 71
   <211> 708
   <212> DNA
   <213> Mycobacterium tuberculosis
<400> 71
   cgagttcacc gtccatcatc ttggtgaaca aatccagccc caccgcacgg accaggggtt    60
   cggcgaacgg at cgt t gat c aaaccgcgcg gat cct t ggt ggccagcgca cgcccgaccg 120
   cgacaat ggt cgcggt gacg ccgacgct ag acgt cagat c ccagt t gt cg t cgt cggt gc 180
   gggccaccag cccaccctag tctgattgcc cggttcctcc tcgcgccgca aacggcgcgc   240
   atcgtcaccg ggcgtcgtct gattgcccgg ttcctcctcg cgccgcaaac ggcgcgcatc   300
   gtcaccgggc gtcgtctgat tgcccggttc ctcctcgcgc cgcaaacggc gcgcatcgtc   360
   accgggcgtc gtctgattgc ccggttcctc ctcgcgccgc aaacggcgcg catcgtcacc   420
   gggcgtcgtc tgattgcccg gttcctcctc gcgccgcaaa ccaagccggc tggtgctgtg   480
   ctattggcgt cggaacagac ggccgtgctg gctacagaac caggcgatgt tgccgtccgg   540
   cccgcgcacg aagttggagc gactgccggt gggcttgttg tcgggtccaa ggtcgagccc   600
   atagtcgggc cgatagaagg cgaggcccag attggcgctg ttttggccat ccgggttggc   660
   atcgtcggtg ctcatgcttc cagcaagcaa gtcgatgacc gttgtctc                708
<210> 72
   <211> 651
   <212> DNA
   <213> Mycobacterium tuberculosis
<400> 72
   cgagttcacc gtccatcatc ttggtgaaca aatccagccc caccgcacgg accaggggtt    60
   cggcgaacgg at cgt t gat c aaaccgcgcg gat cct t ggt ggccagcgca cgcccgaccg 120
   cgacaat ggt cgcggt gacg ccgacgct ag acgt cagat c ccagt t gt cg t cgt cggt gc 180
   gggccaccag cccaccctag tctgattgcc cggttcctcc tcgcgccgca aacggcgcgc   240
   atcgtcaccg ggcgtcgtct gattgcccgg ttcctcctcg cgccgcaaac ggcgcgcatc   300
   gtcaccgggc gtcgtctgat tgcccggttc ctcctcgcgc cgcaaacggc gcgcatcgtc   360
   accgggcgtc gtctgattgc ccggttcctc ctcgcgccgc aaaccaagcc ggctggtgct   420
   gtgctattgg cgtcggaaca gacggccgtg ctggctacag aaccaggcga tgttgccgtc   480
   cggcccgcgc acgaagttgg agcgactgcc ggtgggcttg ttgtcgggtc caaggtcgag   540
   cccatagtcg ggccgataga aggcgaggcc cagattggcg ctgttttggc catccgggtt   600
   ggcat cgt cg gt gct cat gc t t ccagcaag caagt cgat g accgt t gt ct c 651
<210> 73
   <211> 594
   <212> DNA
   <213> Mycobacterium tuberculosis
<400> 73
   cgagttcacc gtccatcatc ttggtgaaca aatccagccc caccgcacgg accaggggtt 60
   cggcgaacgg atcgttgatc aaaccgcgcg gatccttggt ggccagcgca cgcccgaccg 120
   cgacaatggt cgcggtgacg ccgacgctag acgtcagatc ccagttgtcg tcgtcggtgc 180
   gggccaccag cccaccctag tctgattgcc cggttcctcc tcgcgccgca aacggcgcgc 240
   atcgtcaccg ggcgtcgtct gattgcccgg ttcctcctcg cgccgcaaac ggcgcgcatc 300
   gtcaccgggc gtcgtctgat tgcccggttc ctcctcgcgc cgcaaaccaa gccggctggt 360
   gctgtgctat tggcgtcgga acagacggcc gtgctggcta cagaaccagg cgatgttgcc 420
   gtccggcccg cgcacgaagt tggagcgact gccggtgggc ttgttgtcgg gtccaaggtc 480
   gagcccatag tcgggccgat agaaggcgag gcccagattg gcgctgtttt ggccatccgg 540
   gttggcatcg tcggtgctca tgcttccagc aagcaagtcg atgaccgttg tctc 594
<210> 74
   <211> 708
   <212> DNA
   <213> Mycobacterium tuberculosis
<400> 74
   cgagttcacc gtccatcatc ttggtgaaca aatccagccc caccgcacgg accaggggtt 60
   cggcgaacgg atcgttgatc aaaccgcgcg gatccttggt ggccagcgca cgcccgaccg 120
   cgacaatggt cgcggtgacg ccgacgctag acgtcagatc ccagttgtcg tcgtcggtgc 180
   gggccaccag cccaccctag tctgattgcc cggttcctcc tcgcgccgca aacggcgcgc 240
   atcgtcaccg ggcgtagtct gattgcccgg ttcctcctcg cgccgcaaac ggcgcgcatc 300
   gtcaccgggc gtagtctgat tgcccggttc ctcctcgcgc cgcaaacggc gcgcatcgtc 360
   accgggcgtc gtctgattgc ccggttcctc ctcgcgccgc aaacggcgcg catcgtcacc 420
   gggcgtcgtc tgattgcccg gttcctcctc gcgccgcaaa ccaagccggc tggtgctgtg 480
   ctattggcgt cggaacagac ggccgtgctg gctacagaac caggcgatgt tgccgtccgg 540
   cccgcgcacg aagttggagc gactgccggt gggcttgttg tcgggtccaa ggtcgagccc 600
   atagtcgggc cgatagaagg cgaggcccag attggcgctg ttttggccat ccgggttggc 660
   atcgtcggtg ctcatgcttc cagcaagcaa gtcgatgacc gttgtctc 708
<210> 75

   <211> 822
   <212> DNA
   <213> Mycobacterium tuberculosis
<400> 75
   cgagttcacc gtccatcatc ttggtgaaca aatccagccc caccgcacgg accaggggtt 60
   cggcgaacgg atcgttgatc aaaccgcgcg gatccttggt ggccagcgca cgcccgaccg 120
   cgacaatggt cgcggtgacg ccgacgctag acgtcagatc ccagttgtcg tcgtcggtgc 180
   gggccaccag cccaccctag tctgattgcc cggttcctcc tcgcgccgca aacggcgcgc 240
   atcgtcaccg ggcgtagtct gattgcccgg ttcctcctcg cgccgcaaac ggcgcgcatc 300
   gtcaccgggc gtagtctgat tgcccggttc ctcctcgcgc cgcaaacggc gcgcatcgtc 360
   accgggcgta gtctgattgc ccggttcctc ctcgcgccgc aaacggcgcg catcgtcacc 420
   gggcgtcgtc tgattgcccg gttcctcctc gcgccgcaaa cggcgcgcat cgtcaccggg 480
   cgtcgtctga ttgcccggtt cctcctcgcg ccgcaaacgg cgcgcatcgt caccgggcgt 540
   cgtctgattg cccggttcct cctcgcgccg caaaccaagc cggctggtgc tgtgctattg 600
   gcgtcggaac agacggccgt gctggctaca gaaccaggcg atgttgccgt ccggcccgcg 660
   cacgaagttg gagcgactgc cggtgggctt gttgtcgggt ccaaggtcga gcccatagtc 720
   gggccgatag aaggcgaggc ccagattggc gctgttttgg ccatccgggt tggcatcgtc 780
   ggtgctcatg cttccagcaa gcaagtcgat gaccgttgtc tc 822
<210> 76
   <211> 651
   <212> DNA
   <213> Mycobacterium tuberculosis
<400> 76
   cgagttcacc gtccatcatc ttggtgaaca aatccagccc caccgcacgg accaggggtt 60
   cggcgaacgg atcgttgatc aaaccgcgcg gatccttggt ggccagcgca cgcccgaccg 120
   cgacaatggt cgcggtgacg ccgacgctag acgtcagatc ccagttgtcg tcgtcggtgc 180
   gggccaccag cccaccctag tctgattgcc cggttcctcc tcgcgccgca aacggcgcgc 240
   atcgtcaccg ggcgtagtct gattgcccgg ttcctcctcg cgccgcaaac ggcgcgcatc 300
   gtcaccgggc gtagtctgat tgcccggttc ctcctcgcgc cgcaaacggc gcgcatcgtc 360
   accgggcgtc gtctgattgc ccggttcctc ctcgcgccgc aaaccaagcc ggctggtgct 420
   gtgctattgg cgtcggaaca gacggccgtg ctggctacag aaccaggcga tgttgccgtc 480
   cggcccgctc acgaagttgg agcgactgcc ggtgggcttg ttgtcgggtc caaggtcgag 540
   cccatagtcg ggccgataga aggcgaggcc cagattggcg ctgttttggc catccgggtt 600
   ggcatcgtcg gtgctcatgc ttccagcaag caagtcgatg accgttgtct c 651
<210> 77
   <211> 777
   <212> DNA
   <213> Mycobacterium tuberculosis
<400> 77
   cgagttcacc gtccatcatc ttggtgaaca aatccagccc caccgcacgg accaggggtt 60
   cggcgaacgg atcgttgatc aaaccgcgcg gatccttggt ggccagcgca cgcccgaccg 120
   cgacaatggt cgcggtgacg ccgacgctag acgtcagatc ccagttgtcg tcgtcggtgc 180
   gggccaccag cccaccctag tctgattgcc cggttcctcc tcgcgccgca aacggcgcgc 240
   atcgtcaccg ggcgtagtct gattgcccgg ttcctcctcg cgccgcaaac ggcgcgcatc 300
   gtcaccgggc gtagtctgat tgcccggttc ctcctcgcgc cgcaaacggc gcgcatcgtc 360
   accgggcgta gtctgattgc ccggttcctc ctcgcgccgc aaacggcgcg catcgtcacc 420
   gggcgtagtc tgattgcccg gttcctcctc gcgccgcaaa cggcgcgcat cgtcaccggg 480
   cgtcgtctga ttgcccggtt cctcctcgcg ccgcaaacgg cgcgcatcgt caccgggcgt 540
   gtctgattgc ccggttcctc ctcgcgccgc aaacggcgcg catcgtcacc gggcgtgtct 600
   gattgcccgg ttcctcctcg cgccgcaaac caagccggct ggtgctgtgc tattggcgtc 660
   ggaacagacg gccgtgctgg ctacagaacc aggcgatgtt gccgtccggc ccgcgcacga 720
   agttggagcg actgccggtg ggcttgttgt cgggtccaag tcgatgaccg ttgtctc 777
<210> 78
   <211> 537
   <212> DNA
   <213> Mycobacterium tuberculosis
<400> 78
   cgagttcacc gtccatcatc ttggtgaaca aatccagccc caccgcacgg accaggggtt 60
   cggcgaacgg atcgttgatc aaaccgcgcg gatccttggt ggccagcgca cgcccgaccg 120
   cgacaatggt cgcggtgacg ccgacgctag acgtcagatc ccagttgtcg tcgtcggtgc 180
   gggccaccag cccaccctag tctgattgcc cggttcctcc tcgcgccgca aacggcgcgc 240
   atcgtcaccg ggcgtcgtct gattgcccgg ttcctcctcg cgccgcaaac caagccggct 300
   ggtgctgtgc tattggcgtc ggaacagacg gccgtgctgg ctacagaacc aggcgatgtt 360
   gccgtccggc ccgcgcacga agttggagcg actgccggtg ggcttgttgt cgggtccaag 420
   gtcgagccca tagtcgggcc gatagaaggc gaggcccaga ttggcgctgt tttggccatc 480
   cgggttggca tcgtcggtgc tcatgcttcc agcaagcaag tcgatgaccg ttgtctc 537
<210> 79
   <211> 651

   <212> DNA
   <213> Mycobacterium tuberculosis
<400> 79
   cgagttcacc gtccatcatc ttggtgaaca aatccagccc caccgcacgg accaggggtt 60
   cggcgaacgg atcgttgatc aaaccgcgcg gatccttggt ggccagcgca cgcccgaccg 120
   cgacaatggt cgcggtgacg ccgacgctag acgtcagatc ccagttgtcg tcgtcggtgc 180
   gggccaccag cccaccctag tctgattgcc cggttcctcc tcgcgccgca aacggcgcgc 240
   atcgtcaccg ggcgtcgtct gattgcccgg ttcctcctcg cgccgcaaac ggcgcgcatc 300
   gtcaccgggc gtcgtctgat tgcccggttc ctcctcgcgc cgcaaacggc gcgcatcgtc 360
   accgggcgtc gtctgattgc ccggttcctc ctcgcgccgc aaaccaagcc ggctggtgct 420
   gtgctattgg cgtcggaaca gacggccgtg ctggctacag aaccaggcga tgttgccgtc 480
   cggcccgcgc acgaagttgg agcgactgcc ggtgggcttg ttgtcgggtc caaggtcgag 540
   cccatagtcg ggccgataga aggcgaggcc cagattggcg ctgttttggc catccgggtt 600
   ggcatcgtct gtgctcatgc ttccagcaag caagtcgatg accgttgtct c 651
<210> 80
   <211> 20
   <212> DNA
   <213> artificial sequence
<220>
   <223> séquence consensus amorce des bactéries du genre Mycobacterium
<400> 80
   gggtggggtgtggtgtttga 20
<210> 81
   <211> 19
   <212> DNA
   <213> artificial sequence
<220>
   <223> séquence consensus amorce des bactéries du genre Mycobacterium
<400> 81
   caaggcatccaccatgcgc 19
<210> 82
   <211> 21
   <212> DNA
   <213> artificial sequence
<220>
   <223> sonde de genre des bactéries Mycobacterium
<400> 82
   tggatagtggttgcgagcatc 21
<210> 83
   <211> 21
   <212> DNA
   <213> artificial sequence
<220>
   <223> sonde spécifique des bactéries du complexe Mycobacterium tuberculosis
<400> 83
   gctagccggcagcgtatccat 21